# EUROPEAN PATENT SPECIFICATION

(11) **EP 2 897 599 B1**
(45) Date of publication and mention of the grant of the patent: **30.12.2020**
(21) Application number: 13839254.3
(22) Date of filing: 18.09.2013
(51) Int. Cl.: A61K 31/10

(54) **SUBSTITUTED ALKYL DIARYL DERIVATIVES, METHODS OF PREPARATION AND USES**
SUBSTITUIERTE ALKYLDIARYLDERIVATE, VERFAHREN ZUR HERSTELLUNG UND VERWENDUNGEN
DÉRIVÉS ALKYL DIARYLES SUBSTITUÉS, PROCÉDÉS DE PRÉPARATION ET D'UTILISATIONS

(30) Priority: 20.09.2012 US 201261703368 P
(43) Date of publication of application: 29.07.2015
(73) Proprietor: Temple University - Of The Commonwealth System of Higher Education, Philadelphia, PA 19122 (US)
(72) Inventor: REDDY, M., V., Ramana, Upper Darby, PA 19082 (US); REDDY, E., Premkumar, Villanova, PA 19085 (US)
(74) Representative: Murgitroyd & Company
(86) International application number: PCT/US2013/060294
(87) International publication number: WO 2014/047110

(56) References cited:
- WO-A1-98/17648
- WO-A2-2009/009041
- US-A- 4 443 477
- US-A- 5 070 099
- US-A- 5 545 750
- US-A1- 2009 124 828
- US-A1- 2011 054 037
- BARBOSA FILHO, JOSE M. ET AL: "Synthesis of several new phenylethylamides of substituted benzoic acids", QUIMICA NOVA , 13(4), 332-4 CODEN: QUNODK; ISSN: 0100-4042, 1990, XP002755972,
- DOI, SATOSHI ET AL: "Abnormal products in the Bischler-Napieralski isoquinoline synthesis", JOURNAL OF THE CHEMICAL SOCIETY, PERKIN TRANSACTIONS 1: ORGANIC AND BIO-ORGANIC CHEMISTRY , (15), 2217-2221 CODEN: JCPRB4; ISSN: 0300-922X, 1997, XP002755973,
- PINGAEW, RATCHANOK ET AL.: "Synthesis and cytotoxicity of novel N-sulfonyl-1,2,3,4-tetrahydroisoquinoline thiosemicarbazone derivatives", MEDICINAL CHEMISTRY RESEARCH, vol. 22, no. 1, 27 March 2012 (2012-03-27) , pages 267-277, XP035154266, DOI: 10.1007/s00044-012-0025-y
- RAJA, ERUM K. ET AL.: "Friedel-Crafts acylation with amides", JOURNAL OF ORGANIC CHEMISTRY, vol. 77, no. 13, 12 June 2012 (2012-06-12) , pages 5788-5793, XP055327115, DOI: 10.1021/jo300922p
- DATABASE CA [Online] CHEMICAL ABSTRACTS SERVICE, COLUMBUS, OHIO, US; WANG, YANXIANG ET AL: "Synthesis and structure-activity relationship of N-(2-arylethyl) isoquinoline derivatives as anti- cancer agents", retrieved from STN Database accession no. 2012:1630172

## Description

### Cross-Reference to Related Application

The benefit of the filing date of U.S. Provisional Patent Applications No. 61/703,368, filed September 20, 2012 is hereby claimed.

### Field of the Invention

The invention relates to compounds, methods for their preparation, and compositions including them, and said compounds and compositions for use in the treatment of cellular proliferative disorders, including, but not limited to, cancer.

### Background of the Invention

Cellular proliferative disorders such as cancer are among the most common causes of death in developed countries. That said, many cellular proliferative disorders have no available cures or few, if any, treatment options to slow the progression of the disease. For cellular proliferative diseases for which treatments exist, undesirable side effects and limited efficacy often call into question the utility of a given treatment. This is particularly true when the available treatment option(s) may not appreciably prolong life, but have a definitive adverse effect on the quality of time remaining. Thus, identifying new effective drugs for cellular proliferative disorders, and in particular cancer, is a continuing focus of medical research.
WO 2009/009041A2 describes compounds for use in modulation of a component of the kinase signaling cascade. A compound disclosed therein is Z-Z-CH₂-SO₂-CH₂-CH₂-Z, wherein Z is unsubstituted phenyl.

### Summary of the Invention

It has been found that certain compounds and compositions are useful for the treatment of cancer and other cellular proliferative disorders. The biologically active compounds of the invention are substituted alkyl diaryl derivatives and related derivatives thereof.

Provided is a compound of Formula I or a salt thereof:

Q₂-X-Y-CHR₃-CHR₄-Q₁ (I)

wherein:
Q₁ is substituted phenyl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by - N(-R₁)-, -O- or -S-; wherein Q₁ has at least one substituent selected from the group consisting of -O-R₁₀ and fluoro;
Q₂ is substituted phenyl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
   wherein at least the 4-position of both Q₁ and Q₂ are substituted and one of Q1 and Q2 is substituted with a substituent other than unsubstituted phenyl;
X is -CH₂-;
Y is
   -S(=O)₂-;
R₁ is selected from the group consisting of H; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
R₃ and R₄ are each H;
R₅ and R₆ are each independently selected from the group consisting of H; halo; and a C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₆) alkyl, (C₁-C₆) dialkyl amino, or acylamino;
or
R₅ and R₆ may combine to form a saturated or unsaturated carbocyclic ring with from 3 to 6 carbon atoms, wherein one or more carbon atoms is optionally replaced with -N(-R₁)-, -O- , or -S-;
R₇ is selected from the group consisting of H; a C₁-C₁₀ saturated or unsaturated, straight or branched, cyclic or acyclic, chiral or achiral hydrocarbyl group; and an inorganic cation or an organic cation, to form a salt;
R₈ is each independently selected from the group consisting of H and (C₁-C₇) hydrocarbyl;
R₉ is selected from the group consisting of H and -(CH₂)ₘ-C(-R₅)(-R₆)-(CH₂)ₙ-COOR₇;
R₁₀ is selected from the group consisting of H, branched or unbranched (C₁-C₆)alkyl, and (C₂-C₈)acyl;
R₁₁ is selected from the group consisting of H, branched or unbranched (C₁-C₆)alkyl, and (C₂-C₈)acyl;
m and n are each independently 0-2;
z is 1-2.

The present disclosure further provides a pharmaceutical composition comprising a pharmaceutically acceptable carrier and a compound of Formula I, or a pharmaceutically acceptable salt thereof.

The present disclosure further provides a compound of Formula I, or a pharmaceutically acceptable salt thereof, for use in the treatment of a cellular proliferative disorder.

In particular embodiments, the cellular proliferative disorder is selected from the group consisting of cancer, malignant and benign tumors, blood vessel proliferative disorders, autoimmune disorders, and fibrotic disorders.

In particular embodiments, the cellular proliferative disorder is selected from the group consisting of hemangiomatosis in newborn, secondary progressive multiple sclerosis, atherosclerosis, neurofibromatosis, ganglioneuromatosis, keloid formation, Paget's disease of the bone, fibrocystic disease of the breast, uterine fibroids, Peyronie's disease, Dupuytren's disease, restenosis, benign proliferative breast disease, benign prostatic hyperplasia, X linked lymphocellular proliferative disorder, post transplantation lymphocellular proliferative disorder, macular degeneration, retinopathies, proliferative vitreoretinopathy, noncancerous lymphocellular proliferative disorders, and cancer.

In particular embodiments, the cellular proliferative disorder is cancer. In certain embodiments, the cancer is selected from the group consisting of ovarian cancer; cervical cancer; breast cancer; prostate cancer; testicular cancer, lung cancer, renal cancer; colorectal cancer; skin cancer; brain cancer; leukemia, including acute myeloid leukemia, chronic myeloid leukemia, acute lymphoid leukemia, and chronic lymphoid leukemia.

Also described is a method of inducing apoptosis of cancer cells in an individual afflicted with cancer, comprising administering to the individual an effective amount of at least one compound of Formula I, or a pharmaceutically acceptable salt thereof.

In certain options, the cancer cells are tumor cells. In particular embodiments, the tumor cells are selected from the group consisting of ovarian, cervical, breast, prostate, testicular, lung, renal, colorectal, skin and brain tumor cells.

Also provided is a compound of Formula I, or salt thereof, for use in medicine. Described is the use of a compound according to Formula I, or a salt thereof, for preparation of a medicament for treatment of a cellular proliferative disorder.

The invention further provides the compound 4-(2-((4-chlorobenzyl)sulfonyl) ethyl)benzoic acid, or a pharmaceutically acceptable salt thereof.

As envisioned in the present invention with respect to the disclosed compositions of matter and methods, in one aspect the embodiments of the invention comprise the components and/or steps disclosed herein. In another aspect, the embodiments of the invention consist essentially of the components and/or steps disclosed herein. In yet another aspect, the embodiments of the invention consist of the components and/or steps disclosed herein.

### Detailed Description of the Invention

The compounds and compositions of the invention are believed to selectively inhibit proliferation of cancer cells, and kill various tumor cell types without killing (or with reduced killing of) normal cells. Cancer cells are killed at concentrations where normal cells may be temporarily growth-arrested but not killed.

The compounds of the invention are believed to inhibit the proliferation of tumor cells, and for some compounds, induce cell death. Cell death results from the induction of apoptosis. The compounds are believed effective against a broad range of tumor types, including but not limited to the following: ovarian cancer, breast cancer, prostate cancer, lung cancer, renal cancer, colorectal cancer, brain cancer and leukemia.

The compounds are also believed useful in the treatment of non-cancer cellular proliferative disorders, including but not limited to the following: hemangiomatosis in newborn, secondary progressive multiple sclerosis, atherosclerosis, neurofibromatosis, ganglioneuromatosis, keloid formation, Paget's disease of the bone, fibrocystic disease of the breast, uterine fibroids, Peyronie's disease, Dupuytren's disease, restenosis, benign proliferative breast disease, benign prostatic hyperplasia, X linked lymphocellular proliferative disorder, post transplantation lymphocellular proliferative disorder, macular degeneration, retinopathies, proliferative vitreoretinopathy, noncancerous lymphocellular proliferative disorders, and cancer.

### I. Definitions

### 1. General

As used in the specification and the appended claims, the singular forms "a," "an" and "the" include plural referents unless the context clearly dictates otherwise.

As used herein, the terms "treat" and "treatment" are used interchangeably and are meant to indicate a postponement of development of a disorder and/or a reduction in the severity of symptoms that will or are expected to develop. The terms further include ameliorating existing symptoms, preventing additional symptoms, and ameliorating or preventing the underlying metabolic causes of symptoms.

As used herein, "individual" (as in the subject of the treatment) means both mammals and non-mammals. Mammals include, for example, humans; non-human primates, e.g. apes and monkeys; cattle; horses; sheep; and goats. Non-mammals include, for example, fish and birds.

The expression "effective amount", when used to describe therapy to an individual suffering from a cancer or other cellular proliferative disorder, refers to the amount of a compound according to Formula I that inhibits the abnormal growth or proliferation, or alternatively induces apoptosis of cancer cells, preferably tumor cells, resulting in a therapeutically useful and selective cytotoxic effect on proliferative cells.

The term "cellular proliferative disorder" means a disorder wherein unwanted cell proliferation of one or more subsets of cells in a multicellular organism occurs. In some such disorders, cells are made by the organism at an atypically accelerated rate.

### 2. Chemical

In the following paragraphs some of the definitions include examples. The examples are intended to be illustrative, and not limiting.

The term "alkyl", by itself or as part of another substituent means, unless otherwise stated, a straight, or branched chain hydrocarbon having the number of carbon atoms designated (*i.e.* C₁-C₆ means one to six carbons) and includes straight, branched chain or cyclic groups. Examples include: methyl, ethyl, propyl, isopropyl, butyl, isobutyl, tert-butyl, pentyl, neopentyl, and hexyl. Most preferred is (C₁-C₆) alkyl, particularly ethyl, methyl and isopropyl.

The term "alkoxy" employed alone or in combination with other terms means, unless otherwise stated, an alkyl group, as defined above, connected to the rest of the molecule via an oxygen atom, such as, for example, methoxy, ethoxy, 1-propoxy, 2-propoxy (isopropoxy) and the higher homologs and isomers. The alkyl portion of the alkoxy group can have a designated number of carbon atoms as defined for alkyl groups above. Preferred are (C₁-C₃)alkoxy, particularly ethoxy and methoxy.

The term "carboxy" means -C(=O)-O-J, wherein J can be H, an inorganic or an organic counter ion, including an alkaline metal and a quaternary ammonium ion formed with an organic base, for example, trimethamine. For example, a carboxy includes a carboxylic acid -(C=O)-OH and metal carboxylate, such as -(C=O)-O⁻Na⁺.

The term "alkylamino" means -NH-alkyl, preferably -NH-(C₁-C₆)alkyl.

The term "acylamino" means -NH-(C=O)-alkyl, preferably -NH-(C=O)-(C₁-C₆)alkyl.

The term "dialkyl amino" means -N[alkyl]₂, preferably -N[(C₁-C₆)alkyl]₂.

The term "aroylamino" means -NH-(C=O)-aryl.

The term "carboxamido" means -(C=O)-NH₂.

The term "carbocyclic ring" refers to an cycloalkane ring formed by combining substituents attached to different carbon atoms. Preferably, R₄ and Q₂ can combine to form a cyclohexyl ring.

The terms "halo" or "halogen" by themselves or as part of another substituent mean, unless otherwise stated, a fluorine, chlorine, bromine, or iodine atom. Preferably, a halogen includes fluorine, chlorine, or bromine, more preferably, fluorine or chlorine.

The term "aromatic" refers to a carbocycle or heterocycle having one or more polyunsaturated rings having aromatic character (i.e. having (4n + 2) delocalized π (pi) electrons where n is an integer).

The term "aryl", employed alone or in combination with other terms, means, unless otherwise stated, a carbocyclic aromatic system containing one or more rings (typically one, two or three rings) wherein such rings may be attached together in a pendent manner, such as a biphenyl, or may be fused, such as naphthalene. Examples include phenyl; anthracyl; and naphthyl. Preferred are phenyl and naphthyl, most preferred is phenyl.

"Substituted aryl" means an aryl, as defined above, substituted by one, two, three, four, or five substituents. In some embodiments, the substituents are selected from among the group consisting of halogen, fluoro; chloro; bromo; nitro; -NR₁₀R₁₁; aroylamino; cyano; carboxy; carboxamido; trifluoromethyl; -O-R₁₀; [-N(-R₁)-(CH₂)ₘ-C(-R₅)(-R₆)-(CH₂)ₙ-COOR₇]_{z}; [-N(-R₉)-(CH₂)ₘ-C(-R₅)(-R₆)-(CH₂)ₙ-COOR₇]_{z}; and C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-. Preferably, a substituted aryl contains one to three substituents selected from methoxy, hydroxy, amino, and chloro, and fluoro, more preferably selected from amino, hydroxy, and methoxy.

In some embodiments, when a substituent is referred to as located at a 2, 3, 4, or 5 position, and/or otho, meta, or para substituted or an aryl group, then the aryl group is a phenyl group numbered as shown below:

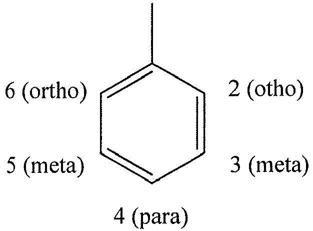

The term "unsubstituted aryl" refers to an aryl, as defined above, which has no substituents.

The term "heteroaryl" or "heteroaromatic" refers to a heterocycle having aromatic character. A polycyclic heteroaryl may include one or more rings which are partially saturated. Examples of heteroaryl groups include: pyridyl, pyrazinyl, pyrimidinyl, particularly 2- and 4-pyrimidinyl, pyridazinyl, indolyl (2-, 3-, 4-, 5-, 6- and 7-), thienyl, furyl, and pyrrolyl, preferably 2-, 3- and 4-pyridyl.

The term "substituted heteroaryl" refers to a heteroaryl, as defined above, substituted by one, two, three, four, or five substituents. In some embodiments, the substituents are selected from among the group consisting of fluoro; chloro; bromo; nitro; amino; acylamino; aroylamino; cyano; carboxy; carboxamido; trifluoromethyl; [-N(-R₁)-(CH₂)ₘ-C(-R₅)(-R₆)-(CH₂)ₙ-COOR₇]_{z}; -OR₁₀; [-N(-R₉)-(CH₂)ₘ-C(-R₅)(-R₆)-(CH₂)ₙ-COOR₇]_{z;} and C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-. Preferably, a substituted heteroaryl contains one to three substituents selected from methoxy, hydroxy, amino, chloro, and fluoro, more preferably selected from amino, and methoxy.

The term "unsubstituted heteroaryl" refers to a heteroaryl, as defined above, which has no substituents.

The term "heterocycle" or "heterocyclyl" or "heterocyclic" by itself or as part of another substituent means, unless otherwise stated, an unsubstituted or substituted, stable, mono- or multi-cyclic heterocyclic ring system which consists of carbon atoms and at least one heteroatom selected from the group consisting of N, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom which affords a stable structure.

Examples of heterocyclyl (non-aromatic) include monocyclic groups such as: aziridinyl, oxiranyl, thiiranyl, azetidinyl, oxetanyl, thietanyl, pyrrolidinyl, pyrrolinyl, imidazolinyl, pyrazolidinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydropyridinyl, 1,4-dihydropyridinyl, piperazinyl, morpholinyl, thiomorpholinyl, pyranyl, 2,3-dihydropyranyl, tetrahydropyranyl, 1,4-dioxanyl, 1,3-dioxanyl, homopiperazinyl, homopiperidinyl, 1,3-dioxepanyl, 4,7-dihydro-1,3-dioxepinyl and hexamethyleneoxidyl, preferably piperidinyl, piperazinyl and morpholinyl.

Examples of polycyclic heterocycles include: indolyl, particularly 3-, 4-, 5-, 6- and 7-indolyl, indolinyl, quinolyl, tetrahydroquinolyl, isoquinolyl, particularly 1- and 5-isoquinolyl, 1,2,3,4-tetrahydroisoquinolyl, cinnolinyl, quinoxalinyl, particularly 2- and 5-quinoxalinyl, quinazolinyl, phthalazinyl, 1,8-naphthyridinyl, 1,4-benzodioxanyl, coumarin, dihydrocoumarin, benzofuryl, particularly 3-, 4-, 1,5-naphthyridinyl, 5-, 6- and 7-benzofuryl, 2,3-dihydrobenzofuryl, 1,2-benzisoxazolyl, benzothienyl, particularly 3-, 4-, 5-, 6-, and 7-benzothienyl, benzoxazolyl, benzthiazolyl, particularly 2-benzothiazolyl and 5-benzothiazolyl, purinyl, benzimidazolyl, particularly 2-benzimidazolyl, benztriazolyl, thioxanthinyl, carbazolyl, carbolinyl, acridinyl, pyrrolizidinyl and quinolizidinyl.

The aforementioned listing of heterocyclyl and heteroaryl moieties is intended to be representative and not limiting.

The term "hydrocarbyl" refers to any moiety comprising only hydrogen and carbon atoms, unless otherwise explicitly stated. A hydrocarbyl can also form a non-aromatic, such as cyclohexane, or an aromatic ring, such as a substituted or unsubstituted (C₆-C₁₀)aryl group. Preferred hydrocarbyl groups are (C₁-C₁₂)hydrocarbyl, more preferred are (C₁-C₇)hydrocarbyl, and most preferred are benzyl and (C₁-C₆) alkyl. For example, a hydrocarbyl group that is substituted by an oxygen includes ethers, such as -CH₂-O-CH₃.

The term "substituted" means that an atom or group of atoms has replaced hydrogen as the substituent attached to another group. For aryl and heteroaryl groups, the term "substituted" refers to any level of substitution, namely mono-, di-, tri-, tetra-, or penta-substitution, where such substitution is permitted. The substituents are independently selected, and substitution may be at any chemically accessible position.

Where a substituent is an alkyl or alkoxy group, the carbon chain may be branched, straight or cyclic, with straight being preferred.

### II. Compounds of the Invention

In one aspect, the invention is directed to a compound of Formula I, or a salt thereof:

Q₂-X-Y-CHR₃-CHR₄-Q₁ (I)

wherein:
Q₁ is substituted phenyl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by - N(-R₁)-, -O- or -S-; wherein Q₁ has at least one substituent selected from the group consisting of -O-R₁₀ and fluoro;
Q₂ is substituted phenyl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
   wherein at least the 4-position of both Q₁ and Q₂ are substituted and one of Q₁ and Q₂ is substituted with a substituent other than unsubstituted phenyl;
X is -CH₂-;
Y is -S(=O)₂-;
R₁ is selected from the group consisting of H; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
R₅ and R₆ are each independently selected from the group consisting of H; halo; and C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₆) alkyl, (C₁-C₆) dialkyl amino or acylamino;
or
R₅ and R₆ may combine to form a saturated or unsaturated carbocyclic ring with from 3 to 6 carbon atoms, wherein one or more carbon atoms is optionally replaced with -N(-R₁)-, -O- or -S-;
R₇ is selected from the group consisting of H; and a C₁-C₁₀ saturated or unsaturated, straight or branched, cyclic or acyclic, chiral or achiral hydrocarbyl group; and an inorganic cation or an organic cation, to form a salt;
R₈ is each independently selected from the group consisting of H and (C₁-C₇) hydrocarbyl;
R₉ is selected from the group consisting of H and -(CH₂)ₘ-C(-R₅)(-R₆)-(CH₂)ₙ-COOR_{7;}
R₁₀ is selected from the group consisting of H, branched or unbranched (C₁-C₆)alkyl, and (C₂-C₈)acyl;
R₁₁ is selected from the group consisting of H, branched or unbranched (C₁-C₆)alkyl, and (C₂-C₈)acyl;
m and n are each independently 0-2;
z is 1-2.

In certain embodiments, the salt of a compound of Formula I is a pharmaceutically acceptable salt.

Q₁ is substituted phenyl, and is substituted in at least the 4-position. In some embodiments, Q₁ is di-substituted phenyl, and is substituted at the 2- and 4-positions. In some embodiments, Q₁ is tri-substituted phenyl, and is substituted at the 2-, 4- and 6-positions.

Q₂ is substituted phenyl, and is substituted in at least the 4-position. In some embodiments, Q₂ is di-substituted phenyl, and is substituted at the 3- and 4-positions.

R₃ is H and R₄ is H.

Another particular embodiment of the invention comprises a compound of Formula I, or a salt thereof, wherein Q₁ is substituted phenyl, with up to 5 substituents selected from the group consisting of fluoro; chloro; bromo; nitro; -NR₁₀R₁₁; aroylamino; cyano; carboxamido; trifluoromethyl; [-N(-R₁)-(CH₂)ₘ-C(-R₅)(-R₆)-(CH₂)ₙ-COOR₇]_{z}; and -O-R₁₀.

Another particular embodiment of the invention comprises a compound of Formula I, or a salt thereof, wherein Q₁ is substituted phenyl, with up to 5 substituents selected from the group consisting of nitro; -NR₁₀R₁₁; aroylamino; cyano; carboxamido; trifluoromethyl; [-N(-R₁)-(CH₂)ₘ-C(-R₅)(-R₆)-(CH₂)ₙ-COOR₇]_{z}; and -O-R₁₀.

Another particular embodiment of the invention comprises a compound of Formula I, or a salt thereof, wherein Q₁ is substituted phenyl, with up to 5 substituents selected from the group consisting of fluoro; chloro; bromo; (C₁-C₃)alkoxy; nitro; -NR₁₀R₁₁; and [-N(-R₁)-(CH₂)ₘ-C(-R₅)(-R₆)-(CH₂)ₙ-COOR₇]_{z}.

Another particular embodiment of the invention comprises a compound of Formula I, or a salt thereof, wherein Q₁ is substituted phenyl, with up to 5 fluoro substituents.

Another particular embodiment of the invention comprises a compound of Formula I, or a salt thereof, wherein Q₁ is substituted phenyl, with up to 5 (C₁-C₃)alkoxy substituents. In certain embodiments of (C₁-C₃)alkoxy substitution, the aryl is phenyl, and the phenyl is substituted with (C₁-C₃)alkoxy in at least the 4-position. In some embodiments, the phenyl is trisubstituted with (C₁-C₃)alkoxy, at the 2-, 4- and 6-positions.

Another particular embodiment of the invention comprises a compound of Formula I, or a salt thereof, wherein Q₁ is substituted phenyl, with up to 5 substituents consisting of (Ci-C₃)alkoxy.

Another particular embodiment of the invention comprises a compound of Formula I, or a salt thereof, wherein Q₁ is substituted phenyl, with up to 5 substituents selected from the group consisting of (C₁-C₃)alkoxy; and [-N(-R₁)-(CH₂)ₘ-C(-R₅)(-R₆)-(CH₂)ₙ-COOR₇]_{z}.

Another particular embodiment of the invention comprises a compound of Formula I, or a salt thereof, wherein Q₂ is substituted phenyl, with up to 5 substituents selected from the group consisting of fluoro; chloro; bromo; nitro; -NR₁₀R₁₁; aroylamino; cyano; carboxy; carboxamido; trifluoromethyl; -O-SO₂-OH; -O-P(=O)(OR₈)₂; [-N(-R₉)-(CH₂)ₘ-C(-R₅)(-R₆)-(CH₂)ₙ-COOR₇]_{z}; and -O-R₁₀.

Another particular embodiment of the invention comprises a compound of Formula I, or a salt thereof, wherein Q₂ is substituted phenyl, with up to 5 substituents selected from the group consisting of nitro; -NR₁₀R₁₁; aroylamino; cyano; carboxy; carboxamido; trifluoromethyl; -O-SO₂-OH; -O-P(=O)(OR₈)₂; [-N(-R₉)-(CH₂)ₘ-C(-R₅)(-R₆)-(CH₂)ₙ-COOR₇]_{z}; and -O-R₁₀.

Another particular embodiment of the invention comprises a compound of Formula I, or a salt thereof, wherein Q₂ is substituted phenyl, with up to 5 substituents selected from the group consisting of chloro; (C₁-C₃)alkoxy; nitro; -NR₁₀R₁₁; hydroxy; -O-SO₂-OH; -OP(=O)(OR₈)₂; and [-N(-R₉)-(CH₂)ₘ-C(-R₅)(-R₆)-(CH₂)ₙ-COOR₇]_{z}.

Another particular embodiment of the invention comprises a compound of Formula I, or a salt thereof, wherein Q₂ is substituted phenyl, with up to 5 substituents selected from the group consisting of fluoro; (C₁-C₃)alkoxy; cyano; carboxy; carboxamido; and hydroxy.

Another particular embodiment of the invention comprises a compound of Formula I, or a salt thereof, wherein Q₂ is substituted phenyl, with up to 5 substituents selected from the group consisting of (C₁-C₃)alkoxy and chloro.

Another particular embodiment of the invention comprises a compound of Formula I, or a salt thereof, wherein Q₂ is substituted phenyl, with up to 5 substituents selected from the group consisting of -NR₁₀R₁₁; and hydroxy.

Another particular embodiment of the invention comprises a compound of Formula I, or a salt thereof, wherein Q₂ is substituted phenyl, with up to 5 substituents selected from the group consisting of (C₁-C₃)alkoxy; and -NR₁₀R₁₁.

Another particular embodiment of the invention comprises a compound of Formula I, or a salt thereof, wherein Q₂ is substituted phenyl, with up to 5 substituents selected from the group consisting of (C₁-C₃)alkoxy; and [-N(-R₉)-(CH₂)ₘ-C(-R₅)(-R₆)-(CH₂)ₙ-COOR₇]_{z}.

Another particular embodiment of the invention comprises a compound of Formula I, or a salt thereof, wherein Q₂ is substituted phenyl, with up to 5 substituents selected from the group consisting of (C₁-C₃)alkoxy; and hydroxy.

In some embodiments, Q₂ is di-substituted phenyl, and is substituted at the 3- and 4-positions.

In some embodiments, Q₂ is substituted phenyl with 1, 2, 3, 4 or 5 substituents thereon being aryl and/or heteroaryl.

Another particular embodiment of the invention comprises a compound of Formula I, or a salt thereof, wherein R₁ is selected from the group consisting of H and a C₁-C₁₀ saturated or unsaturated, straight or branched, acyclic, chiral or achiral hydrocarbyl group, wherein optionally at least one carbon atom of the hydrocarbyl group is replaced by -N(-R₁)-, -O- or -S-.

Another particular embodiment of the invention comprises a compound of Formula I, or a salt thereof, wherein R₁ is selected from the group consisting of H and a C₁-C₁₀ saturated, straight or branched, acyclic, chiral or achiral hydrocarbyl group, wherein optionally at least one carbon atom of the hydrocarbyl group is replaced by -N(-R₁)-, -O- or -S-.

Another particular embodiment of the invention comprises a compound of Formula I, or a salt thereof, wherein R₁ is H.

Another particular embodiment of the invention comprises a compound of Formula I, or a salt thereof, wherein R₁ is selected from the group consisting of H and a C₁-C₁₀ saturated or unsaturated, straight or branched, cyclic or acyclic, chiral or achiral hydrocarbyl group, wherein optionally one or two carbon atoms of the hydrocarbyl group is replaced by -N(-R₁)-, -O- or -S-.

Another particular embodiment of the invention comprises a compound of Formula I, or a salt thereof, wherein R₅ is selected from the group consisting of H; halo; and a C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₃)dialkyl amino, or acylamino.

Another particular embodiment of the invention comprises a compound of Formula I, or a salt thereof, wherein R₅ is H.

Another particular embodiment of the invention comprises a compound of Formula I, or a salt thereof, wherein R₅ is halo.

Another particular embodiment of the invention comprises a compound of Formula I, or a salt thereof, wherein R₅ is a C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₃)dialkyl amino, or acylamino.

Another particular embodiment of the invention comprises a compound of Formula I, or a salt thereof, wherein R₅ is a C₁-C₁₀ saturated or unsaturated, straight or branched, cyclic or acyclic, chiral or achiral hydrocarbyl group.

Another particular embodiment of the invention comprises a compound of Formula I, or a salt thereof, wherein R₅ is a C₁-C₁₀ saturated or unsaturated, straight or branched, cyclic or acyclic, chiral or achiral hydrocarbyl group substituted with halogen.

Another particular embodiment of the invention comprises a compound of Formula I, or a salt thereof, wherein R₆ is selected from the group consisting of H; halo; and C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₃)dialkyl amino, or acylamino.

Another particular embodiment of the invention comprises a compound of Formula I, or a salt thereof, wherein R₆ is H.

Another particular embodiment of the invention comprises a compound of Formula I, or a salt thereof, wherein R₆ is halo.

Another particular embodiment of the invention comprises a compound of Formula I, or a salt thereof, wherein R₆ is a C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₃)dialkyl amino, or acylamino.

Another particular embodiment of the invention comprises a compound of Formula I, or a salt thereof, wherein R₆ is a C₁-C₁₀ saturated or unsaturated, straight or branched, cyclic or acyclic, chiral or achiral hydrocarbyl group.

Another particular embodiment of the invention comprises a compound of Formula I, or a salt thereof, wherein R₆ is a C₁-C₁₀ saturated or unsaturated, straight or branched, cyclic or acyclic, chiral or achiral hydrocarbyl group substituted with halogen.

Another particular embodiment of the invention comprises a compound of Formula I, or a salt thereof, wherein R₅ and R₆ may combine to form a saturated or unsaturated carbocyclic ring with from 3 to 6 carbon atoms, wherein one or more carbon atoms is optionally replaced with -N(-R₁)-, -O-, or -S-. In a sub-embodiment, one or two carbon atoms is optionally replaced with -N(-R₁)- or -S- to form a heterocycle

Another particular embodiment of the invention comprises a compound of Formula I, or a salt thereof, wherein R₅ and R₆ may combine to form a saturated or unsaturated carbocyclic ring with from 3 to 6 carbon atoms.

Another particular embodiment of the invention comprises a compound of Formula I, or a salt thereof, wherein R₇ is H.

Another particular embodiment of the invention comprises a compound of Formula I, or a salt thereof, wherein R₇ is a C₁-C₁₀ saturated or unsaturated, straight or branched, cyclic or acyclic, chiral or achiral hydrocarbyl group.

Another particular embodiment of the invention comprises a compound of Formula I, or a salt thereof, wherein R₇ is an inorganic cation, to form a salt.

Another particular embodiment of the invention comprises a compound of Formula I, or a salt thereof, wherein R₇ is Na⁺, to form a salt.

Another particular embodiment of the invention comprises a compound of Formula I, or a salt thereof, wherein R₇ is an organic cation, to form a salt.

Another particular embodiment of the invention comprises a compound of Formula I, or a salt thereof, wherein R₈ is H.

Another particular embodiment of the invention comprises a compound of Formula I, or a salt thereof, wherein R₈ is (C₁-C₇) hydrocarbyl.

Another particular embodiment of the invention comprises a compound of Formula I, or a salt thereof, wherein R₉ is -(CH₂)ₘ-C(-R₅)(-R₆)-(CH₂)ₙ-COOR₇.

Another particular embodiment of the invention comprises a compound of Formula I, or a salt thereof, wherein R₉ is -(CH₂)ₘ-C(-R₅)(-R₆)-(CH₂)ₙ-COOR₇ and z is 1.

Another particular embodiment of the invention comprises a compound of Formula I, or a salt thereof, if Q₁ is substituted with hydroxy or alkoxy, then Q₂ cannot be substituted by bromo in the 2-position.

In one embodiment of the compound of Formula I, or salt thereof:
Q₂ is substituted phenyl, with up to 5 substituents selected from the group consisting of:
chloro; nitro; -NR₁₀R₁₁; aroylamino; cyano; carboxy; carboxamido; trifluoromethyl; - O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, acyclic, chiral or achiral hydrocarbyl group, wherein optionally at least one carbon atom of the hydrocarbyl group is replaced by - N(-R₁)-, -O- or -S-.

In one aspect, the invention is a compound of Formula I, or a salt thereof, wherein:
Q₁ is a substituted phenyl with up to 5 substituents selected from the group consisting of fluoro; (C₁-C₃)alkoxy; and carboxy;
Q₂ is a substituted phenyl with up to 5 substituents selected from the group consisting of chloro; -NR₁₀R₁₁; carboxy; -O-R₁₀; and [-N(-R₉)-(CH₂)ₘ-C(-R₅)(-R₆)-(CH₂)ₙ-COOR₇]_{z};
R₁ is H; and
R₅ and R₆ are each independently selected from the group consisting of H; a C₁-C₁₀ saturated or unsaturated, straight or branched, cyclic or acyclic, chiral or achiral hydrocarbyl group, optionally substituted with bromo or chloro.

In preferred embodiments of the preceding embodiment of the invention, Q₁ and Q₂ are defined as follows. Q₁ is phenyl substituted in at least the 4-position with fluoro, (C₁-C₃)alkoxy or carboxy. In some embodiments, the 4-position substituent is (C₁-C₃)alkoxy. In some embodiments, the phenyl is substituted at the 2-, 4- and 6-positions with (C₁-C₃)alkoxy, most preferably, methoxy. Q₂ is phenyl substituted in at least the 4-position with chloro; - NR₁₀R₁₁; carboxy; -O-R₁₀; and [-N(-R₉)-(CH₂)ₘ-C(-R₅)(-R₆)-(CH₂)ₙ-COOR₇]_{z}. In some embodiments, the 4-position substituent is selected from NR₁₀R₁₁,[-N(-R₉)-(CH₂)ₘ-C(-R₅)(-R₆)-(CH₂)ₙ-COOR₇]_{z} and -O-R₁₀, with preferred [-N(-R₉)-(CH₂)ₘ-C(-R₅)(-R₆)-(CH₂)ₙ-COOR₇]_{z} groups comprising -NHCH₂COOH and -NHCH₂COOCH₃ and preferred -O-R₁₀ groups comprising -OH and (C₁-C₃)alkoxy, most preferably, methoxy. In some embodiments, Q₂ is a di-substituted phenyl substituted at the 4-position with NR₁₀R₁₁, -O-R₁₀ or [-N(-R₉)-(CH₂)ₘ-C(-R₅)(-R₆)-(CH₂)ₙ-COOR₇]_{z} and at the 3-position with NR₁₀R₁₁, carboxy, -O-R₁₀ or [-N(-R₉)-(CH₂)ₘ-C(-R₅)(-R₆)-(CH₂)ₙ-COOR₇]_{z}. In particular embodiments of said 3-,4-disubstitued phenyl, the 4-position substiutent is -O-R₁₀ and the 3-position substituent is NR₁₀R₁₁, -O-R₁₀ or [-N(-R₉)-(CH₂)ₘ-C(-R₅)(-R₆)-(CH₂)ₙ-COOR₇]_{z}.

In one aspect, the compound of Formula I is:
1-chloro-4-(((4-fluorophenethyl)sulfonyl)methyl)benzene;
2-methoxy-5-(((2,4,6-trimethoxyphenethyl)sulfonyl)methyl)aniline;
2-methoxy-5-(((2,4,6-trimethoxyphenethyl)sulfonyl)methyl)phenol;
*N*-(3-amino-4-methoxyphenyl)-3-(2,4,6-trimethoxyphenyl)propanamide;
methyl 2-((2-methoxy-5-(((2,4,6-trimethoxyphenethyl)sulfonyl)methyl) phenyl)amino)acetate;
dimethyl 2,2'-((2-methoxy-5-(((2,4,6-trimethoxyphenethyl)sulfonyl) methyl)phenyl)azanediyl)diacetate;
2-((2-methoxy-5-(((2,4,6-trimethoxyphenethyl)sulfonyl)methyl)phenyl) amino)acetic acid;
2-((2-methoxy-5-(((2,4,6-trimethoxyphenethyl)sulfonyl)methyl)phenyl) amino)propanoic acid;
2-((2-methoxy-5-(((2,4,6-trimethoxyphenethyl)sulfonyl)methyl)phenyl)amino)-2-methylpropanoic acid;
2-((2-methoxy-5-(((2,4,6-trimethoxyphenethyl)sulfonyl)methyl)phenyl)amino)-2-phenylacetic acid; 2-(4-fluorophenyl)-2-((2-methoxy-5-(((2,4,6-trimethoxyphenethyl)sulfonyl)methyl)phenyl)amino)acetic acid;
2-(4-chlorophenyl)-2-((2-methoxy-5-(((2,4,6-trimethoxyphenethyl)sulfonyl) methyl)phenyl)amino)acetic acid;
2-((2-methoxy-5-(((2,4,6-trimethoxyphenethyl)sulfonyl)methyl)phenyl)amino)-3-phenylpropanoic acid;
2-cyclopropyl-2-((2-methoxy-5-(((2,4,6-trimethoxyphenethyl)sulfonyl)methyl) phenyl)amino)acetic acid;
2-((2-methoxy-5-(((2,4,6-trimethoxyphenethyl)sulfonyl)methyl)phenyl)amino)-2-(1*H*-pyrrol-3-yl)acetic acid;
1,3,5-trimethoxy-2-(2-((4-methoxybenzyl)sulfonyl)ethyl)benzene;
*N*-(4-methoxyphenethyl)-4-methylbenzenesulfonamide;
4-methoxy-*N*-(4-methoxyphenethyl)-3-nitrobenzenesulfonamide;
3-amino-4-methoxy-*N*-(4-methoxyphenethyl)benzenesulfonamide;
4-methoxy-3-nitro-*N*-(2,4,6-trimethoxyphenethyl)benzenesulfonamide;
3-amino-4-methoxy-*N*-(2,4,6-trimethoxyphenethyl)benzenesulfonamide,
4-methoxy-*N*-(4-methoxyphenethyl)benzenesulfonamide;
4-methoxy-*N*-(2,4,6-trimethoxyphenethyl)benzenesulfonamide;
4-methyl-*N*-(2,4,6-trimethoxyphenethyl)benzenesulfonamide;
4-methoxy-*N*-(2,4,6-trimethoxyphenethyl)benzamide;
4-methoxy-*N*-(4-methoxyphenethyl)benzamide;
4-methoxy-*N*-(4-methoxyphenethyl)-3-nitrobenzamide;
3-amino-4-methoxy-*N*-(4-methoxyphenethyl)benzamide;
4-methoxy-3-nitro-*N*-(2,4,6-trimethoxyphenethyl)benzamide;
3-amino-4-methoxy-*N*-(2,4,6-trimethoxyphenethyl)benzamide;
N-(4-methoxyphenethyl)-4-methylbenzenesulfonamide;
4-methoxy-N-(4-methoxyphenethyl)-3-nitrobenzenesulfonamide;
3-amino-4-methoxy-N-(4-methoxyphenethyl)benzenesulfonamide;
4-methoxy-3-nitro-N-(2,4,6-trimethoxyphenethyl)benzenesulfonamide;
3-amino-4-methoxy-N-(2,4,6-trimethoxyphenethyl)benzenesulfonamide;
4-methoxy-N-(4-methoxyphenethyl)benzenesulfonamide;
4-methoxy-N-(2,4,6-trimethoxyphenethyl)benzenesulfonamide;
4-methyl-N-(2,4,6-trimethoxyphenethyl)benzenesulfonamide;
2-(4-methoxyphenyl)-N-(2,4,6-trimethoxyphenyl)ethanesulfonamide;
4-(((3 ,4-dimethoxyphenethyl)thio)methyl)-2-methoxyphenol;
4-(((3,4-dimethoxyphenethyl)sulfonyl)methyl)-2-methoxyphenol;
or a salt thereof.

Particularly preferred compounds include the following and their pharmaceutically acceptable salts:
2-((2-methoxy-5-(((2,4,6-trimethoxyphenethyl)sulfonyl)methyl)phenyl)amino) acetic acid;
4-(2-((4-chlorobenzyl)sulfonyl)ethyl)benzoic acid; and
3-amino-4-methoxy-*N*-(2,4,6-trimethoxyphenethyl)benzenesulfonamide.

Preferred salts include, for example, sodium salts. A preferred sodum salt is sodium 2-((2-methoxy-5-(((2,4,6-trimethoxyphenethyl)sulfonyl) methyl)phenyl) amino) acetate.

### III. Methods for Preparing Compounds of the Invention and Intermediates Useful in the Synthesis of Compounds of the Invention

There are provided processes for preparing compounds according to Formula I, intermediates that are useful in the preparation of such compounds, and processes for preparing such intermediates.

Processes for producing embodiments of Formula I are disclosed, wherein the embodiments of Formula I are identified as Formula Ia, Formula Ib, Formula Ic, Formula Id, Formula Ie, Formula If, Formula Ig, Formula Ih, and Formula Ii. In the formulas and schemes that follow, unless otherwise indicated, Q₁, Q₂, X, Y, R₁, R₂, R₃, R₄, R₅, R₆, R₇, R₈, R₉, m, n, and z are as defined above for Formula I.

In an embodiment, a process for preparing a compound of Formula Ia or a salt thereof is provided, wherein Formula Ia is a sulfide compound of Formula I. The process comprises:
reacting a compound of Formula AA with a compound of Formula BB to produce a compound of Formula Ia: 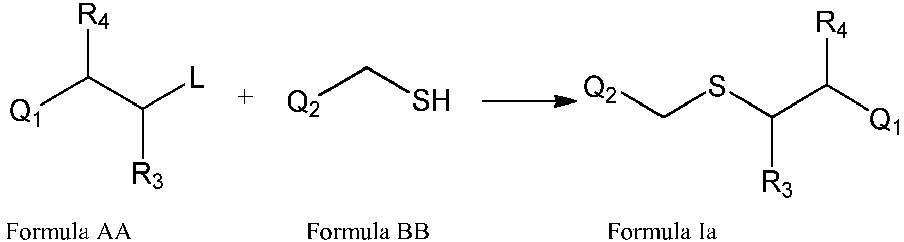 in a reaction mixture, wherein Q₁, Q₂, R₃, and R₄ are as defined above; L is a leaving group selected from the group consisting of Cl, Br, and I; and optionally, isolating from the reaction mixture the compound of Formula Ia or a salt thereof.

In an embodiment, the aforesaid reaction takes place in the presence of a base.

In an embodiment, a process for providing a compound of Formula Ib or a salt thereof is provided, wherein Formula Ib is a sulfone compound of Formula I. The process comprises:
oxidizing a compound of Formula Ia to produce a compound of Formula Ib, 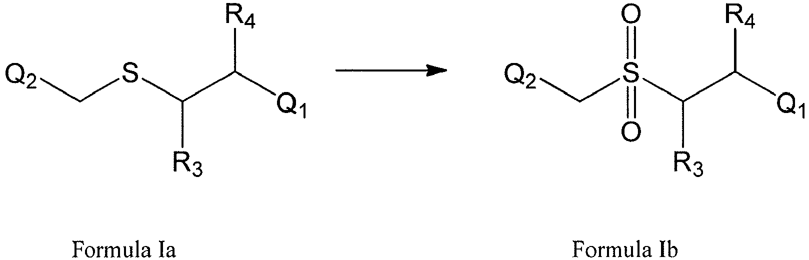 in a reaction mixture, wherein Q₁, Q₂, R₃, and R₄ are as defined above; and optionally, isolating from the reaction mixture the compound of Formula Ib or a salt thereof.

In an embodiment, the aforesaid reaction takes place in the presence of an acid and a peroxide.

In an embodiment, a process for providing a compound of Formula Ic or a salt thereof is provided, wherein Formula Ic is a sulfonamide compound of Formula I. The process comprises a reaction step of:
reacting a compound of Formula CC with a compound of Formula DD to produce a compound of Formula Ic, 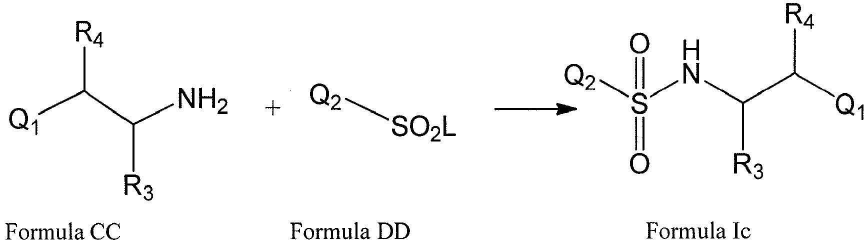 in a reaction mixture, wherein Q₁, Q₂, R₃, and R₄ are as defined above; and optionally, isolating from the reaction mixture the compound of Formula Ic or a salt thereof.

In an embodiment, the aforesaid reaction takes place in the presence of a base.

In an embodiment, a process for providing a compound of Formula Id or a salt thereof is provided, wherein Formula Id is a sulfonamide compound of Formula I. The process comprises a reaction step of:
reacting a compound of Formula EE with a compound of Formula FF to produce a compound of Formula Id, 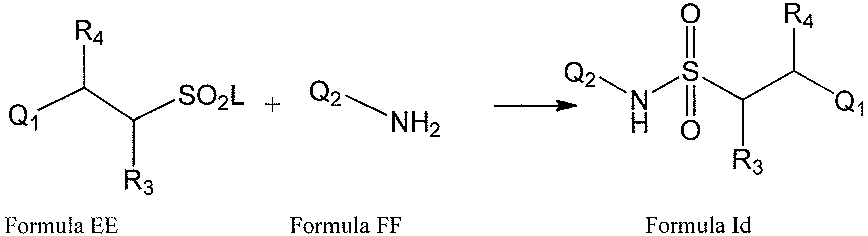 in a reaction mixture, wherein Q₁, Q₂, R₃, and R₄ are as defined above; and optionally, isolating from the reaction mixture the compound of Formula Id or a salt thereof.

In an embodiment, a process for providing a compound of Formula If or a salt thereof is provided, wherein Formula If is a subset of Formula I. The process comprises a reaction step of:
reducing a compound of Formula Ie to produce a compound of Formula If, 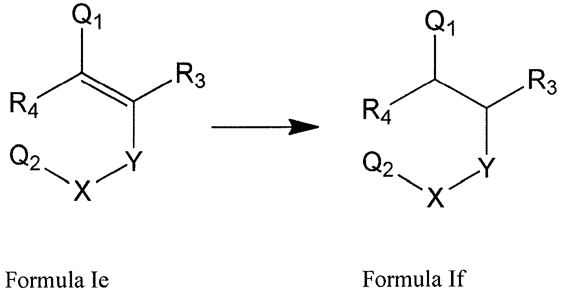 in a reaction mixture, wherein X, Y, Q₁, Q₂, R₃, and R₄ are as defined above; and optionally, isolating from the reaction mixture the compound of Formula If or a salt thereof.

In an embodiment, the aforesaid reaction takes place in the presence of a platinum, rhodium, raney nickel, or palladium catalyst, preferably a palladium catalyst.

In an embodiment, a process for providing Formula Ii or a salt thereof is provided, wherein Formula Ii is a subset of compound of Formula I. The process comprises a reaction step of:
reacting a compound of Formula Ih to produce a compound of Formula Ii, 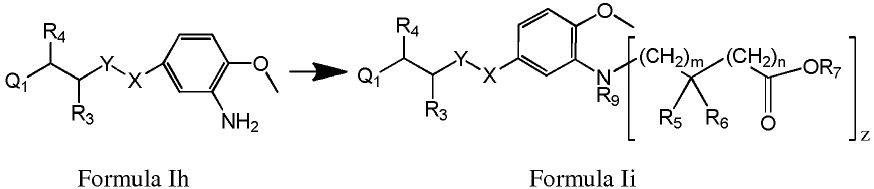 in a reaction mixture, wherein X, Y, Q₁, R₃, R₄, R₅, R₆, R₇, and R₉, m, n, and z are as defined above, and optionally, wherein R₇ is not H, then a second reaction can be performed to convert the R₇ into H. From the forgoing reaction(s) the compound of Formula Ii or a salt thereof may be isolated from the reaction mixture.

In the following paragraphs, a more detailed discussion of the processes for producing embodiments of Formula I is provided, along with preferred embodiments and reaction details. The embodiments of Formula I are identified as Formula Ia, Formula Ib, Formula Ic, Formula Id, Formula Ie, Formula If, Formula Ig, Formula Ih, and Formula Ii.

In an embodiment, a detailed process for preparing Formula Ia or a salt thereof is further provided, wherein Formula Ia is a sulfide compound of Formula I. 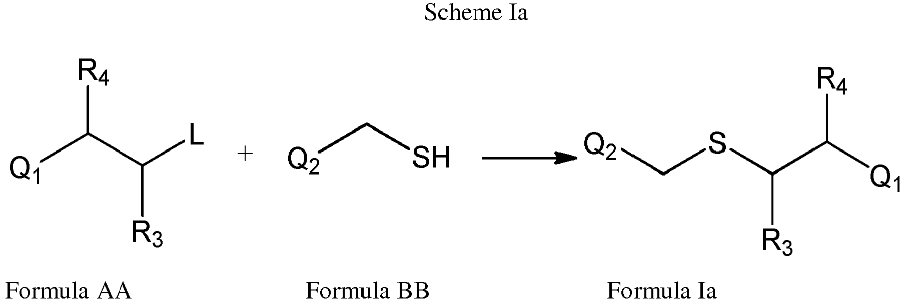 In Scheme Ia, Q₁, Q₂, R₃, and R₄ are as defined above; and L is a leaving group selected from the group consisting of: Cl, Br, and I.

Particular embodiments of the process in Scheme Ia include those wherein:
R₃ is H;
R₄ is H;
Q₁ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by - N(-R₁)-, -O- or -S-; and
   Q₂ is selected from the group consisting of substituted aryl and substituted heteroaryl, with up to 5 substituents selected from the group consisting of:
      fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-.

In another embodiment of Scheme Ia, R₃ is H; R₄ is H; Q₁ is F; and Q₂ is Cl.

The reaction in Scheme Ia can be achieved in the presence of base catalysts or reagents. Preferably, the base is at least one of K₂CO₃ or NaOH. The reaction in Scheme Ia is preferably carried out in an appropriate solvent, preferably methanol. The reactions in Scheme Ia are typically carried out at a temperature between 20 °C and the reflux temperature of the solvent, which is typically about 50-100°C. The temperature during the reaction in Scheme Ia is preferably room temperature for 3 hours. The reaction preferably takes place under an inert atmosphere, such as nitrogen gas.

In an embodiment, a detailed process for preparing Formula Ib or a salt thereof is further provided, wherein Formula Ib is a sulfone compound of Formula I. 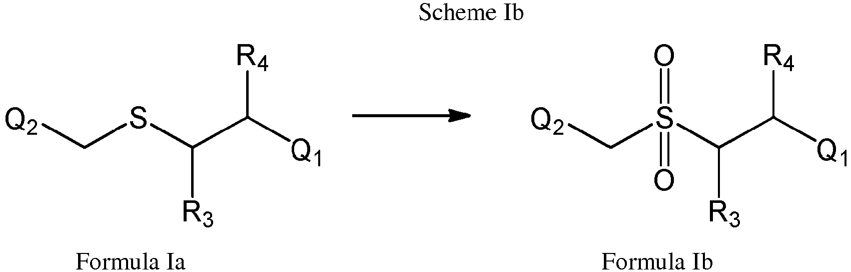 In Scheme Ib, Q₁, Q₂, R₃, and R₄ are as defined above.

Particular embodiments of the process in Scheme Ib include those wherein:
R₃ is H; R₄ is H; and Q₁ is selected from the group consisting of substituted aryl and substituted heteroaryl, with up to 5 substituents selected from the group consisting of:
fluoro; chloro; bromo; nitro; -NR₁₀R₁₁; aroylamino; cyano; carboxamido; trifluoromethyl; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-; and
Q₂ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-.

In another embodiment of Scheme Ib, R₃ is H; R₄ is H; Q₁ is F; and Q₂ is Cl.

The reaction in Scheme Ib can proceed in the presence of an acid, such a glacial acetic acid, and a peroxide, such as hydrogen peroxide. The reaction in Scheme Ib is preferably carried out in an appropriate solvent, but can be carried out in a liquid acid without the addition of a solvent. The reactions in Scheme Ib are typically carried out at under ambient conditions. The reaction in Scheme Ib is preferably carried out at room temperature for 24 hours under an inert atmosphere.

In another embodiment, the peroxide is meta-chloroperoxybenzoic acid (mCPBA), which may seletively oxidze a sulfide to form sulfoxide as shown in Scheme Ib-2.

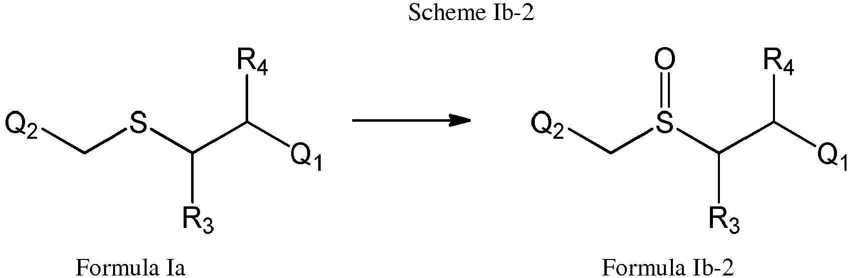

In Scheme Ib, Q₁, Q₂, R₃, and R₄ are as defined above.

Also described is a detailed process for preparing Formula Ic or a salt thereof, wherein Formula Ic is a sulfonamide compound of Formula I.

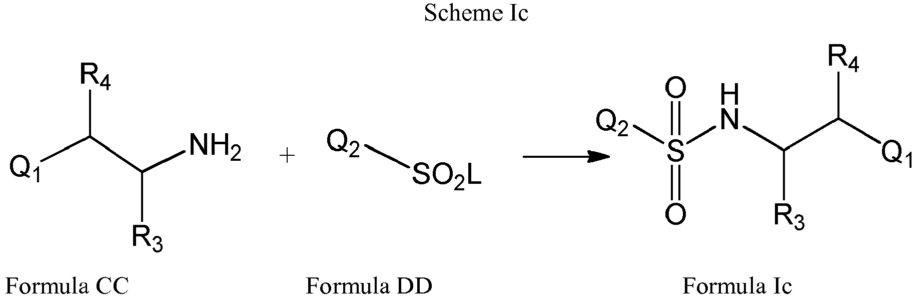

In Scheme Ic, Q₁, Q₂, R₃, and R₄ are as defined above, and L is a leaving group selected from the group consisting of: Cl, Br, and I.

Particular options for the process in Scheme Ic include those wherein:
R₃ is H; R₄ is H; and Q₁ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-; and
Q₂ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-.

The reaction in Scheme Ic can be achieved in the presence of base catalysts or reagents. Preferably, the base is at least one of pyridine or Et₃N. The reaction in Scheme Ic is preferably carried out in an appropriate solvent, preferably anhydrous acetone or methylene chloride. The reactions in Scheme Ic are typically carried out at a temperature between room temperature and the reflux temperature of the solvent, which is typically about 50-100°C. The temperature during the reaction in Scheme Ic is preferably room temperature for 3 hours. The reaction preferably takes place under an inert atmosphere, such as nitrogen gas.

A detailed process for preparing Formula Id or a salt thereof is also described, wherein Formula Id is a sulfonamide compound of Formula I. 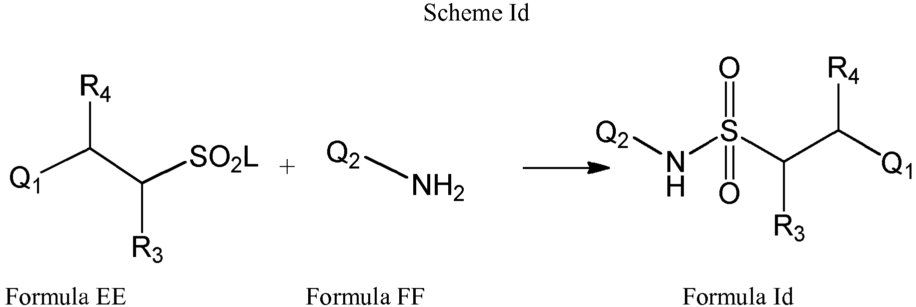 In Scheme Id, Q₁, Q₂, R₃, and R₄ are as defined above and L is a leaving group selected from the group consisting of: Cl, Br, and I.

Particular embodiments of the process in Scheme Id include those wherein:
R₃ is H; R₄ is H; and Q₁ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-; and
Q₂ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-.

The reaction in Scheme Id can be achieved in the presence of base catalysts or reagents. Preferably, the base is at least one of pyridine, K₂CO₃ or NaOH. The reaction in Scheme Id is preferably carried out in an appropriate solvent, preferably anhydrous acetone. The reactions in Scheme Id are typically carried out at a temperature between 20 °C and the reflux temperature of the solvent, which is typically about 50-100°C. The temperature during the reaction in Scheme Id is preferably room temperature for 3 hours. The reaction preferably takes place under an inert atmosphere, such as nitrogen gas.

A detailed process for preparing Formula Ie or a salt thereof is described, wherein Formula Ie is a subset of a compound of Formula I. 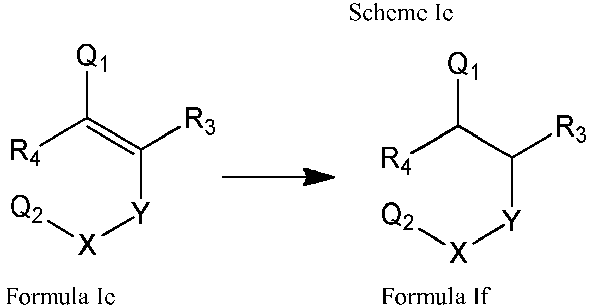 In Scheme Ie, X, Y, Q₁, Q₂, R₃, and R₄ are as defined above.

Particular options for the process in Scheme Ie include those wherein:
X is selected from the group consisting of -CH(R₂)- and -N(-R₁)-; Y is selected from the group consisting of -S(=O)₂- and -C(=O)- ; R₁ is H; R₂ is H; R₃ is H; R₄ is H; and Q₁ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-; and
Q₂ is selected from the group consisting of substituted aryl and substituted heteroaryl, with up to 5 substituents selected from the group consisting of:
   fluoro; chloro; bromo; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-.

In another option of Scheme Ie, X is -CH(R₂)- ; Y is -S(=O)₂- ; R₂ is H; R₃ is H; R₄ is H; Q₁ is substituted aryl, with up to 5 (C₁-C₃)alkoxy substituents; and Q₂ is aryl, with up to 5 substituents selected from the group consisting of: (C₁-C₃)alkoxy, and -NR₁₀R₁₁.

In another option of Scheme Ie, X is -CH(R₂)- ; Y is -S(=O)₂- ; R₂ is H; R₃ is H; R₄ is H; Q₁ is substituted aryl, with up to 5 (C₁-C₃)alkoxy substituents; and Q₂ is aryl, with up to 5 substituents selected from the group consisting of (C₁-C₃)alkoxy, [-N(-R₉)-(CH₂)ₘ-C(-R₅)(-R₆)-(CH₂)ₙ-COOR₇]_{z}; R₉ is H; and z is 1.

In another option of Scheme Ie, X is -CH(R₂)- ; Y is -S(=O)₂- ; R₂ is H; R₃ is H; R₄ is H; Q₁ is substituted aryl, with up to 5 (C₁-C₃)alkoxy substituents; and Q₂ is aryl, with up to 5 substituents selected from the group consisting of: (C₁-C₃)alkoxy, and hydroxy.

In another option of Scheme Ie, X is -CH(R₂)- ; Y is -S(=O)₂- ; R₂ is H; R₃ is H; R₄ is H; Q₁ is substituted aryl, with up to 5 and -(C=O)-OH substituents; and Q₂ is aryl, with up to 5 chloro substituents.

In another option of Scheme Ie, X is -N(-R₁)-; Y is -C(=O)-; R₁ is H; R₃ is H; R₄ is H; Q₁ is substituted aryl, with up to 5 (C₁-C₃)alkoxy substituents; and Q₂ is aryl, with up to 5 substituents selected from the group consisting of: (C₁-C₃)alkoxy, and amino.

The reaction in Scheme Ie can take place in the presence of catalyst comprising a platinum, rhodium, raney nickel, or palladium. Preferably, the catalyst is a palladium catalyst, including 10% palladium on carbon (Pd-C) and a hydrogen atmosphere. Preferably, the reaction takes place in one or more suitable solvents, such as methanol and MeOH/EtOAc (1:1). The reaction can take place at 1 atmosphere over 5-10% Pd-C. Preferably, the aforementioned reaction can take place in ≤ 48 h.

A detailed process for preparing Formula Ig or a salt thereof is further described, wherein Formula Ig is a subset of a compound of Formula I.

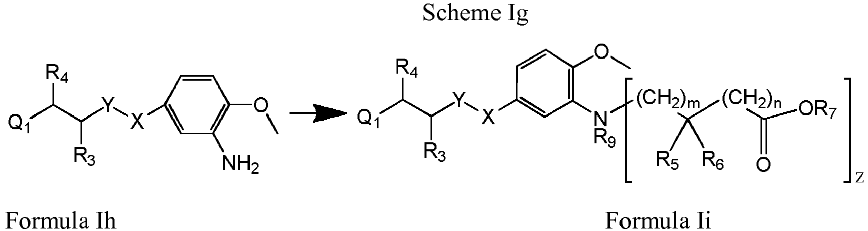

In Scheme Ig, X, Y, Q₁, R₃, R₄, R₅, R₆, R₇, R₉, m, n, and z are as defined above.

Particular options of the process in Scheme Ig include those wherein:
Q₁ is selected from the group consisting of substituted aryl and substituted heteroaryl, with up to 5 substituents selected from the group consisting of:
   fluoro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
R₅ and R₆ are each independently selected from the group consisting of: H; halo; and a C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₆) alkyl, (C₁-C₆) dialkyl amino, or acylamino; and R₇ is selected from the group consisting of: H; a C₁-C₁₀ saturated or unsaturated, straight or branched, cyclic or acyclic, chiral or achiral hydrocarbyl group; and an inorganic cation to form a salt .

In another option of Scheme Ig, Q₁ is substituted phenyl, with up to 5 (C₁-C₃)alkoxy substituents; and R₅ is H, R₆ is H, R₇ is methyl; R₉ is H; and m is 0, n is 0, and z is 1.

In another option of Scheme Ig, Q₁ is substituted phenyl, with up to 5 (C₁-C₃)alkoxy substituents; and R₅ is H, R₆ is H, R₇ is methyl; R₉ is [-(CH₂)ₘ-C(-R₅)(-R₆)-(CH₂)ₙ-COOR₇]; and m is 0, n is 0, and z is 1.

In another option of Scheme Ig, Q₁ is substituted phenyl, with up to 5 (C₁-C₃)alkoxy substituents; and R₅ is methyl; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is methyl; R₆ is methyl; R₇ H; R₉ is H; and m is 0, n is 0, and z is 1.

In another option of Scheme Ig, Q₁ is substituted phenyl, with up to 5 (C₁-C₃)alkoxy substituents; and R₅ is phenyl; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is 4-F-phenyl-; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is 4-Cl-phenyl-; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is -CH₂-phenyl; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is cyclopropyl; R₆ is H; R₇ H; R₉ is H; and m is 0, n is 0, and z is 1.

In another option of Scheme Ig, Q₁ is substituted phenyl, with up to 5 (C₁-C₃)alkoxy substituents; and R₅ is H; R₆ is H; R₇ is Na⁺; R₉ is H; and m is 0, n is 0, and z is 1.

A process of making a compound of Formula Ia or salt thereof, which is a subset of Formula I, is provided

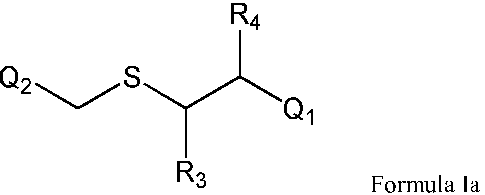

The process comprises a reaction step of:
reacting a compound of Formula AA; 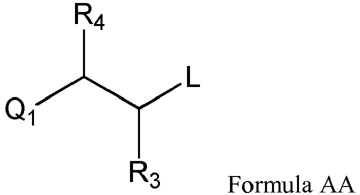 with a compound of Formula BB; 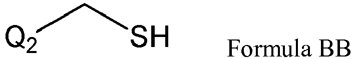 pto produce a compound of Formula Ia in a reaction mixture, and optionally isolating the compound of Formula Ia from the reaction mixture, wherein Q₁, Q₂, R₃, and R₄ are as defined above, and L is a leaving group selected from the group consisting of: Cl, Br, and I.

In an embodiment, the aforesaid reaction takes place in the presence of a base.

A process of making a compound of Formula Ib or salt thereof, which is a subset of Formula I, is provided.

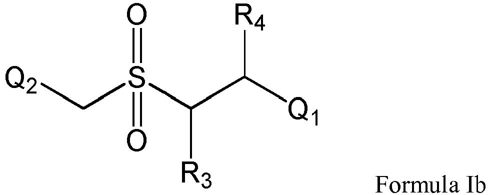

The process comprises a reaction step of:
oxidizing a compound of Formula Ia : 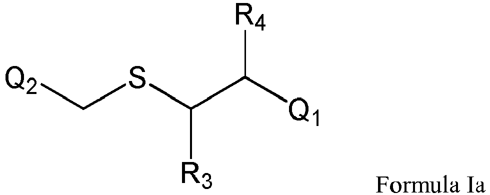 to produce a compound of Formula Ib in a reaction mixture, and optionally isolating compound Formula Ib from the reaction mixture, wherein Q₁, Q₂, R₃, and R₄ are as defined above.

In an embodiment, the aforesaid reaction takes place in the presence of an acid and a peroxide.

A process of making a compound of Formula Ic or salt thereof, which is a subset of Formula I, isdescribed:

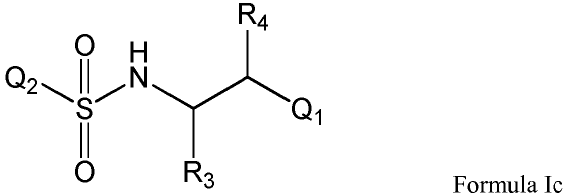

The process comprises a reaction step of:
reacting a compound of Formula CC; 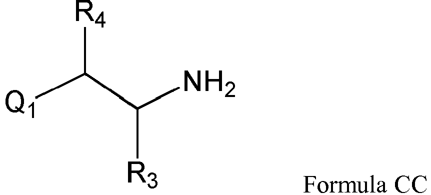 with a compound of Formula DD; 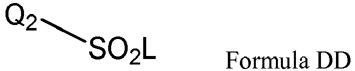 to produce a compound of Formula Ic in a reaction mixture, and optionally isolating the compound of Formula Ic from the reaction mixture, wherein Q₁, Q₂, R₃, and R₄ are as defined in above, and L is a leaving group selected from the group consisting of: Cl, Br, and I.

Optionally, the aforesaid reaction takes place in the presence of a base.

A process of making a compound of Formula Id or salt thereof, which is a subset of Formula I, is described. 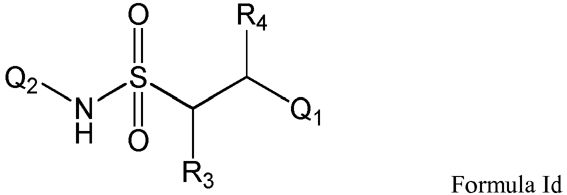 The process comprises a reaction step of:
reacting a compound of Formula EE: 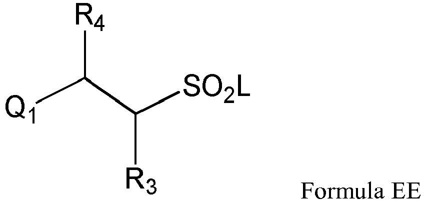 with a compound of Formula FF; 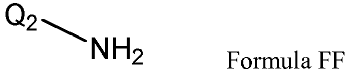 to produce a compound of Formula Id in a reaction mixture, and optionally isolating the compound of Formula Id from the reaction mixture, wherein Q₁, Q₂, R₃, and R₄ are as defined above, and L is a leaving group selected from the group consisting of: Cl, Br, and I.

Optionally, the aforesaid reaction takes place in the presence of a base.

A process of making a compound of Formula If or salt thereof, which is a subset of Formula I, is provided. 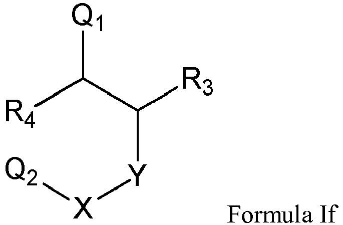 The process comprises a reaction step of:
reducing a compound of Formula Ie; 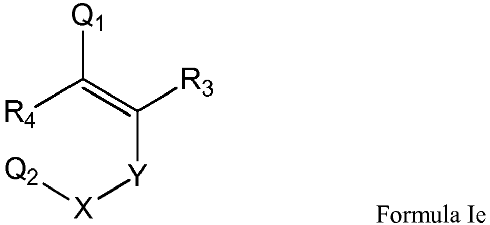 to produce a compound of Formula If in a reaction mixture, and optionally isolating the comopund of Formula Ie from the reaction mixture, wherein X, Y, Q₁, Q₂, R₃, and R₄ are as defined above.

In an embodiment, the aforesaid reaction takes place in the presence of a palladium catalyst.

A process of making a compound of Formula Ii or salt thereof, which is a subset of Formula I, is provided. 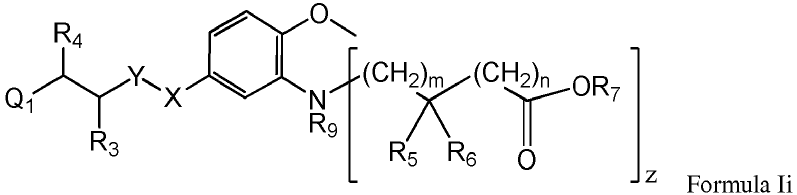 The process comprises a reaction step comprising:
reacting a compound of Formula Ih: 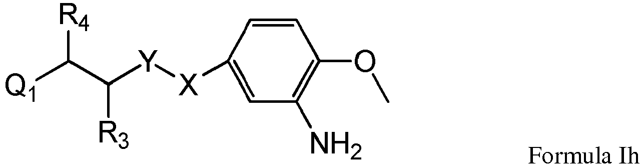 to produce a compound of Formula Ii in a reaction mixture, and optionally isolating the compound Formula Ii from the reaction mixture, wherein X, Y, Q₁, R₃, R₄, R₅, R₆, R₇, R₉, m, n, and z are as defined above.

In an embodiment, the aforesaid reaction takes place in the presence of a base and a compound L-CH₂-(C=O)-O-(C₁-C₆-alkyl) wherein L is a halogen.

### IV. Treatment of Cellular Proliferative Disorders Using Compounds of the Invention

A method of treating an individual suffering from a cellular proliferative disorder, particularly cancer, is described, comprising administering to said individual an effective amount of at least one compound according to Formula I, or a pharmaceutically acceptable salt thereof, either alone, or in combination with a pharmaceutically acceptable carrier.

Also described is a method of inducing apoptosis of cancer cells, preferably tumor cells, in an individual afflicted with cancer is provided, comprising administering to said individual an effective amount of at least one compound according to Formula I, or a pharmaceutically acceptable salt thereof, either alone, or in combination with a pharmaceutically acceptable carrier.

The compounds according to the invention may be administered to individuals (mammals, including animals and humans) afflicted with a cellular proliferative disorder such as cancer, malignant and benign tumors, blood vessel proliferative disorders, autoimmune disorders, and fibrotic disorders. In a particular embodiment of the invention, the individual treated is a human.

The compounds are believed effective against a broad range of tumor types, including but not limited to the following: ovarian cancer; cervical cancer; breast cancer; prostate cancer; testicular cancer, lung cancer, renal cancer; colorectal cancer; skin cancer; brain cancer; leukemia, including acute myeloid leukemia, chronic myeloid leukemia, acute lymphoid leukemia, and chronic lymphoid leukemia.

More particularly, cancers that may be treated by the compounds, compositions and methods of the invention include, but are not limited to, the following:
cardiac cancers, including, for example sarcoma, e.g., angiosarcoma, fibrosarcoma, rhabdomyosarcoma, and liposarcoma; myxoma; rhabdomyoma; fibroma; lipoma and teratoma;
lung cancers, including, for example, bronchogenic carcinoma, e.g., squamous cell, undifferentiated small cell, undifferentiated large cell, and adenocarcinoma; alveolar and bronchiolar carcinoma; bronchial adenoma; sarcoma; lymphoma; chondromatous hamartoma; and mesothelioma;
gastrointestinal cancer, including, for example, cancers of the esophagus, e.g., squamous cell carcinoma, adenocarcinoma, leiomyosarcoma, and lymphoma; cancers of the stomach, e.g., carcinoma, lymphoma, and leiomyosarcoma; cancers of the pancreas, e.g., ductal adenocarcinoma, insulinoma, glucagonoma, gastrinoma, carcinoid tumors, and vipoma; cancers of the small bowel, e.g., adenocarcinoma, lymphoma, carcinoid tumors, Kaposi's sarcoma, leiomyoma, hemangioma, lipoma, neurofibroma, and fibroma; cancers of the large bowel, e.g., adenocarcinoma, tubular adenoma, villous adenoma, hamartoma, and leiomyoma;
genitourinary tract cancers, including, for example, cancers of the kidney, e.g., adenocarcinoma, Wilm's tumor (nephroblastoma), lymphoma, and leukemia; cancers of the bladder and urethra, e.g., squamous cell carcinoma, transitional cell carcinoma, and adenocarcinoma; cancers of the prostate, e.g., adenocarcinoma, and sarcoma; cancer of the testis, e.g., seminoma, teratoma, embryonal carcinoma, teratocarcinoma, choriocarcinoma, sarcoma, interstitial cell carcinoma, fibroma, fibroadenoma, adenomatoid tumors, and lipoma;
liver cancers, including, for example, hepatoma, e.g., hepatocellular carcinoma; cholangiocarcinoma; hepatoblastoma; angiosarcoma; hepatocellular adenoma; and hemangioma;
bone cancers, including, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochrondroma (osteocartilaginous exostoses), benign chondroma, chondroblastoma, chondromyxofibroma, osteoid osteoma and giant cell tumors;
nervous system cancers, including, for example, cancers of the skull, e.g., osteoma, hemangioma, granuloma, xanthoma, and osteitis deformans; cancers of the meninges, e.g., meningioma, meningiosarcoma, and gliomatosis; cancers of the brain, e.g., astrocytoma, medulloblastoma, glioma, ependymoma, germinoma (pinealoma), glioblastoma multiform, oligodendroglioma, schwannoma, retinoblastoma, and congenital tumors; and cancers of the spinal cord, e.g., neurofibroma, meningioma, glioma, and sarcoma;
gynecological cancers, including, for example, cancers of the uterus, e.g., endometrial carcinoma; cancers of the cervix, e.g., cervical carcinoma, and pre-tumor cervical dysplasia; cancers of the ovaries, e.g., ovarian carcinoma, including serous cystadenocarcinoma, mucinous cystadenocarcinoma, unclassified carcinoma, granulosa-thecal cell tumors, Sertoli-Leydig cell tumors, dysgerminoma, and malignant teratoma; cancers of the vulva, e.g., squamous cell carcinoma, intraepithelial carcinoma, adenocarcinoma, fibrosarcoma, and melanoma; cancers of the vagina, e.g., clear cell carcinoma, squamous cell carcinoma, botryoid sarcoma, and embryonal rhabdomyosarcoma; and cancers of the fallopian tubes, e.g., carcinoma;
hematologic cancers, including, for example, cancers of the blood, e.g., acute myeloid leukemia, chronic myeloid leukemia, acute lymphoblastic leukemia, chronic lymphocytic leukemia, myeloproliferative diseases, multiple myeloma, and myelodysplastic syndrome, Hodgkin's lymphoma, non-Hodgkin's lymphoma (malignant lymphoma) and Waldenstrom's macroglobulinemia;
skin cancers, including, for example, malignant melanoma, basal cell carcinoma, squamous cell carcinoma, Kaposi's sarcoma, moles dysplastic nevi, lipoma, angioma, dermatofibroma, keloids, psoriasis; and
adrenal gland cancers, including, for example, neuroblastoma.

Cancers may be solid tumors that may or may not be metastatic. Cancers may also occur, as in leukemia, as a diffuse tissue. Thus, the term "tumor cell", as provided herein, includes a cell afflicted by any one of the above identified disorders.

The compounds are also believed useful in the treatment of non-cancer cellular proliferative disorders, that is, cellular proliferative disorders which are characterized by benign indications. Such disorders may also be known as "cytoproliferative" or "hyperproliferative" in that cells are made by the body at an atypically elevated rate. Non-cancer cellular proliferative disorders believed treatable by compounds according to the invention include, for example: hemangiomatosis in newborn, secondary progressive multiple sclerosis, atherosclerosis, neurofibromatosis, ganglioneuromatosis, keloid formation, Paget's disease of the bone, fibrocystic disease of the breast, uterine fibroids, Peyronie's disease, Dupuytren's disease, restenosis, benign proliferative breast disease, benign prostatic hyperplasia, X-linked lymphocellular proliferative disorder (Duncan disease), post-transplantation lymphocellular proliferative disorder (PTLD), macular degeneration, and retinopathies, such as diabetic retinopathies and proliferative vitreoretinopathy (PVR)

Other non-cancer cellular proliferative disorders believed treatable by compounds according to the invention include the presence of pre-cancerous lymphoproliferative cells associated with an elevated risk of progression to a cancerous disorder. Many non-cancerous lymphocellular proliferative disorders are associated with latent viral infections such as Epstein-Barr virus (EBV) and Hepatitis C. These disorders often begin as a benign pathology and progress into lymphoid neoplasia as a function of time.

A method of treating an individual suffering from a cellular proliferative disorder may comprise administering to the individual an effective amount of at least one compound, or a pharmaceutically acceptable salt thereof, according to Formula I.

In such a method, the compound of Formula I may be selected from the group consisting of 2-((2-methoxy-5-(((2,4,6-trimethoxyphenethyl)sulfonyl)methyl)phenyl)amino) acetic acid, and pharmaceutically acceptable salts thereof.

A preferable embodiment of a sodium salt of a compound of Formula I for use in treatment is sodium 2-((2-methoxy-5-(((2,4,6-trimethoxyphenethyl)sulfonyl)methyl)phenyl) amino)acetate.

In such a method, the cellular proliferative disorder may be selected from the group consisting of: cancer, malignant and benign tumors, blood vessel proliferative disorders, autoimmune disorders, and fibrotic disorders.

In such a method, the cellular proliferative disorder may be a cancer selected from the group consisting of: ovarian cancer; cervical cancer; breast cancer; prostate cancer; testicular cancer, lung cancer, renal cancer; colorectal cancer; skin cancer; brain cancer; and leukemia.

In such a method, the leukemia may be selected from the group consisting of: acute myeloid leukemia, chronic myeloid leukemia, acute lymphoid leukemia, and chronic lymphoid leukemia.

A method of inducing apoptosis of cancer cells in an individual afflicted with cancer comprises administering to the individual an effective amount of at least one compound, or a pharmaceutically acceptable salt thereof, according to Formula I.

Also described is the use of a compound according to Formula I or a salt thereof for therapy. In another aspect, the invention is directed to the use of a compound according to Formula I or a salt thereof for use in medicine.

Also described is the use of a compound according to Formula I or a pharmaceutically acceptable salt thereof for treating an individual suffering from a cellular proliferative disorder.

In another aspect, the invention is directed to the use of a compound according to Formula I or a pharmaceutically acceptable salt thereof for treating an individual suffering from a cellular proliferative disorder, wherein the cellular proliferative disorder is selected from the group consisting of cancer, malignant and benign tumors, blood vessel proliferative disorders, autoimmune disorders, and fibrotic disorders.

Also described is the use of a compound according to Formula I or a pharmaceutically acceptable salt thereof for treating an individual suffering from a cellular proliferative disorder, wherein the cellular proliferative disorder is a cancer selected from the group consisting of: ovarian cancer; cervical cancer; breast cancer; prostate cancer; testicular cancer, lung cancer, renal cancer; colorectal cancer; skin cancer; brain cancer; and leukemia.

Also described is the use of a compound according to Formula I or a pharmaceutically acceptable salt thereof for treating an individual suffering from a cellular proliferative disorder, wherein the cellular proliferative disorder is a leukemia is selected from the group consisting of: acute myeloid leukemia, chronic myeloid leukemia, acute lymphoid leukemia, and chronic lymphoid leukemia.

Another aspect of the invention is directed to a compound of Formula I or a pharmaceutically acceptable salt thereof for use in treatment of a cellular proliferative disorder.

Another aspect of the invention is directed to a compound of Formula I or a pharmaceutically acceptable salt thereof for use in treatment of a cellular proliferative disorder wherein the cellular proliferative disorder is selected from the group consisting of cancer, malignant and benign tumors, blood vessel proliferative disorders, autoimmune disorders, and fibrotic disorders.

Another aspect of the invention is directed to a compound of Formula I or a pharmaceutically acceptable salt thereof for use in treatment of a cellular proliferative disorder wherein the cellular proliferative disorder is a cancer selected from the group consisting of: ovarian cancer; cervical cancer; breast cancer; prostate cancer; testicular cancer, lung cancer, renal cancer; colorectal cancer; skin cancer; brain cancer; and leukemia.

Another aspect of the invention is directed to a compound of Formula I or a pharmaceutically acceptable salt thereof for use in treatment of a cellular proliferative disorder, wherein the cellular proliferative disorder is a leukemia is selected from the group consisting of: acute myeloid leukemia, chronic myeloid leukemia, acute lymphoid leukemia, and chronic lymphoid leukemia.

### V. Salts of Compounds According to the Invention

The compounds of the present invention may take the form of salts when appropriately substituted with groups or atoms capable of forming salts. Such groups and atoms are well known to those of ordinary skill in the art of organic chemistry. The term "salts" embraces addition salts of free acids or free bases which are compounds of the invention. The term "pharmaceutically acceptable salt" refers to salts which possess toxicity profiles within a range that affords utility in pharmaceutical applications. Pharmaceutically unacceptable salts may nonetheless possess properties such as high crystallinity, which have utility in the practice of the present invention, such as for example utility in process of synthesis, purification or formulation of compounds of the invention.

Suitable pharmaceutically-acceptable acid addition salts may be prepared from an inorganic acid or from an organic acid. Examples of inorganic acids include hydrochloric, hydrobromic, hydriodic, nitric, carbonic, sulfuric, and phosphoric acids. Appropriate organic acids may be selected from aliphatic, cycloaliphatic, aromatic, araliphatic, heterocyclic, carboxylic and sulfonic classes of organic acids, examples of which include formic, acetic, pivalic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid. Examples of pharmaceutically unacceptable acid addition salts include, for example, perchlorates and tetrafluoroborates.

Suitable pharmaceutically acceptable base addition salts of compounds of the invention include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, *N*,*N*'-dibenzylethylenediamine, chloroprocaine, choline, diethanolamine, ethylenediamine, tromethamine, meglumine (N-methylglucamine) and procaine. Examples of pharmaceutically unacceptable base addition salts include lithium salts and cyanate salts.

All of these salts may be prepared by conventional means from the corresponding compound according to Formula I by reacting, for example, the appropriate acid or base with the compound according to Formula I. Preferably the salts are in crystalline form, and preferably prepared by crystallization of the salt from a suitable solvent. The person skilled in the art will know how to prepare and select suitable salt forms for example, as described in Handbook of Pharmaceutical Salts: Properties, Selection, and Use By P. H. Stahl and C. G. Wermuth (Wiley-VCH 2002).

### VI. Pharmaceutical Compositions

A pharmaceutical composition comprises a pharmaceutically acceptable carrier and a compound, or a pharmaceutically acceptable salt thereof, according to any of Formula I.

The compounds of the invention may be administered in the form of a pharmaceutical composition, in combination with a pharmaceutically acceptable carrier. The active ingredient or agent in such formulations (i.e. a compound of Formula I) may comprise from 0.1 to 99.99 weight percent of the formulation. "Pharmaceutically acceptable carrier" means any carrier, diluent or excipient which is compatible with the other ingredients of the formulation and not deleterious to the recipient.

The active agent is preferably administered with a pharmaceutically acceptable carrier selected on the basis of the selected route of administration and standard pharmaceutical practice. The active agent may be formulated into dosage forms according to standard practices in the field of pharmaceutical preparations. See Alphonso Gennaro, ed., Remington's Pharmaceutical Sciences, 18th Edition (1990), Mack Publishing Co., Easton, PA. Suitable dosage forms may comprise, for example, tablets, capsules, solutions, parenteral solutions, troches, suppositories, or suspensions.

For parenteral administration, the active agent may be mixed with a suitable carrier or diluent such as water, an oil (particularly a vegetable oil), ethanol, saline solution, aqueous dextrose (glucose) and related sugar solutions, glycerol, or a glycol such as propylene glycol or polyethylene glycol. Solutions for parenteral administration preferably contain a water soluble salt of the active agent. Stabilizing agents, antioxidant agents and preservatives may also be added. Suitable antioxidant agents include sulfite, ascorbic acid, citric acid and its salts, and sodium EDTA. Suitable preservatives include benzalkonium chloride, methyl- or propyl-paraben, and chlorbutanol. The composition for parenteral administration may take the form of an aqueous or non-aqueous solution, dispersion, suspension or emulsion.

For oral administration, the active agent may be combined with one or more solid inactive ingredients for the preparation of tablets, capsules, pills, powders, granules or other suitable oral dosage forms. For example, the active agent may be combined with at least one excipient such as fillers, binders, humectants, disintegrating agents, solution retarders, absorption accelerators, wetting agents absorbents or lubricating agents. According to one tablet embodiment, the active agent may be combined with carboxymethylcellulose calcium, magnesium stearate, mannitol and starch, and then formed into tablets by conventional tableting methods.

The specific dose of a compound according to the invention to obtain therapeutic benefit for treatment of a cellular proliferative disorder will, of course, be determined by the particular circumstances of the individual patient including the size, weight, age and sex of the patient, the nature and stage of the cellular proliferative disorder, the aggressiveness of the cellular proliferative disorder, and the route of administration of the compound.

For example, a daily dosage from about 0.05 to about 50 mg/kg/day may be utilized, more preferably from about 0.1 to about 10 mg/kg/day. Higher or lower doses are also contemplated as it may be necessary to use dosages outside these ranges in some cases. The daily dosage may be divided, such as being divided equally into two to four times per day daily dosing. The compositions are preferably formulated in a unit dosage form, each dosage containing from about 1 to about 500mg, more typically, about 10 to about 100mg of active agent per unit dosage. The term "unit dosage form" refers to physically discrete units suitable as a unitary dosage for human subjects and other mammals, each unit containing a predetermined quantity of active material calculated to produce the desired therapeutic effect, in association with a suitable pharmaceutical excipient.

The pharmaceutical compositions of the present invention may also be formulated so as to provide slow or controlled release of the active ingredient therein using, for example, hydropropylmethyl cellulose in varying proportions to provide the desired release profile, other polymer matrices, gels, permeable membranes, osmotic systems, multilayer coatings, microparticles, liposomes and/or microspheres.

In general, a controlled-release preparation is a pharmaceutical composition capable of releasing the active ingredient at the required rate to maintain constant pharmacological activity for a desirable period of time. Such dosage forms provide a supply of a drug to the body during a predetermined period of time and thus maintain drug levels in the therapeutic range for longer periods of time than conventional non-controlled formulations.

U.S. Patent No. 5,674,533 discloses controlled-release pharmaceutical compositions in liquid dosage forms for the administration of moguisteine, a potent peripheral antitussive. U.S. Patent No. 5,059,595 describes the controlled-release of active agents by the use of a gastro-resistant tablet for the therapy of organic mental disturbances. U.S. Patent No. 5,591,767 describes a liquid reservoir transdermal patch for the controlled administration of ketorolac, a non-steroidal anti-inflammatory agent with potent analgesic properties. U.S. Patent No. 5,120,548 discloses a controlled-release drug delivery device comprised of swellable polymers. U.S. Patent No. 5,073,543 describes controlled-release formulations containing a trophic factor entrapped by a ganglioside-liposome vehicle. U.S. Patent No. 5,639,476 discloses a stable solid controlled-release formulation having a coating derived from an aqueous dispersion of a hydrophobic acrylic polymer. Biodegradable microparticles are known for use in controlled-release formulations. U.S. Patent No. 5,733,566 describes the use of polymeric microparticles that release antiparasitic compositions.

The controlled-release of the active ingredient may be stimulated by various inducers, for example pH, temperature, enzymes, water, or other physiological conditions or compounds. Various mechanisms of drug release exist. For example, in one embodiment, the controlled-release component may swell and form porous openings large enough to release the active ingredient after administration to a patient. The term "controlled-release component" in the context of the present invention is defined herein as a compound or compounds, such as polymers, polymer matrices, gels, permeable membranes, liposomes and/or microspheres, that facilitate the controlled-release of the active ingredient in the pharmaceutical composition. In another embodiment, the controlled-release component is biodegradable, induced by exposure to the aqueous environment, pH, temperature, or enzymes in the body. In another embodiment, sol-gels may be used, wherein the active ingredient is incorporated into a sol-gel matrix that is a solid at room temperature. This matrix is implanted into a patient, preferably a mammal, having a body temperature high enough to induce gel formation of the sol-gel matrix, thereby releasing the active ingredient into the patient.

The components used to formulate the pharmaceutical compositions are of high purity and are substantially free of potentially harmful contaminants (e.g., at least National Food grade, generally at least analytical grade, and more typically at least pharmaceutical grade). Particularly for human consumption, the composition is preferably manufactured or formulated under Good Manufacturing Practice standards as defined in the applicable regulations of the U.S. Food and Drug Administration. For example, suitable formulations may be sterile and/or substantially isotonic and/or in full compliance with all Good Manufacturing Practice regulations of the U.S. Food and Drug Administration.

### VII. Routes of Administration of Compounds and Compositions of the Invention

The compounds of Formula I, including pharmaceutically acceptable salts thereof, may be administered by any route, including oral, rectal, sublingual, and parenteral administration. Parenteral administration includes, for example, intravenous, intramuscular, intraarterial, intraperitoneal, intranasal, intravaginal, intravesical (e.g., to the bladder), intradermal, transdermal, topical or subcutaneous administration. Also contemplated within the scope of the invention is the instillation of a drug in the body of the patient in a controlled formulation, with systemic or local release of the drug to occur at a later time. For example, the drug may be localized in a depot for controlled release to the circulation, or for release to a local site of tumor growth.

One or more compounds useful in the practice of the present inventions may be administered simultaneously, by the same or different routes, or at different times during treatment. The compounds may be administered before, along with, or after other medications, including other antiproliferative compounds.

The treatment may be carried out for as long a period as necessary, either in a single, uninterrupted session, or in discrete sessions. The treating physician will know how to increase, decrease, or interrupt treatment based on patient response. According to one embodiment, treatment is carried out for from about four to about sixteen weeks. The treatment schedule may be repeated as required.

### VIII. Isomerism in Compounds of the Invention

### A. Optical Isomerism

It will be understood that when or if compounds of the present invention contain one or more chiral centers, the compounds may exist in, and may be isolated as pure enantiomeric or diastereomeric forms or as racemic mixtures. The present invention therefore includes any possible enantiomers, diastereomers, racemates or mixtures thereof of the compounds of the invention.

The isomers resulting from the presence of a chiral center comprise a pair of non-superimposable isomers that are called "enantiomers." Single enantiomers of a pure compound are optically active, *i.e.,* they are capable of rotating the plane of plane polarized light. Single enantiomers are designated according to the *Cahn-Ingold-Prelog* system. Once the priority ranking of the four groups is determined, the molecule is oriented so that the lowest ranking group is pointed away from the viewer. Then, if the descending rank order of the other groups proceeds clockwise, the molecule is designated (*R)* and if the descending rank of the other groups proceeds counterclockwise, the molecule is designated (*S)*. In the example below, the *Cahn-Ingold-Prelog* ranking is A > B > C > D. The lowest ranking atom, D is oriented away from the viewer.

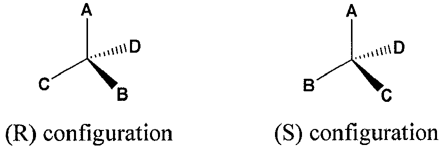

The present invention is meant to encompass diastereomers as well as their racemic and resolved, diastereomerically and enantiomerically pure forms and salts thereof. Diastereomeric pairs may be resolved by known separation techniques including normal and reverse phase chromatography, and crystallization.

"Isolated optical isomer" means a compound which has been substantially purified from the corresponding optical isomer(s) of the same formula. Preferably, the isolated isomer is at least about 80%, more preferably at least 90% pure, even more preferably at least 98% pure, most preferably at least about 99% pure, by weight.

Isolated optical isomers may be purified from racemic mixtures by well-known chiral separation techniques. According to one such method, a racemic mixture of a compound having the structure of Formula I, or a chiral intermediate thereof, is separated into 99% wt.% pure optical isomers by HPLC using a suitable chiral column, such as a member of the series of DAICEL® CHIRALPAK® family of columns (Daicel Chemical Industries, Ltd., Tokyo, Japan). The column is operated according to the manufacturer's instructions.

### B. Rotational Isomerism

It is understood that due to chemical properties (*i.e.,* resonance lending some double bond character to the C-N bond) of restricted rotation about the amide bond linkage (as illustrated below) it is possible to observe separate rotamer species and even, under some circumstances, to isolate such species. It is further understood that certain structural elements, including steric bulk or substituents on the amide nitrogen, may enhance the stability of a rotamer to the extent that a compound may be isolated as, and exist indefinitely, as a single stable rotamer. The present invention therefore includes any possible stable rotamers of Formula I.

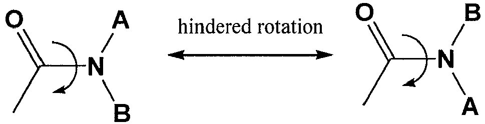

### IX. Examples

The following non-limiting examples are provided to illustrate the invention. The synthetic procedures described as "general methods" describe what it is believed will be typically effective to perform the synthesis indicated. However, the person skilled in the art will appreciate that it may be necessary to vary the procedures for any given embodiment of the invention. For example, reaction monitoring, such as by using thin layer chromatography, or HPLC may be used to determine the optimum reaction time. Products may be purified by conventional techniques that will vary, for example, according to the amount of side products produced and the physical properties of the compounds. On a laboratory scale, recrystallization from a suitable solvent, column chromatography, normal or reverse phase HPLC, or distillation are all techniques which may be useful. The person skilled in the art will appreciate how to vary the reaction conditions to synthesize any given compound within the scope of the invention without undue experimentation. *See, e.g.,* Vogel's Textbook of Practical Organic Chemistry, by A. I. Vogel, et al, Experimental Organic Chemistry: Standard and Microscale, by L. M. Harwood et al. (2nd Ed., Blackwell Scientific Publications, 1998), and Advanced Practical Organic Chemistry, by J. Leonard, et al. (2nd Edition, CRC Press 1994).

### Scheme 1

General Procedures I-VI are used in preparing Compounds 1-7 in Scheme 1.

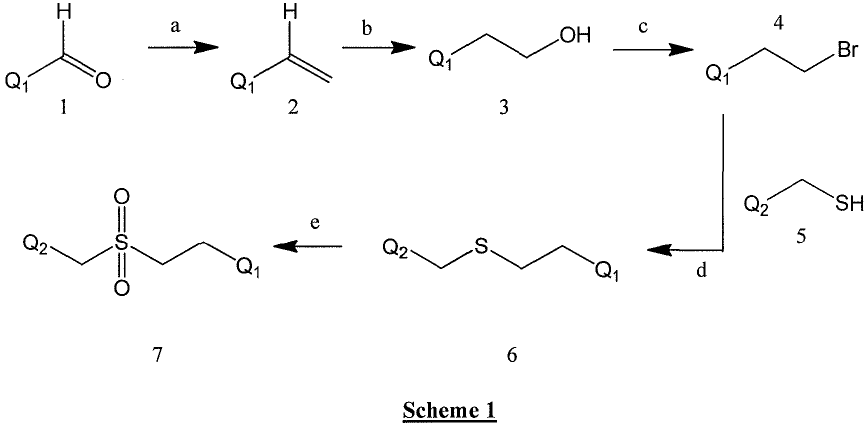

Non-limiting embodiments of reaction conditions for each step may include: a) ⁺[Ph₃PCH₃] ⁻Br, t-BuOK, THF, O°C-r.t., 2.5 hours; b) i. 9-Borabicyclo(3.3.1)nonane, THF, O°-r.t., 18 hours; ii. H₂O₂, 3N NaOH, 3 hours; c) CBr₄, PPh₃, 2 hours; d) NaOH, MeOH, 2 hours; and e) H₂O₂, AcOH, 24 hours.

### General Procedure I:

### Preparation of Compound 2 of Scheme 1: Substituted styrenes.

To the mixture of methyltriphenylphosphonium bromide (47.0 mmol) and potassium tert-butoxide (53.0 mmol), dry tetrahydrofuran ("THF") (36 mL) is added at room temperature under nitrogen. To the reaction mixture, an appropriate amount of substituted benzaldehyde 1 (45.0 mmol) is dissolved in dry THF (18 mL) and added dropwise for 90 min under cooling in an ice bath. Then the reaction is kept stirring for 60 min. The reaction mixture is poured into cold water, and the organic layer is separated. The aqueous mixture is extracted three times with diethyl ether. The combined organic layer is dried over anhydrous MgSO₄. After evaporation, the layer is poured into a large amount of n-hexane to precipitate triphenylphosphine oxide. After filtration, the filtrate is evaporated. The concentrated liquid is distilled under vacuum to give a colorless liquid of substituted styrene **2** (74-86%).

### General Procedure II:

### Preparation of Compound 3 of Scheme 1: substituted 2-phenyethanols.

To an ice-cold solution of substituted styrene **2** (12.7 mmol) in THF (40 mL) is added 9-Borabicyclo(3.3.1)nonane ("9-BBN") (0.5 M in THF, 25 mmol). The reaction is conducted at ambient temperature for 18 h and quenched by addition of MeOH (40 mL). After 30 min of stirring, 3 N NaOH (40 mL, 120 mmol) and 35% H₂O₂ (75 mmol) are added slowly. The resulting mixture is stirred at room temperature for 3 hours and poured into H₂O. The product is extracted with ether twice. The combined organic layers are washed with brine, dried over MgSO₄, and concentrated to afford a residual oil, which is purified by chromatography on silica gel with hexane/EtOAc (from 5:1 to 1:2) to afford substituted 2-phenyethanols **3** (85-90%) as colorless solids.

### General Procedure III

### Preparation of Compound 4 of Scheme 1: Substituted (2-bromoethyl)benzenes.

To an ice-cold solution of 2-phenyethanols **3** (11.1 mmol) and PPh₃ (13.3 mmol) in CH₂Cl₂ (20 mL) is added CBr₄ (12.2 mmol). The mixture is stirred at room temperature for 2 hours and concentrated to afford a residual oil, which is purified by chromatography on silica gel with hexane/EtOAc (10:1) to afford substituted (2-bromoethyl)benzenes **4** (80-94%).

### General Procedure IV

### Preparation of Compound 5 of Scheme 1: substituted phenylmethanethiols.

A solution of substituted benzylbromide (41 mmol) and thiourea (55 mmol) in 50 mL water is heated under reflux for 2 hours. The reaction mixture is cooled and stirred at roomtemperature for 2 hours, and the solid is filtered, dried, and the resulting dried intermediate, isothiouronium salt, is used in next step without further purification. The yield of this reaction is 90%.

The above isothiouronium salt (10g) is decomposed by boiling three times with ammonium hydroxide and hexane (100 mL, 15:85) followed by three times with ammonium hydroxide, ethyl acetate, and hexane (100 mL, 15: 5: 80). Concentration of the combined extracts provides crude substituted phenylmethanethiol **5,** which is purified by silica gel flash column chromatography (hexane/ethylacetate, 4:1). The yield of this reaction is 25%.

### General Procedure V

### Preparation of Compound 6 of Scheme 1: substituted benzyl(substituted phenethyl)sulfanes.

To a cooled solution of sodium hydroxide (100 mmol) in absolute methanol (50 mL), arylmethanethiol **5** (100mmol) is added slowly and the reaction mixture is stirred for 5 min. An appropriate substituted (2-bromoethyl)benzene **4** (100 mmol) is added in portions to the contents of the flask, and the mixture is stirred for 2 h. After completion of the reaction (monitored by thin-layer chromatography ("TLC"), the contents of the flask are poured into crushed ice with stirring, filtered the compound formed, washed with ice-cold water and dried to get the compounds **6** of scheme 1 as white solids (82-95%).

### General Procedure VI

### Preparation of Compound 7 of Scheme 1: (2-(substituted benzylsulfonyl)ethyl) substituted benzenes.

To the substituted benzyl(substituted phenethyl)sulfanes **6** (50 mmol) in glacial acetic acid (100 mL) is added 30% hydrogen peroxide (60 mL) in portions at frequent intervals. Then the reaction mixture is kept at room temperature for 24 hours. The solid, if any formed, is separated by filtration, and the filtrate is poured onto crushed ice. The separated compound is filtered, washed with water, dried, and added to the first crop, if any. The total product on recrystallization from methanol affords pure (2-(substituted benzylsulfonyl)ethyl) substituted benzenes 7, (72-76%) as white solid.

### Example 1

### 4-Chlorobenzyl-4-fluorophenethylsulfane

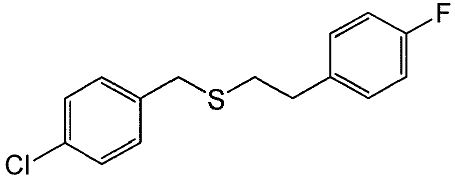

The title compound was prepared according to Scheme 1. A solution of 0.01 mole sodium hydroxide in methanol 30 mL was taken in a 100 mL round-bottomed flask and cooled 10 °C. To the cooled solution, 0.01 mole of 4-chlorobenzyl thiol was added slowly to the contents of the flask. A vigorous reaction occurred immediately. On completion of the addition and when the reaction was no longer exothermic, 1-(2-chloroethyl)-4-fluorobenzene (0.01 mole) was added portion wise, and the reaction mixture was stirred at room temperature for 3 h. The reaction was checked by TLC (thin layer chromatography) for completion. After completion, the reaction mixture was poured into crushed ice. The compound formed was filtered, washed with ice-cold water, and dried. The procedure according to General Procedure V produced the title compound with a yield of 90% and melting point 36-38 °C.

### Example 2

### 1-Chloro-4-(((4-fluorophenethyl)sulfonyl)methyl)benzene

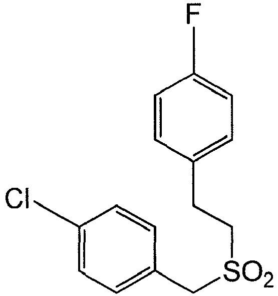

The title compound was prepared according to Scheme 1. An ice-cold solution of 4-chlorobenzyl-4-fluorophenethylsulfane (4.0 g) in glacial acetic acid (25 mL) was added to a 100 mL round-bottomed flask. 30% hydrogen peroxide (8.0 mL) was added to the mixture at frequent intervals. Then, the reaction mixture was kept at room temperature for 24 h. The mixture was poured onto crushed ice. The solid formed was filtered, washed with ice-cold water, and dried. The procedure according to Reaction VI produced the title compound with a yield of 80% and a melting point of 142-144 °C.

¹H NMR (CDCl₃, 300 MHz): δ 3.55-3.60 (m, 2H, CH₂), 3.75-3.82 (m, 2H, CH₂), 4.30 (s, 2H, CH₂), 6.92-7.10 (m, 6H, Ar-H), 7.15 (d, J = 8.1 Hz, 1H, Ar-H).). HRMS: m/z calcd [M + H] 313.69; found 313.40.

### Scheme 2

### General Procedures VII-XI are used to prepare Compounds 1-5 of Scheme 2.

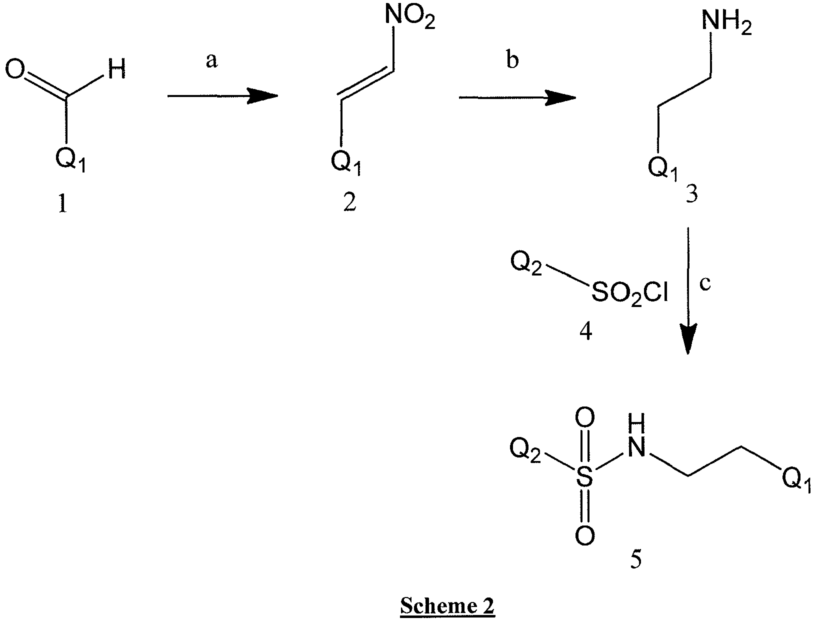

Non-limiting embodiments of reaction conditions for each step may include: a) CH₃NO₂, NH₄OAC, AcOH, EtOH, reflux; b) LiAlH₄, Et₂O, THF; and c) anhydrous acetone/ pyridine, overnight or CH₂Cl₂/Et₃N, rt.

### General Procedure VII

### Preparation of Compound 1 of Scheme 2: substituted benzaldehyde.

A 500 mL multi-necked flask is equipped with a reflux condenser, stirrer, a gas inlet, and thermometer. 0.425 mol of substituted benzene, 0.625 mol of zinc cyanide, and 200 mL of 1,1,2,2- tetrachloroethane, is placed in the flask and the mixture is stirred while a rapid stream of dry hydrogen chloride is passed through it until the zinc cyanide is decomposed. The flask is immersed in a bath of crushed ice, the inlet tube is removed and 197 g of finely ground, anhydrous aluminum chloride is added over a period of 10 minutes. The mixture is stirred very vigorously and removed from the ice bath after addition, and the passage of hydrogen chloride gas is resumed for 3.5 h, while heating the reaction mass to about 70 °C. The temperature at 67-72 °C is maintained until the reaction is complete. After completion of the reaction, the reaction mixture is cooled and poured into a mixture of crushed ice and 50 mL of concentrated hydrochloric acid. The reaction mixture is allowed to stand overnight and then refluxed for 3 h. The reaction mixture is allowed to cool and separate into an organic layer and extract aqueous layer with tetrachloroethane. The organic layer is washed with 10% sodium carbonate solution and evaporated to get crude substituted benzaldehyde, which on purification results in pure substituted benzaldehyde **1.** The yield is about 79%.

### General Procedure VIII

### Preparation of Compound 2 of Scheme 2: substituted (E)-(2-nitrovinyl)benzenes.

A solution of (0.50 mol) of substituted benzaldehyde **1,** (1.55 mol) of nitromethane, and (0.50 mol) of ammonium acetate in 400 mL glacial acetic acid is refluxed for 2 h and then poured into an ice-water mixture. This gives a solid, which is collected by filtration and recrystallized from ethanol to afforded pure substituted (E)-(2-nitrovinyl) benzenes **2,** (80-82%).

### General Procedure IX

### Preparation of Compound 3 of Scheme 2: substituted 2-phenylethanamines.

A solution of (0.421 mol) of substituted (E)-(2-nitrovinyl) benzenes **2** in 500 mL of THF is added slowly to a slurry of (0.782 mol) of LiAlH₄ in 400 mL of Et₂O under nitrogen atmosphere. After the addition is complete, the mixture is refluxed for 1.5 h and then 140 mL of 1N NaOH is added and the mixture is filtered. The solid is extracted with 200 mL of boiling THF. The THF extract and the filtrate are combined and concentrated under vacuum. The residue is dissolved in Et₂O, which is dried over K₂CO₃, and then concentrated to give crude substituted 2-phenylethanamines **3,** which on purification gives 55% of the desired product.

### General Procedure X

### Preparation of Compound 4 of Scheme 2: substituted benzenesulfonyl chloride.

A mixture of substituted benzene (1.09 mol) in chloroform (500 mL) is magnetically stirred at O °C while chlorosulphonic acid (3.44 mol) is added for 15 minutes under cooling and exclusion of moisture. The stirring is continued for 45 minutes at room temperature and the mixture is poured on to crushed ice. The product is extracted with chloroform, dried over anhydrous sodium sulphate, and freed from solvent *in vacuo* at 30 °C. The residue is dissolved in warm pentane, and the solution is filtered and concentrated to 2/3 and upon standing. The substituted benzenesulfonyl chloride **4** is crystallized, filtered and washed with -30 °C chilled pentane, then dry under phosphorus pentoxide to provide an 86% yield.

### General Procedure XI

### Preparation of Compound 5 of Scheme 2: N-substituted phenethyl substituted benzenesulfonamides.

0.05 mol of substituted 2-phenylethanamine **3** is dissolve in a mixture of 40 mL of anhydrous acetone and 6 mL of dry pyridine. To the mixture, 0.05 mol of substituted benzenesulfonyl chloride **4** add is added. The reaction mixture is set aside overnight and the solid formed filtered to provide crude N-substituted phenethyl substituted benzenesulfonamides **5.** Pure substituted phenethyl substituted benzenesulfonamides **5** are obtained upon on recrystallization result with a 55% yield.

### Scheme 4

General Procedures XV-XVIII are used to prepare Compounds 1-5 of Scheme 4.

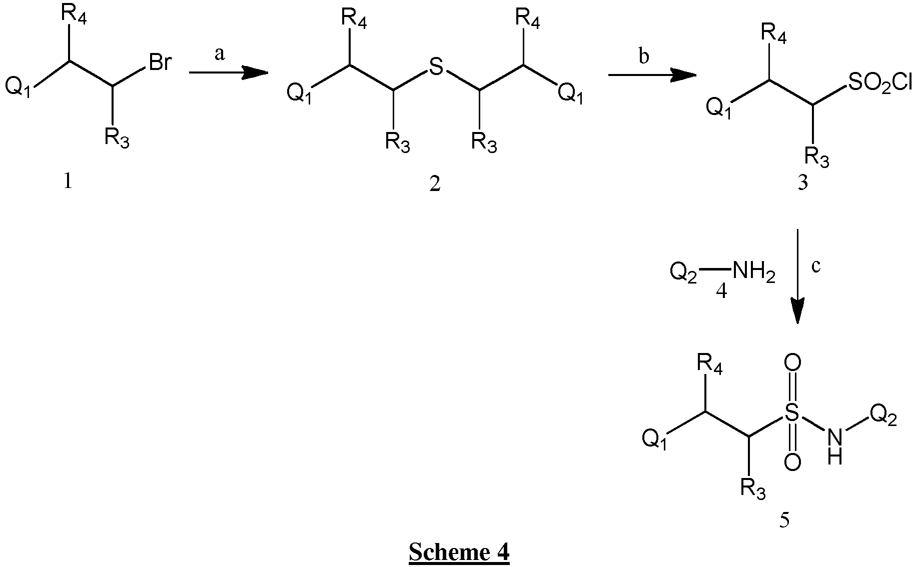

Non-limiting embodiments of reaction conditions for each step may include: a) Na₂S, EtOH, and 2 hours; b) iodosobenzene and hydrogen chloride-treated silica gel; and c) pyridine, anhydrous acetone.

### General Procedure XV

### Preparation of Compound 1 of Scheme 4: (2-bromoethyl)substituted benzenes.

Compound 1 of Scheme 4 can be prepared by following General Procedures I-III above.

### General Procedure XVI

### Preparation of Compound 2 of Scheme 4: substituted diphenethylsulfanes.

Sodium sulphide (0.5 mole) is placed in 500 mL ethanol. The mixture is heated on a water bath until the sulphide dissolves. 0.5 mole of finely powdered sulfur is added and the heating is continued until all the sulfur dissolves. A solution of 0.66 mole of substituted 2-bromoethylbenzenes prepared and added to 175 mL ethanol. To this mixture, the sodium disulphide solution is slowly added at such a rate that the reaction is under control. The mixture is heated on a water bath gently and then water is boiled vigorously for 2 hours. The reaction mixture is cooled, filtered, washed with ethanol, and dried to produce a substituted diphenethylsulfanes **2** with a yield: 68%.

### General Procedure XVII

### Preparation of Compound 3 of Scheme 4: substituted 2-phenylethanesulfonyl chlorides.

The substituted diphenethylsulfanes **2** are pulverized with iodosobenzene and hydrogen chloride-treated silica gel (HCl-silica gel) to yield substituted-2-phenylethane sulfonyl chlorides **3** by simultaneously oxidation and chlorination.

### General Procedure XVIII

### Preparation of Compound 5 of Scheme 4: substituted N,2-diphenylethane sulfonamides.

Substituted anilines **4** (0.05 mol) are dissolved in a mixture of 40 mL of anhydrous acetone and 6 mL of dry pyridine. 0.05 mol of substituted 2-phenylethanesulfonyl chlorides **3** is added. The reaction mixture is set aside overnight and the solid that forms is filtered to obtain crude **5,** which on recrystallization result pure substituted N-2-diphenyiethane sulfonamides for a yield of 55%.

### Scheme 5

General Procedure XIX is used to prepare Compounds 1 and 2 of Scheme 5. 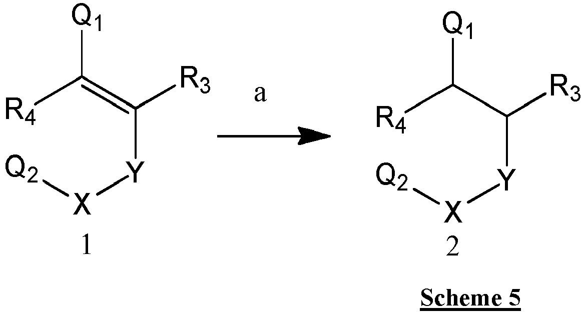 Non-limiting embodiments of reaction conditions for each step may include: 10% PD-C, H₂, EtOAc/MeOH, 24-48 hours.

### General Procedure XIX

Reduction of unsaturated Compound **1** of Scheme 5 with 10% Pd-C/H₂ is carried out as follows. A solution of Compound **1** (1.5 mmol) in a mixture of MeOH/EtOAc (40 mL) is treated with wet 10% palladium on carbon (50 mg) then exposed to hydrogen atmosphere by using hydrogenation apparatus. After completion of the reaction (monitored by TLC), the reaction mixture is filtered through Celite, washed the Celite pad thoroughly with a mixture of methanol and chloroform (50%) and the filtrate evaporated under reduced pressure. The filtered solid is washed with ether and dried under vacuum to obtain pure Compound **2** of scheme 5.

Sulfones of Compound **1** [Y= -S(=O)₂-; X= -CH(-R₂)- or X= -S(=O)₂-; Y= -CH(-R₂)-] of Scheme 5 can be prepared as disclosed in U.S. Patents 6,201,154; 6,359,013; 7,598,232; 6,762,207; 6,541,475; 6,486,210; and 7,161,031. Sulfones [Y= -S(=O)₂-; X= -CH(-R₂)- or X= -S(=O)₂-; Y= -CH(-R₂)-], Sulfoxides [Y= -S(=O)-; X= -CH(-R₂)- or X= -S(=O)-; Y= -CH(-R₂)-], Sulfides [Y= -S-; X= -CH(-R₂)- or X= -S-; Y= -CH(-R₂)-], and Sulfonamides [Y= -S(=O)₂-; X= -N(-R₁)- or X= -S(=O)₂-; Y= -N(-R₁)-] of Compound **1** of Scheme 5 can be prepared as disclosed in U.S. Patent Appl. Publs. 2009/0124828 and 2008/058290. Sulfoxides [Y= -S(=O)-; X= -CH(-R₂)- or X= -S(=O)-; Y= -CH(-R₂)-] of Compound **1** of Scheme 5 can be prepared as disclosed in U.S. Patent Appl. Publs. 2006/0280746. Amides of Compound **1** [Y= -N(-R₁)-; X= -C(=O)₂- or X= -N(-R₁)-; Y= -C(=O)₂-] of Scheme 5 can be prepared as disclosed in U.S. Patent Appl. Publs. 2006/0167317. Sulfonamides of Compound 1 [Y= -S(=O)₂-; X= -N(-R₁)- or X= -S(=O)₂-; Y= -N(-R₁)-] of Scheme 5 can be prepared as in U.S. Patent Appl. Publs. 2002/0165412,.

### Example 3

### 2-Methoxy-5-(((2,4,6-trimethoxyphenethyl)sulfonyl)methyl)aniline

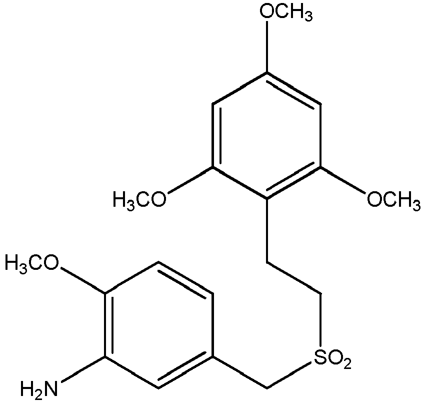

The title compound was prepared according to Scheme 5. A solution of (E)-2-methoxy-5-((2,4,6-trimethoxystyrylsulfonyl)methyl)aniline (1.5 mmol) in a mixture of MeOH (60 mL) was treated with wet 5% palladium on carbon (50 mg) then exposed to a hydrogen atmosphere by using hydrogenation apparatus. After completion of the reaction (monitored by TLC), the reaction mixture was filtered through Celite, the Celite pad was washed thoroughly with methanol, and the filtrate evaporated under reduced pressure and dried under vacuum to obtain the title compound according to General Procedure XIX with a yield of 95% and had a melting point: 128-130 °C.

¹H NMR (CDCl₃, 300 MHz): δ 2.98-3.04 (m, 2H, CH₂), 3.08-3.15 (m, 2H, CH₂), 3.80 (s, 2H, NH₂), 3.81 (s, 6H, 2 X OCH₃), 3.82 (s, 3H, OCH₃), 3.86 (s, 3H, OCH₃), 4.13 (s, 2H, CH₂), 6.13 (s, 2H, Ar-H), 6.76 (s, 3H, Ar-H). HRMS: m/z calcd [M + H] 396.14; found 396.12.

### Example 4

### 2-((2-Methoxy-5-(((2,4,6-trimethoxyphenethyl)sulfonyl)methyl)phenyl)amino)acetic acid

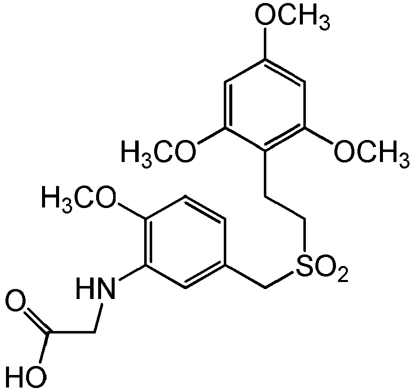

The title compound was prepared according to Scheme 5. A solution of (E)-2-((2-methoxy-5-(((2,4,6-trimethoxystyryl)sulfonyl) methyl)phenyl)amino)acetic acid (1.5 mmol) in a mixture of MeOH/EtOAc (40 mL) was treated with wet 10% palladium on carbon (50 mg) then exposed to hydrogen atmosphere by using hydrogenation apparatus. After completion of the reaction (monitored by TLC), the reaction mixture was filtered through Celite, washed the Celite pad thoroughly with a mixture of methanol and chloroform (50%) and the filtrate evaporated under reduced pressure. Filtered the solid, washed with ether and dried under vacuum to obtain the title compound according to General Procedure XIX with a yield of (96-98%).

### Example 5

### 2-Methoxy-5-(((2,4,6-trimethoxyphenethyl)sulfonyl)methyl)phenol

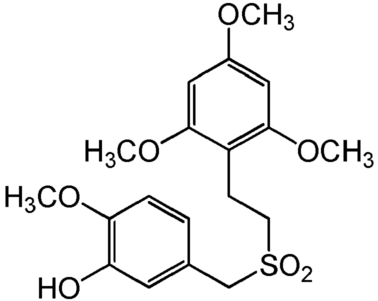

The title compound was prepared according to Scheme 5. A solution of (E)-2-methoxy-5-((2,4,6-trimethoxystyrylsulfonyl)methyl)phenol (1.5 mmol) in a mixture of MeOH (60 mL) was treated with wet 5% palladium on carbon (50 mg) then exposed to hydrogen atmosphere by using hydrogenation apparatus. After completion of the reaction (monitored by TLC), the reaction mixture was filtered through Celite, the Celite pad was washed thoroughly with methanol, and the filtrate evaporated under reduced pressure and dried under vacuum to obtain the title compound according to General Procedure XIX with a 95% yield and a melting point of 136-138 °C.

¹H NMR (CDC13, 300 MHz): δ 2.97-3.04 (m, 2H, CH₂), 3.09-3.15 (m, 2H, CH₂), 3.82 (s, 3H, OCH₃), 3.84 (s, 6H, 2 X OCH₃), 3.91 (s, 3H, OCH₃), 4.17 (s, 2H, CH₂), 5.63 (s, 1H, OH), 6.13 (s, 2H, Ar-H), 6.86 (d, J = 8.1 Hz, 1H, Ar-H), 6.93-7.00 (m, 2H, Ar-H). HRMS: m/z calcd [M + H] 397.47; found 397.20.

### Example 6

### 4-(2-((4-Chlorobenzyl)sulfonyl)ethyl)benzoic acid

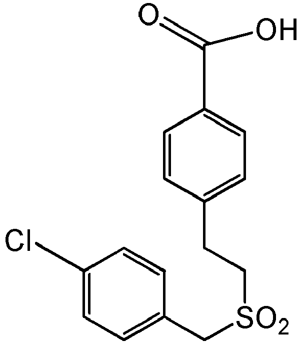

The title compound was prepared according to Scheme 5. A solution of (E)-4-(2-((4-chlorobenzyl)sulfonyl)vinyl)benzoic acid (500 mg, 1.48 mmol) in MeOH/EtOAc (1:1, 40 mL) was hydrogenated at 1 atm over 10% Pd-C for 48 h and then filtered through Celite. The Celite pad thoroughly washed with a mixture of methanol/chloroform (1:5). The combined solvent was removed under reduced pressure. The crude product was treated with methanol, filtered, washed with methanol, and dried to obtain the analytically pure title compound according to General Procedure XIX in an amount of yield of 480 mg and a melting point of 265-267 °C.

¹H NMR: 3.12-3.10 (m, 2H, Ar-CH₂-CH₂-SO₂), 3.43-3.41 (m, 2H, Ar-CH₂-CH₂-SO₂), 4.58 (s, 2H, Ar-CH₂), 7.43 (d, 2H, Ar-H, J = 6.0 Hz), 7.47 (d, 2H, Ar-H, J = 6.0 Hz), 7.52 (d, 2H, Ar-H, J = 6.0 Hz), 7.91 (d, 2H, Ar-H, J = 6.0 Hz). HRMS: (M+Na): 360.99.

### Example 7

### N-(3-Amino-4-methoxyphenyl)-3-(2,4,6-trimethoxyphenyl)propanamide

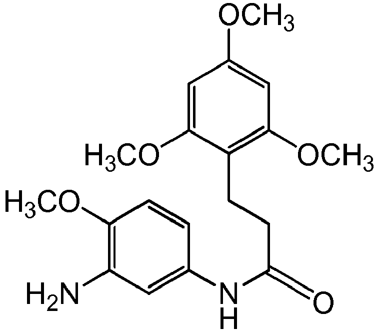

The title compound was prepared according to Scheme 5. A solution of (E)-N-(3-amino-4-methoxyphenyl)-3-(2,4,6-trimethoxyphenyl)acrylamide (1.5 mmol) in a mixture of MeOH (60 mL) was treated with wet 5% palladium on carbon (50 mg), then exposed to a hydrogen atmosphere by using a hydrogenation apparatus. After completion of the reaction (monitored by TLC), the reaction mixture was filtered through Celite, the Celite pad was washed thoroughly with a methanol, and the filtrate was evaporated under reduced pressure and dried under vacuum to obtain the pure title compound according to General Procedure XIX with a yield of 96-98% and a melting point: 154-156 °C.

¹H NMR (CDCl₃, 300 MHz): δ 2.55-2.60 (m, 2H, CH₂), 2.95-3.00 (m, 2H, CH₂), 3.77 (s, 3H, OCH₃), 3.81 (s, 6H, 2 X OCH₃), 3.83 (s, 3H, OCH₃), 6.15 (s, 2H, Ar-H), 6.60-6.74 (m, 2H, Ar-H), 7.09 (d, J = 8.1 Hz, 1H, Ar-H).

### Scheme 6

General Procedures XX-XXIII are used to prepare Compounds 1-4 in Scheme 6.

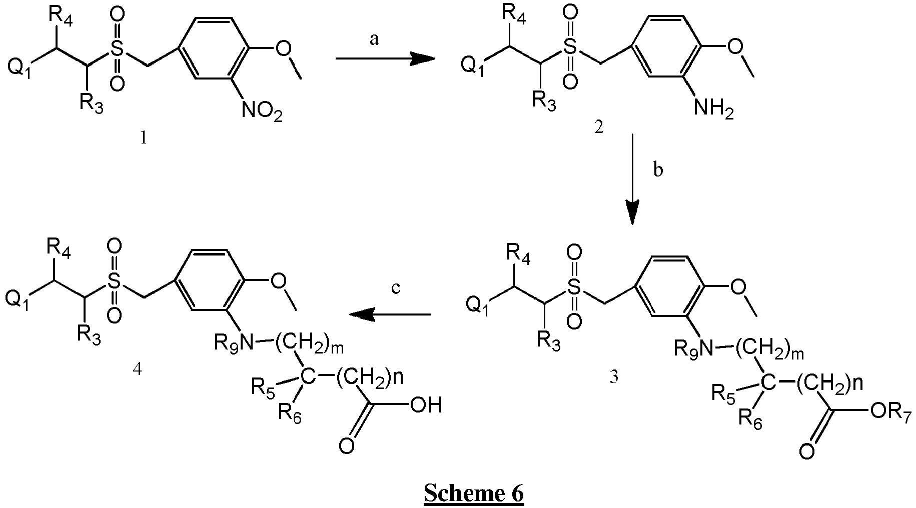

Non-limiting embodiments of reaction conditions for each step may include: a) Fe, MeOH,:AcOH (2:1), reflux, 3 hours; b) i) (R = H) BrCR₄R₅CO₂CH₃, NaOAc, EtOH, reflux, 48 hours; or ii) (Z= (CH₂CO₂CH₃) ICH₂CO₂CH₃, NaOAc, EtOH, reflux, 48 hours; and c) 20% aq. NaOH, EtOH, 2.5 hours.

### General Procedure XX

### Preparation of Compound 1 of Scheme 6: substituted 1-methoxy-2-nitro-4-((phenethylsulfonyl)methyl)benzenes.

Compound 1 of Scheme 6 can be prepared by following the procedure described for the Compound 7 in the Scheme 1.

### General Procedure XXI

### Preparation of Compound 2 of Scheme 6: 2-methoxy-5-((phenethylsulfonyl) methyl)anilines.

To a solution of Compound **1** of scheme 6 (2.4 mmol) in methanol/glacial acetic acid mixture (2:1, 120 ml), iron powder (12.0 mmol) is added and refluxed for 3 h. After completion of the reaction, the reaction mixture is cooled to 25°C and 80 mL of cold water is added. The contents are neutralized with 4N ammonia solution and then 100 ml of dichloromethane ("DCM") is added and stirred for 30 min. The contents are separated in an organic layer, washed with water, sat. sodium bicarbonate solution and finally with brine. The dried organic layer is concentrated to two thirds and crystallized with either cyclohexane or petroleum ether. The solid is filtered and dried to obtain pure compounds **2** of scheme 6 (90%).

### General Procedure XXII

### Preparation of Compound 3 of Scheme 6: substituted methyl 2-((2-methoxy-5-((phenethylsulfonyl)methyl)phenyl)amino)acetates.

Sodium acetate (40.0 mmol) is dissolved in ethanol (20 mL). Methyl bromoacetate (40.0 mmol) (or methyl idoacetate) is added to the above solution and refluxed for 10 min. Compound **2** of scheme 6 (10.0 mmol) is added and then refluxed for 48 h. After completion of the reaction monitored by TLC, the reaction mixture is concentrated under vacuum and poured into ice-water. The solid formed is filtered, washed with water, and dried under vacuum. The crude product on treatment with ethanol resulted in analytical pure products **3** of scheme 6 (70%).

### General Procedure XXIII

### Preparation of Compound 4 of Scheme 6: substituted 2-((2-methoxy-5-((phenethylsulfonyl)methyl)phenyl)amino)acetic acids.

To a solution of Compound **3** of scheme 6 (10.0 mmol) in ethanol (20 mL), 20% aqueous sodium hydroxide solution (20 mL) is added. The reaction mixture is refluxed for 2.5 h. After completion of the reaction (monitored by TLC), the solvent is removed under vacuum and the remaining water layer acidified by acetic acid to pH 4. The solid that formed is filtered and dried to get the crude amino acids **4** of scheme 6 which on crystallization from acetone (2x5 mL) resulted in analytically pure Compound **4** of scheme 6 as white crystals.

### Example 8

### Methyl 2-((2-methoxy-5-(((2,4,6-trimethoxyphenethyl)sulfonyl)methyl) phenyl)amino)acetate

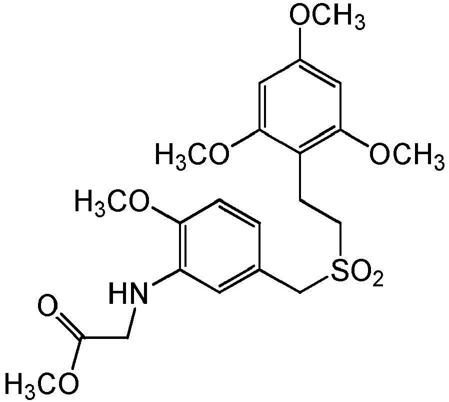

The title compound was prepared according to Scheme 6. Sodium acetate (3.28 g, 39 mmol) was dissolved in methanol (20 mL). Methyl 2-bromoacetate (6.11 g, 40 mmol) was added to the above solution and refluxed for 10 min. To the cooled reaction mixture, 2-methoxy-5-(((2,4,6-trimethoxyphenethyl)sulfonyl)methyl)aniline (3.95 g, 10 mmol) was added and then the reaction was reflux for 4-6 h. The reaction mixture was concentrated under vacuum and poured into ice water. The formed precipitate was filtered, washed with water, and dried under vacuum. The crude product was recrystallized from ethanol to produce the pure product of the title compound according to General Procedure XXII with a yield of 70% as a white solid with a mp of 156-158 °C.

¹H NMR (CDCl₃, 300 MHz): δ 2.98-3.04 (m, 2H, CH₂), 3.08-3.15 (m, 2H, CH₂), 3.79 (s, 3H, OCH₃), 3.81 (s, 6H, 2 X OCH₃), 3.82 (s, 3H, OCH₃), 3.88 (s, 3H, OCH₃), 3.95 (d, J = 4.2 Hz, 2H, CH₂), 4.17 (s, 2H, CH₂), 4.88 (t, J = 5.4 Hz, 1H, NH), 6.13(s, 2H, Ar-H), 6.53 (d, J = 1.2 Hz, 1H, Ar-H), 6.74-6.78 (m, 2H, Ar-H). HRMS: m/z calcd [M + H] 468.14; found 468.1446.

### Example 9

### Dimethyl 2,2'-((2-methoxy-5-(((2,4,6-trimethoxyphenethyl)sulfonyl) methyl)phenyl)azanediyl)diacetate

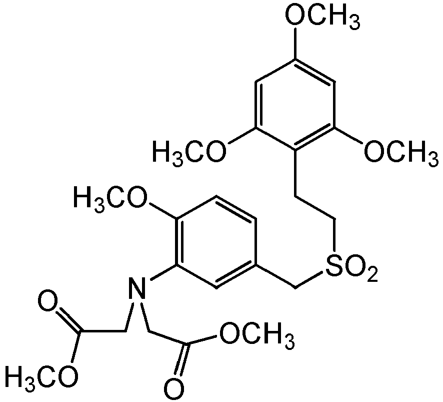

The title compound was prepared according to Scheme 6. Potassium carbonate (80 mmol) was dissolved in acetone (40 mL). Methyl-2-iodoacetate (80 mmol) was added to the above solution and refluxed for 10 min. To the cooled reaction mixture, 2-methoxy-5-((2,4,6-trimethoxyphenethylsulfonyl)methyl)aniline (10 mmol) was added and then refluxed for 60 h. The reaction mixture was concentrated under vacuum and poured into ice water. The formed precipitate was filtered, washed with water and dried under vacuum. The crude product according to General Procedure XXII was purified by column chromatography to produce pure product: yield, 70%; white solid, mp 86-88 °C.

¹H NMR (CDCl₃, 300 MHz): δ 2.95-3.01 (m, 2H, CH₂), 3.06-3.13 (m, 2H, CH₂), 3.72 (s, 6H, 2 X OCH₃), 3.81 (s, 6H, 2 X OCH₃), 3.82 (s, 3H, OCH₃), 3.83 (s, 3H, OCH₃), 4.15 (s, 6H, CH₂), 6.12 (s, 2H, Ar-H), 6.82 (d, J = 8.1 Hz, 1H, Ar-H), 6.91-6.97 (m, 2H, Ar-H).). HRMS: m/z calcd [M + H] 539.68; found 539.58.

### Example 10

### Alternative Synthesis of 2-((2-Methoxy-5-(((2,4,6-trimethoxyphenethyl)sulfonyl)methyl)phenyl)amino)acetic acid

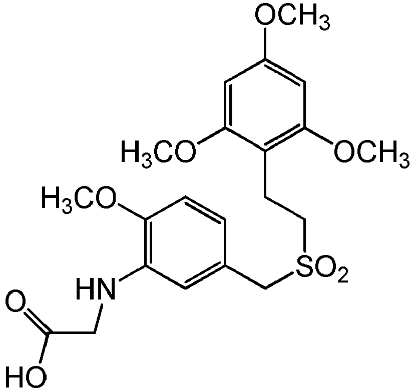

The title compound was prepared according to Scheme 6. To a solution of amine ester (46.5g, 100 mmol) in ethanol (200 mL), 20% aqueous sodium hydroxide solution (200 mL) was added. The reaction mixture was refluxed for 2.5 h. After completion of the reaction (TLC, monitoring, chloroform/methanol, 9:1 on silica gel plate), the solvent was removed under vacuum and the remainder was acidified by acetic acid to pH 4. The solid that formed was filtered and dried to get the crude amino acid, which on crystallization from acetone (2 X 25 mL) resulted in analytically pure crystals of the title compound, according to General Procedure XXIII. The yield of this reaction was 55%. Melting point: 146-150 °C.

¹H NMR (CDCl₃, 300 MHz): δ 2.96-3.01 (m, 2H, CH₂), 3.09-3.15 (m, 2H, CH₂), 3.82 (s, 9H, 3 X OCH₃), 3.93 (s, 3H, OCH₃), 4.00 (s, 2H, CH₂), 4.17 (s, 2H, CH₂), 6.14 (s, 2H, Ar-H), 6.55 (s, 1H, Ar-H), 6.78 (s, 2H, Ar-H).). HRMS: m/z calcd [M + H] 454.52; found 454.20.

### Example 11

### 2-((2-Methoxy-5-(((2,4,6-trimethoxyphenethyl)sulfonyl)methyl)phenyl) amino)propanoic acid

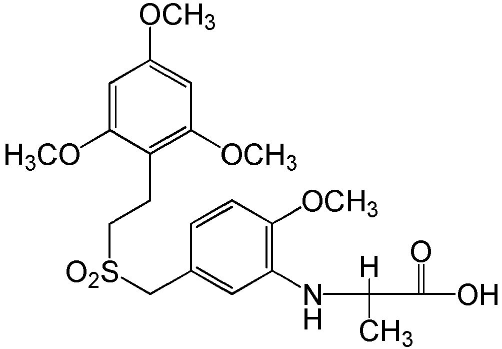

The title compound is prepared according to Scheme 6. Sodium acetate (32.8 g, 400 mmol) is dissolved in ethanol (200 mL). 2-bromopropionate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid forms is filtered, washed with water, and dried under vacuum. The crude product on purification from ethanol results in analytical pure product, according to General Procedure XXII.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure white crystals of the title compound, according to General Procedure XXIII.

### Example 12

### 2-((2-Methoxy-5-(((2,4,6-trimethoxyphenethyl)sulfonyl)methyl)phenyl)amino)-2-methylpropanoic acid

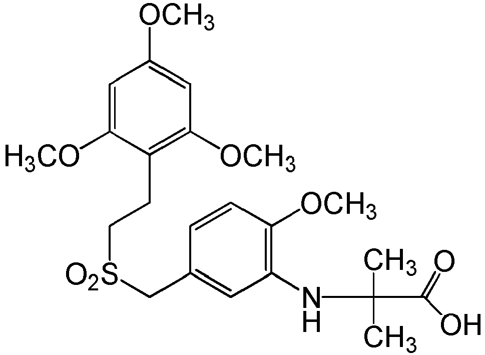

The title compound is prepared according to Scheme 6. Sodium acetate (32.8 g, 400 mmol) is dissolved in ethanol (200 mL). Methyl 2-bromo-2-methylpropionate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid formed is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII.

### Example 13

### 2-((2-Methoxy-5-(((2,4,6-trimethoxyphenethyl)sulfonyl)methyl)phenyl)amino)-2-phenylacetic acid

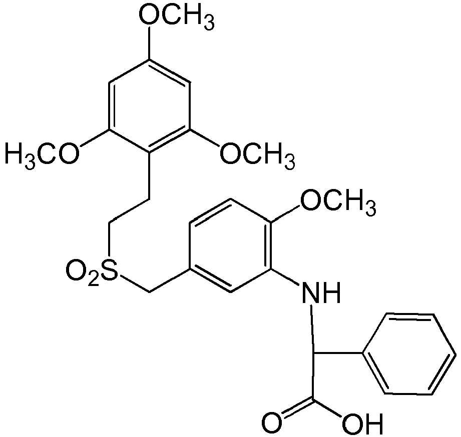

The title compound is prepared according to Scheme 6. Sodium acetate (32.8 g, 400 mmol) is dissolved in ethanol (200 mL). Methyl α-bromophenylacetate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid formed is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII.

### Example 14

### 2-(4-Fluorophenyl)-2-((2-methoxy-5-(((2,4,6-trimethoxylphenethyl)sulfonyl)methyl) phenyl)amino)acetic acid

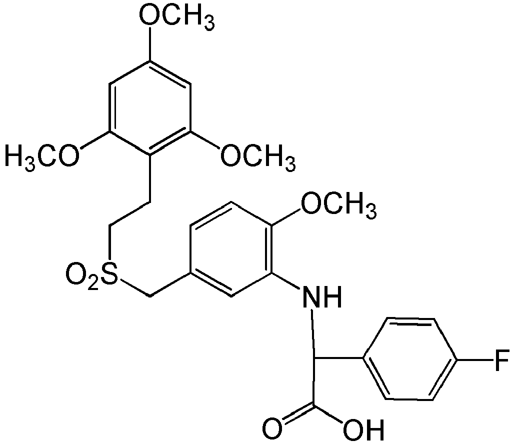

The title compound is prepared according to Scheme 6. Sodium acetate (32.8 g, 400 mmol) is dissolved in ethanol (200 mL). 2-bromo-2-(4-fluorophenyl)acetate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid formed is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII

### Example 15

### 2-(4-Chlorophenyl)-2-((2-methoxy-5-(((2,4,6-trimethoxyphenethyl)sulfonyl) methyl)phenyl)amino)acetic acid

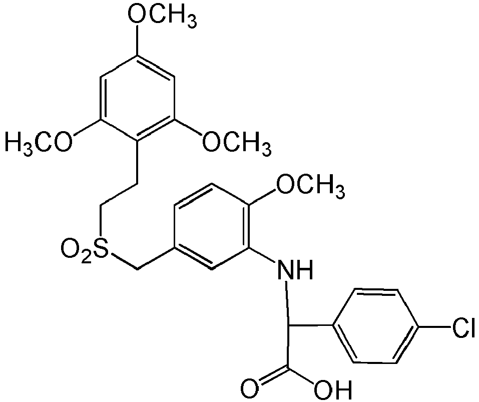

The title compound is prepared according to Scheme 6. Sodium acetate (32.8 g, 400 mmol) is dissolved in ethanol (200 mL). 2-bromo-2-(4-chlorophenyl)acetate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid formed is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII.

### Example 16

### 2-((2-Methoxy-5-(((2,4,6-trimethoxyphenethyl)sulfonyl)methyl)phenyl)amino)-3-phenylpropanoic acid

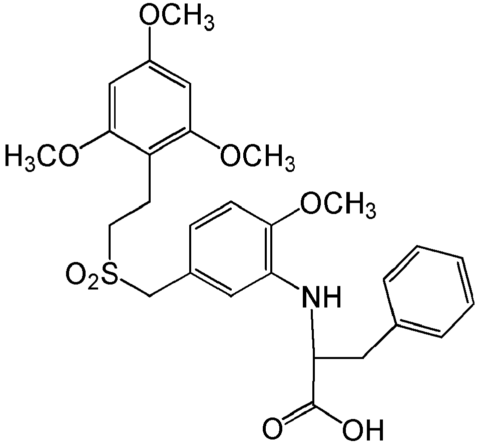

The title compound is prepared according to Scheme 6. Sodium acetate (32.8 g, 400 mmol) is dissolved in ethanol (200 mL). Methyl 2-bromo-3-phenylpropanoate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid form is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII.

### Example 17

### 2-Cyclopropyl-2-((2-methoxy-5-(((2,4,6-trimethoxyphenethyl)sulfonyl)methyl) phenyl)amino)acetic acid

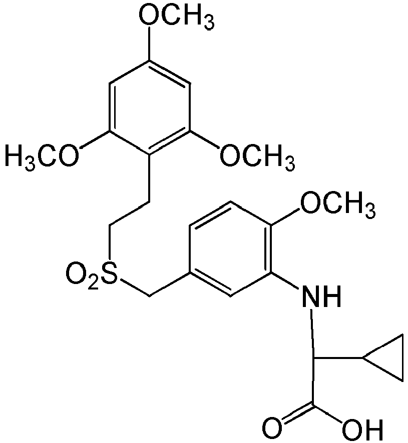

The title compound is prepared according to Scheme 6. Sodium acetate (32.8 g, 400 mmol) is dissolved in ethanol (200 mL). Methyl 2-bromo-2-cyclopropylacetate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid form is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) is added. The reaction mixture is refluxed for 2.5 h. After completion of the reaction (TLC, monitoring, chloroform/methanol, 9:1 on silica gel plate), the solvent is removed under vacuum and the remainder is acidified by acetic acid to pH 4. The solid that forms is filtered and dried to get the crude amino acid which on crystallization from acetone (2-25 mL) results in analytically pure amino acid as white crystals of the title compound, according to General Procedures XXII and XXIII.

### Example 18

### 2-((2-Methoxy-5-(((2,4,6-trimethoxyphenethyl)sulfonyl)methyl)phenyl)amino)-2-(1H-pyrrol-3-yl)acetic acid

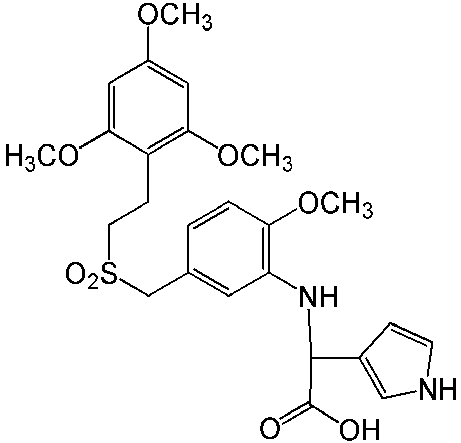

The title compound is prepared according to Scheme 6. Sodium acetate (32.8 g, 400 mmol) is dissolved in ethanol (200 mL). Methyl 2-bromo-2-(1H-pyrrol-3-yl)acetate (400 mmol) is added to the above solution and refluxed for 10 min. To the cooled reaction mixture compound 2-methoxy-5-(((2,4,6-trimethoxyphenethyl) sulfonyl)methyl)aniline (39.35 g, 100 mmol) is added and then refluxed for 48 h. After completion of the reaction monitored by TLC (chloroform/ methanol, 9:1 on silica gel plate), the reaction mixture is concentrated under vacuum and poured into ice-water. The solid forms is filtered, washed with water, and dried under vacuum. The crude product on purification from ethanol results in analytical pure product.

To a solution of above amine ester (46.5 g, 100 mmol) in ethanol (200 mL), 20% aqueous sodium hydroxide solution (200 mL) was added. The reaction mixture is refluxed for 2.5 h. After completion of the reaction (TLC, monitoring, chloroform/methanol, 9:1 on silica gel plate), the solvent is removed under vacuum and the remainder was acidified by acetic acid to pH 4. The solid that forms was filtered and dried to get the crude amino acid which on crystallization from acetone (2-25 mL) results in analytically pure amino acid as white crystals of the Title compound, according to General Procedures XXII and XXIII.

### Example 19

### N-(3-Amino-4-methoxyphenyl)-2-(2,4,6-trimethoxyphenyl)ethanesulfonamide

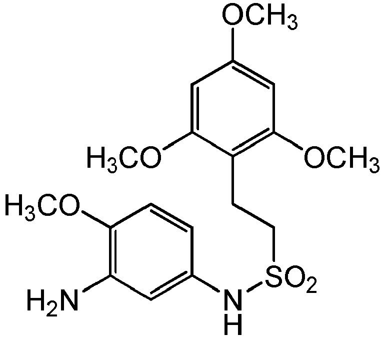

The title compound was prepared according to Scheme 5. A solution of 2-(2,4,6-Trimethoxyphenyl)ethenesulfonic acid (3-amino-4-methoxyphenyl)amide (1.5 mmol) in a mixture of MeOH (60 mL) was treated with wet 5% palladium on carbon (50 mg) then exposed to hydrogen atmosphere by using hydrogenation apparatus. After completion of the reaction (monitored by TLC), the reaction mixture was filtered through Celite, washed the Celite pad thoroughly with methanol and the filtrate evaporated under reduced pressure and dried under vacuum to get pure N-(3-amino-4-methoxyphenyl)-2-(2,4,6-trimethoxyphenyl)-ethanesulfonamide (95%), according to General Procedure XIX.

### Example 20

### 1,3,5-Trimethoxy-2-(2-((4-methoxybenzyl)sulfonyl)ethyl)benzene

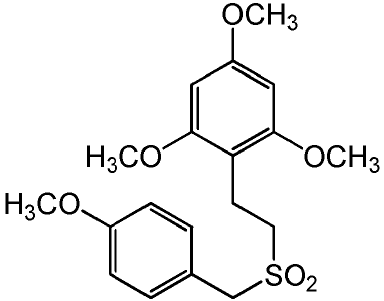

The title compound was prepared according to Scheme 5. A solution of (E)-2,4,6-trimethoxystyryl-4-methoxybenzylsulfone (1.5 mmol) in a mixture of MeOH (60 mL) was treated with wet 5% palladium on carbon (50 mg) then exposed to hydrogen atmosphere by using hydrogenation apparatus. After completion of the reaction (monitored by TLC), the reaction mixture was filtered through Celite, washed the Celite pad thoroughly with methanol and the filtrate evaporated under reduced pressure and dried under vacuum to get pure 1,3,5-trimethoxy-2-(2-((4-methoxybenzyl)sulfonyl)ethyl)benzene (95%), according to General Procedure XIX.

### Example 21

### Sodium 2-((2-methoxy-5-(((2,4,6-trimethoxyphenethyl)sulfonyl)methyl)phenyl)amino) acetate

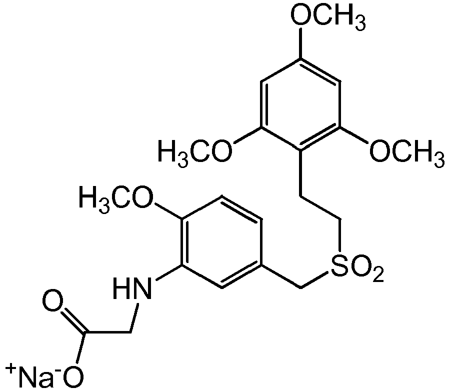

The title compound was prepared according to Scheme 6. Sodium acetate (3.28 g, 39 mmol) was dissolved in methanol (20 mL). Methyl 2-bromoacetate (6.11g, 40 mmol) was added to the above solution and refluxed for 10 min. To the cooled reaction mixture compound 2-methoxy-5-(((2,4,6-trimethoxyphenethyl) sulfonyl)methyl)aniline (3.99 g, 10 mmol) was added, and then the mixture was refluxed for 4-6 h. The reaction mixture was concentrated under vacuum and poured into ice-water. The formed precipitate was filtered, washed with water, and dried under vacuum. The crude product on recrystallization from ethanol resulted in pure product.

To a solution of sodium hydroxide (3.95 g, 99 mmol) in water (11.5 mL) at 20 °C was added ethanol (40 mL), the above purified product (99 mmol) and dichloromethane (200 mL). The resulting mixture was stirred at room temperature for 3-4 h. After completion of the reaction monitored by TLC (chloroform/methanol, 9:1 on silica gel plate), charcoal was added and the mixture was stirred for 30 min. The reaction mixture was filtered through Celite and washed with ethanol (2X20 mL). The combined filtrate was distilled at 50 °C until most of the solvent was removed. Methyl ethyl ketone (70 mL) was added to the residue, and distillation of the methyl ethyl ketone was at 50 °C. To the residue, water (10 mL) was added. The resulting mixture was heated to 70 °C and maintained for 30 min. The reaction mixture was cooled to room temperature and stirred for 2 h at room temperature. The solid formed was filtered, washed with methyl ethyl ketone (2 X 20 mL), and dried to get the purified title compound, according to General Procedures XXII and XXIII.

The compounds of Table 1 were prepared according to Examples 22-32 as follows:

**Table 1**

| | | | | | |
|---|---|---|---|---|---|
| 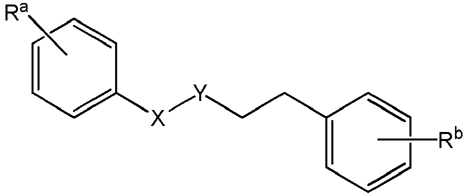 | | | | | |

| **Ex.** | **Compound** | **R^{a}** | **R^{b}** | **X** | **Y** |
|---|---|---|---|---|---|
| 22 | *N*-(4-methoxyphenethyl)-4-methylbenzenesulfonamide | 4-CH₃ | 4-OCH₃ | SO₂ | NH |
| 23 | 4-methoxy-*N*-(4-methoxyphenethyl)-3-nitrobenzenesulfonamide | 3-NO₂, 4-OCH₃ | 4-OCH₃ | SO₂ | NH |
| 24 | 3-amino-4-methoxy-*N*-(4-methoxyphenethyl)benzenesulfonamide | 3-NH₂, 4-OCH₃ | 4-OCH₃ | SO₂ | NH |
| 25 | 4-methoxy-3-nitro-*N*-(2,4,6-trimethoxyphenethyl)benzenesulfonamide | 3-NO₂, 4-OCH₃ | 2,4,6-(CH₃O)₃ | SO₂ | NH |
| 26 | 3-amino-4-methoxy-*N*-(2,4,6-trimethoxyphenethyl)benzenesulfonamide | 3-NH₂, 4-OCH₃ | 2,4,6-(CH₃O)₃ | SO₂ | NH |
| 27 | 4-methoxy-*N*-(4-methoxyphenethyl)benzenesulfonamide | 4-OCH₃ | 4-OCH₃ | SO₂ | NH |
| 28 | 4-methoxy-*N*-(2,4,6-trimethoxyphenethyl)benzenesulfonamide | 4-OCH₃ | 2,4,6-(CH₃O)₃ | SO₂ | NH |
| 29 | 4-methyl-*N*-(2,4,6-trimethoxyphenethyl)benzenesulfonamide | 4-CH₃ | 2,4,6-(CH₃O)₃ | SO₂ | NH |
| 30 | 2-(4-methoxyphenyl)-*N*-(2,4,6-trimethoxyphenyl)ethanesulfonamide | 2,4,6-(CH₃O)₃ | 4-OCH₃ | NH | SO₂ |
| 31 | 4-(((3,4-dimethoxyphenethyl)thio)methyl)-2-methoxyphenol | 3-OCH₃, 4-OH | 3,4-(CH₃O)₂ | CH₂ | S |
| 32 | 4-(((3,4-dimethoxyphenethyl)sulfonyl)methyl)-2-methoxyphenol | 3-OCH₃, 4-OH | 3,4-(CH₃O)₂ | CH₂ | SO₂ |

### Example 22: N-(4-Methoxyphenethyl)-4-methylbenzenesulfonamide

The title compound was prepared from 2-(4-methoxyphenyl)ethanamine and p-toluenesulfonyl chloride according to Scheme 2 and General procedures VII-XI. ¹H NMR (CDCl₃, 300 MHz): δ 2.33 (s, 3H, CH₃), 2.60 (t, J =7.2 Hz, 2H, CH₂), 3.03-3.10 (m, 2H, CH₂), 3.68 (s, 3H, OCH₃), 4.71 (s, 1H, NH), 6.81 (d, J = 8.4 Hz, 2H, Ar-H), 6.90 (d, J = 8.4 Hz, 2H, Ar-H), 7.19 (d, J = 8.1 Hz, 2H, Ar-H), 7.61 (d, J = 8.4 Hz, 2H, Ar-H). HRMS: m/z calcd [M + H] 306.11; found 306.10.

### Example 23: 4-Methoxy-N-(4-methoxyphenethyl)-3-nitrobenzenesulfonamide

The title compound was prepared from 2-(4-methoxyphenyl)ethanamine and 4-methoxy-3-nitrophenylsulfonyl chloride according to Scheme 2 and General procedures VII-XI. ¹H NMR (CDCl₃, 300 MHz): δ 2.75 (t, J =6.6 Hz, 2H, CH₂), 3.24-3.27 (m, 2H, CH₂), 3.80 (s, 3H, OCH₃), 4.06 (s, 3H, OCH₃), 4.45 (t, J = 6.3 Hz, 1H, NH), 6.70 (d, J = 8.4 Hz, 2H, Ar-H), 7.02 (d, J = 8.4 Hz, 2H, Ar-H), 7.15 (d, J = 8.7 Hz, 1H, Ar-H), 7.94 (dd, J = 2.4, 9.0 Hz, 1H, Ar-H), 8.25 (d, J = 2.4 Hz, 1H, Ar-H). HRMS: m/z calcd [M + H] 367.09; found 367.10.

### Example 24: 3-Amino-4-methoxy-N-(4-methoxyphenethyl)benzenesulfonamide

The title compound was prepared from the Example 23 compound according to Scheme 5 and General Procedure XIX. ¹H NMR (CDCl₃, 300 MHz): δ 2.63 (t, J =6.9 Hz, 2H, CH₂), 3.07-3.10 (m, 2H, CH₂), 3.72 (s, 3H, OCH₃), 3.84 (s, 3H, OCH₃), 4.13 (t, J = 6.3 Hz, 1H, NH), 6.74 (dd, J = 2.1, 8.4 Hz, 3H, Ar-H), 6.93 (d, J = 8.7 Hz, 2H, Ar-H), 6.99 (d, J = 2.4 Hz, 1H, Ar-H), 7.13 (dd, J = 2.1, 8.4 Hz, 1H, Ar-H). HRMS: m/z calcd [M + H] 337.11; found 337.20.

### Example 25: 4-Methoxy-3-nitro-N-(2,4,6-trimethoxyphenethyl)benzenesulfonamide

The title compound was prepared from 2-(2,4,6-trimethoxyphenyl)ethanamine and 4-methoxy-3-nitrophenylsulfonyl chloride according to Scheme 2 and General Procedures VII-XI. ¹H NMR (CDCl₃, 300 MHz): δ 2.71 (t, J =6.3 Hz, 2H, CH₂), 3.19-3.25 (m, 2H, CH₂), 3.75 (s, 6H, 2 X OCH₃), 3.80 (s, 3H, OCH₃), 4.03 (s, 3H, OCH₃), 4.90 (t, J = 4.5 Hz, 1H, NH), 5.98 (s, 2H, Ar-H), 6.97 (d, J = 8.7 Hz, 1H, Ar-H), 6.75 (dd, J = 2.4, 9.0 Hz, 1H, Ar-H), 8.09 (d, J = 2.4 Hz, 1H, Ar-H). HRMS: m/z calcd [M + H] 427.11; found 427.20.

### Example 26: 3-Amino-4-methoxy-N-(2,4,6-trimethoxyphenethyl)benzenesulfonamide

The title compound was prepared from the Example 25 compound according to Scheme 5 and General Procedure XIX. ¹H NMR (CDCl₃, 300 MHz): δ 2.73 (t, J =6.3 Hz, 2H, CH₂), 3.10-3.14 (m, 2H, CH₂), 3.76 (s, 6H, 2 X OCH₃), 3.82 (s, 3H, OCH₃), 3.93 (s, 3H, OCH₃), 4.63 (t, J = 4.8 Hz, 1H, NH), 6.07 (s, 2H, Ar-H), 6.72 (d, J = 8.4 Hz, 1H, Ar-H), 6.98 (s, 1H, Ar-H), 7.13 (dd, J = 2.1, 8.4 Hz, 1H, Ar-H). HRMS: m/z calcd [M + H] 397.14; found 397.10.

### Example 27: 4-Methoxy-N-(4-methoxyphenethyl)benzenesulfonamide

The title compound was prepared from 2-(4-methoxyphenyl)ethanamine and 4-methoxyphenylsulfonyl chloride according to Scheme 2 and General Procedures VII-XI. ¹H NMR (CDCl₃, 300 MHz): δ 2.72 (t, J =6.3 Hz, 2H, CH₂), 3.18 (dd, J = 6.9, 13.2 Hz, 2H, CH₂), 3.80 (s, 3H, OCH₃), 3.89 (s, 3H, OCH₃), 4.38 (t, J = 6.3 Hz, 1H, NH), 6.82 (d, J = 8.4 Hz, 2H, Ar-H), 6.97 (d, J = 9.0 Hz, 2H, Ar-H), 7.01 (d, J = 8.7 Hz, 2H, Ar-H), 7.75 (d, J = 8.7 Hz, 2H, Ar-H). HRMS: m/z calcd [M + H] 332.10; found 332.10.

### Example 28: 4-Methoxy-N-(2,4,6-trimethoxyphenethyl)benzenesulfonamide

The title compound was prepared from 2-(2,4,6-trimethoxyphenyl)ethanamine and 4-methoxyphenylsulfonyl chloride according to Scheme 2 and General Procedures VII-XI. ¹H NMR (CDCl₃, 300 MHz): δ 2.63 (t, J =6.3 Hz, 2H, CH₂), 3.02-3.04 (m, 2H, CH₂), 3.65 (s, 6H, 2 X OCH₃), 3.73 (s, 3H, OCH₃), 3.79 (s, 3H, OCH₃), 4.58 (t, J = 4.8 Hz, 1H, NH), 5.95 (s, 2H, Ar-H), 6.76 (d, J = 9.0 Hz, 2H, Ar-H), 7.52 (d, J = 9.0 Hz, 2H, Ar-H). HRMS: m/z calcd [M + H] 382.12; found 382.10.

### Example 29: 4-Methyl-N-(2,4,6-trimethoxyphenethyl)benzenesulfonamide

The title compound was prepared from 2-(2,4,6-trimethoxyphenyl)ethanamine and p-toluenesulfonyl chloride according to Scheme 2 and General Procedures VII-XI. ¹H NMR (CDCl₃, 300 MHz): δ 2.32 (s, 3H, CH₃), 2.62 (t, J =6.3 Hz, 2H, CH₂), 3.00-3.04 (m, 2H, CH₂), 3.65 (s, 6H, 2 X OCH₃), 3.73 (s, 3H, OCH₃), 4.59 (t, J = 4.5 Hz, 1H, NH), 5.96 (s, 2H, Ar-H), 7.10 (d, J = 8.1 Hz, 2H, Ar-H), 7.49 (d, J = 8.1 Hz, 2H, Ar-H). HRMS: m/z calcd [M + H] 366.13; found 366.10.

### Example 30: 2-(4-Methoxyphenyl)-N-(2,4,6-trimethoxyphenyl)ethanesulfonamide

The title compound was prepared from 2,4,6-trimethoxyaniline and 2-(4-methoxyphenyl)ethanesulfonyl chloride according to Scheme 2 and General Procedures VII-XI. ¹H NMR (CDCl₃, 300 MHz): δ 3.09-3.15 (m, 2H, CH₂), 3.38-3.44 (m, 2H, CH₂), 3.72 (s, 3H, OCH₃), 3.73 (s, 3H, OCH₃), 3.76 (s, 6H, 2 X OCH₃), 5.66(s, 1H, NH), 6.08 (s, 2H, Ar-H), 6.79 (d, J = 8.7 Hz, 2H, Ar-H), 7.08 (d, J = 8.7 Hz, 2H, Ar-H). HRMS: m/z calcd [M + H] 382.12; found 382.10.

### Example 31: 4-(((3,4-dimethoxyphenethyl)thio)methyl)-2-methoxyphenol

The title compound was prepared from 4-(2-bromoethyl)-1,2-dimethoxybenzene and 4-(mercaptomethyl)-2-methoxyphenol according to Scheme 1 and General Procedure V. ¹H NMR (CDCl₃, 300 MHz): δ 2.62-2.68 (m, 2H, -CH₂), 2.77-2.95 (m, 2H, -CH₂), 3.68 (s, 2H, Ar-CH₂), 3.87 (s, 6H, 2 x OCH₃), 3.90 (s, 3H, OCH₃), 5.57 (br s, 1H, OH), 6.66-6.88 (m, 6H, Ar-H). HRMS: m/z calcd [M + H] 335.13; found 335.10.

### Example 32: 4-(((3,4-dimethoxyphenethyl)sulfonyl)methyl)-2-methoxyphenol

The title compound was prepared from the Example 31 compound according to Scheme 1 and General Procedure VI. ¹H NMR (CDCl₃, 300 MHz): δ 2.95-3.01 (m, 2H, -CH₂), 3.04-3.10 (m, 2H, -CH₂), 3.79 (s, 6H, 2 x OCH₃), 3.80 (s, 3H, OCH₃), 4.09 (s, 2H, Ar-CH₂), 6.17 (br s, 1H, OH), 6.64-6.69 (m, 2H, Ar-H), 6.71-6.77 (m, 2H, Ar-H), 6.83 (d, J = 8.1 Hz, 1H, Ar-H), 6.88 (d, J = 1.8 Hz, 1H, Ar-H). HRMS: m/z calcd [M + H] 367.12; found 367.20.

### Example 33: Cancer Cell Assays

The effect of the compounds of the invention on tumor cells was determined by the assay described by Latham et al., Oncogene 12:827-837 (1996). Tumor cells K562 (chronic myelogenous leukemia; leukemia cell line +ve for Bcr-Abl) or DU145 (prostate cancer) were plated in 12-well dishes at a cell density of 2.5 x 10⁴ cells well. The plated cells were treated 24 hours later with a compound of the invention dissolved in DMSO at multiple concentrations ranging from 0.01 µM to 100 µM. The plates were examined 96 hours later under an inverted microscope, Olympus CK-2 using a 10x objective, and compound activity was noted by physical observation. When necessary, the total number of viable cells was determined by trypsinizing the wells and counting the number of viable cells, as determined by trypan blue exclusion, using a hemacytometer. The results of these assays are provided below in Table 2.

**Table 2**

| Example # | IC₅₀ (µM) K562 | IC₅₀ (µM) DU145 |
|---|---|---|
| 2 | 1.5 | 1.5 |
| 3 | 0.0025 | 0.003 |
| 4 | 0.5 | 0.25 |
| 5 | 0.003 | 0.0075 |
| 6 | 10 | 10 |
| 7 | 0.15 | 5 |
| 8 | 0.15 | 0.15 |
| 9 | 2 | 2 |
| 19 | 0.003 | 0.005 |
| 20 | 0.05 | 0.15 |
| 21 | 0.04 | 0.15 |
| 22 | 60 | 60 |
| 23 | 35 | 35 |
| 25 | 35 | 35 |
| 26 | 0.75 | 5 |
| 27 | 75 | 75 |
| 28 | >10 | >10 |
| 29 | >10 | >10 |
| 30 | 2.5 | 15 |
| 31 | 5 | 15 |
| 32 | >10 | >10 |

## Claims

1. A compound of Formula I or a salt thereof:
**Q₂-X-Y-CHR₃-CHR₄-Q₁** **(I)**
wherein:
Q₁ is substituted phenyl, with up to 5 substituents selected from the group consisting of:
fluoro; bromo; nitro; -NR₁₀R₁₁; aroylamino; cyano; carboxamido; trifluoromethyl; -O-R₁₀; [-N(-R₁)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-; wherein Q₁ has at least one substituent selected from the group consisting of -O-R₁₀ and fluoro;
Q₂ is substituted phenyl, with up to 5 substituents selected from the group consisting of:
fluoro; chloro; nitro; -NR₁₀R₁₁; aroylamino; cyano; carboxy; carboxamido; trifluoromethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
wherein at least the 4-position of both Q₁ and Q₂ is substituted, and one of Q₁ and Q₂ is substituted with a substituent other than unsubstituted phenyl;
X is -CH₂-;
Y is -S(=O)₂-;
R₁ is selected from the group consisting of H and a C₁-C₁₀ saturated or unsaturated, straight or branched, cyclic or acyclic, chiral or achiral hydrocarbyl group, wherein optionally at least one carbon atom of the hydrocarbyl group is replaced by -N(-R₁)-, -O- or -S-;
R₃ and R₄ are each H;
R₅ and R₆ are each independently selected from the group consisting of H; halo; and C₁-C₁₀ saturated or unsaturated, straight or branched, cyclic or acyclic, chiral or achiral hydrocarbyl group, optionally substituted with one or more of halogen, C₁-C₄ alkyl, C₁-C₄ alkoxy, hydroxy, carboxy, carboxamido, amino, (C₁-C₆) alkyl, (C₁-C₆) dialkyl amino, or acylamino;
or
R₅ and R₆ may combine to form a saturated or unsaturated carbocyclic ring with from 3 to 6 carbon atoms, wherein one or more carbon atoms is optionally replaced with -N(-R₁)-, -O-, or -S-;
R₇ is selected from the group consisting of H; and a C₁-C₁₀ saturated or unsaturated, straight or branched, cyclic or acyclic, chiral or achiral hydrocarbyl group; and an inorganic cation or an organic cation to form a salt;
R₈ is selected from the group consisting of H and (C₁-C₇) hydrocarbyl;
R₉ is selected from the group consisting of H and -(CH₂)ₘ-C(-R₅)(-R₆)-(CH₂)ₙ-COOR_{7;}
R₁₀ is selected from the group consisting of H, branched or unbranched (C₁-C₆)alkyl, and (C₂-C₈)acyl;
R₁₁ is selected from the group consisting of H, branched or unbranched (C₁-C₆)alkyl, and (C₂-C₈)acyl;
m and n are each independently 0-2; and
z is 1-2.

2. A compound according to claim 1, or a salt thereof, wherein Q₂ has at least one substituent selected from the group consisting of chloro; -O-R₁₀; -NR₁₀R₁₁; and [-N(-R₉)-(CH₂)ₘ-C(-R₅)(-R₆)-(CH₂)ₙ-COOR₇]_{z}.

3. A compound according to claim 2, or a salt thereof, wherein Q₁ is substituted at the 2-, 4- and 6-positions with (C₁-C₃)alkoxy, and Q₂ is substituted in at least the 4-position with -O-R₁₀ or -NR₁₀R₁₁.

4. A compound according to claim 3, or a salt thereof, wherein Q₂ is substituted at the 4-position with -O-R₁₀ or -NR₁₀R₁₁ and substituted at the 3-position with -NR₁₀R₁₁, -O-R₁₀ or [-N(-R₉)-(CH₂)ₘ-C(-R₅)(-R₆)-(CH₂)ₙ-COOR₇]_{z}.

5. A compound according to claim 1 selected from the group consisting of:
2-((2-methoxy-5-(((2,4,6-trimethoxyphenethyl)sulfonyl)methyl)phenyl)amino)acetic acid;
1-chloro-4-(((4-fluorophenethyl)sulfonyl)methyl)benzene;
2-methoxy-5-(((2,4,6-trimethoxyphenethyl)sulfonyl)methyl)aniline;
2-methoxy-5-(((2,4,6-trimethoxyphenethyl)sulfonyl)methyl)phenol;
methyl 2-((2-methoxy-5-(((2,4,6-trimethoxyphenethyl)sulfonyl)methyl)phenyl) amino)acetate;
dimethyl 2,2'-((2-methoxy-5-(((2,4,6-trimethoxyphenethyl)sulfonyl) methyl)phenyl)azanediyl)diacetate;
2-((2-methoxy-5-(((2,4,6-trimethoxyphenethyl)sulfonyl)methyl)phenyl) amino)propanoic acid;
2-((2-methoxy-5-(((2,4,6-trimethoxyphenethyl)sulfonyl)methyl)phenyl)amino)-2-methylpropanoic acid;
2-((2-methoxy-5-(((2,4,6-trimethoxyphenethyl)sulfonyl)methyl)phenyl)amino)-2-phenylacetic acid;
2-(4-fluorophenyl)-2-((2-methoxy-5-(((2,4,6-trimethoxyphenethyl)sulfonyl) methyl)phenyl)amino)acetic acid;
2-(4-chlorophenyl)-2-((2-methoxy-5-(((2,4,6-trimethoxyphenethyl)sulfonyl) methyl)phenyl)amino)acetic acid;
2-((2-methoxy-5-(((2,4,6-trimethoxyphenethyl)sulfonyl)methyl)phenyl)amino)-3-phenylpropanoic acid;
2-cyclopropyl-2-((2-methoxy-5-(((2,4,6-trimethoxyphenethyl)sulfonyl)methyl)phenyl)amino)acetic acid;
2-((2-methoxy-5-(((2,4,6-trimethoxyphenethyl)sulfonyl)methyl)phenyl) amino)-2-(1H-pyrrol-3-yl)acetic acid;
1,3,5-trimethoxy-2-(2-((4-methoxybenzyl)sulfonyl)ethyl)benzene;
and salts thereof.

6. A compound according to claim 5, wherein the compound is 2-((2-methoxy-5-(((2,4,6-trimethoxyphenethyl)sulfonyl)methyl)phenyl)amino)acetate or pharmaceutically acceptable salt thereof.

7. The compound according to claim 6 which is sodium 2-((2-methoxy-5-(((2,4,6-trimethoxyphenethyl)sulfonyl)methyl)phenyl)amino)acetate.

8. A process of making a compound of Formula I or a salt thereof, according to claim 1, having a Formula la: 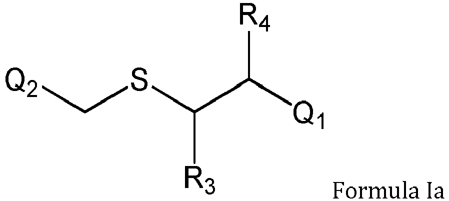 said process comprising:
reacting a compound of Formula AA: 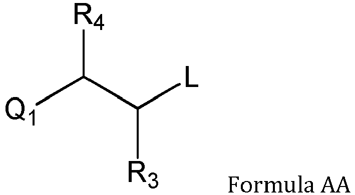 with a compound of Formula BB: 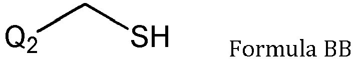 to produce a compound of Formula la or a salt thereof in a reaction mixture, and optionally isolating the compound of Formula la or a salt thereof from the reaction mixture, wherein Q₁, Q₂, R₃, and R₄ are as defined in claim 1, and L is a leaving group selected from the group consisting of Cl, Br, and I.

9. A process of making a compound of Formula I or a salt thereof according to claim 1, having a Formula Ib: 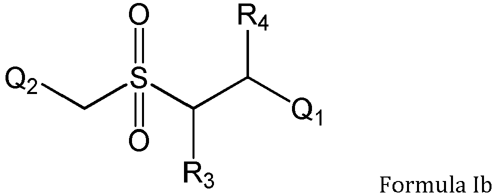 said process comprising:
oxidizing a compound of Formula la: 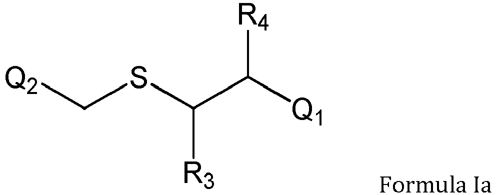 to produce a compound of Formula Ib or a salt thereof in a reaction mixture, and optionally isolating the compound of Formula Ib or a salt thereof from the reaction mixture, wherein Q₁, Q₂, R₃, and R₄ are as defined in claim 1.

10. A process of making a compound of Formula I or a salt therof according to claim 1, having a Formula If; 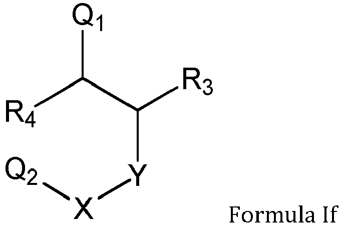 said process comprising:
reacting a compound of Formula Ie: 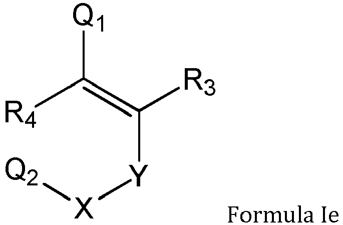 to produce a compound of Formula If or a salt thereof in a reaction mixture, and optionally isolating the compound of Formula If or a salt thereof from the reaction mixture, wherein X, Y, Q₁, Q₂, R₃, and R₄ are as defined in claim 1.

11. A process of making a compound of Formula I or a salt thereof according to claim 1, having the Formula Ii: 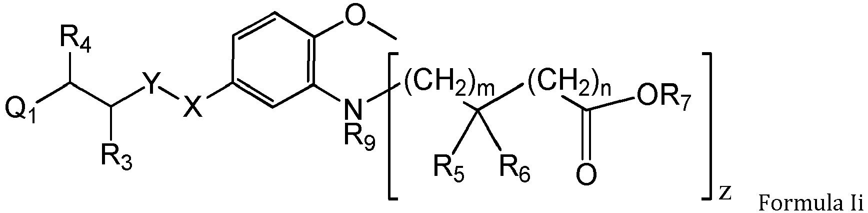 said process comprising:
reacting a compound of Formula Ih: 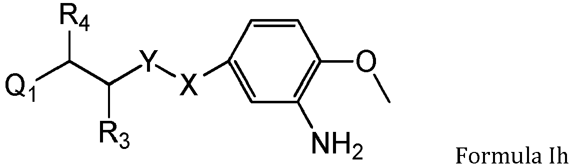 to produce a compound of Formula Ii or a salt thereof in a reaction mixture, and optionally isolating the compound of Formula Ii or a salt thereof from the reaction mixture, wherein X, Y, Q₁, R₃, R₄, R₅, R₆, R₇,R₉, m, n, and z are as defined in claim 1.

12. A pharmaceutical composition comprising a pharmaceutically acceptable carrier and a compound, or a pharmaceutically acceptable salt thereof, according to claim 1.

13. A pharmaceutical composition comprising a pharmaceutically acceptable carrier and a compound, or a pharmaceutically acceptable salt thereof, according to any one of claims 5 to 7.

14. A pharmaceutical composition comprising a pharmaceutically acceptable carrier and a compound, or a pharmaceutically acceptable salt thereof, according to claim 6.

15. A pharmaceutical composition comprising a pharmaceutically acceptable carrier and the compound according to claim 7.

16. A compound, or a pharmaceutically acceptable salt thereof, according to claim 1 for use in the treatment of a cellular proliferative disorder.

17. The compound or pharmaceutically acceptable salt thereof for use according to claim 16, wherein the compound is 2-((2-methoxy-5-(((2,4,6-trimethoxyphenethyl)sulfonyl)methyl)phenyl)amino)acetate or pharmaceutically acceptable salt thereof.

18. The compound or pharmaceutically acceptable salt thereof for use according to claim 16 or 17, wherein the cellular proliferative disorder is cancer.

19. The compound or pharmaceutically acceptable salt thereof for use according to claim 18, wherein the cellular proliferative disorder is a cancer selected from the group consisting of ovarian cancer; cervical cancer; breast cancer; prostate cancer; testicular cancer, lung cancer, renal cancer; colorectal cancer; skin cancer; brain cancer; and leukemia.

20. The compound 4-(2-((4-chlorobenzyl)sulfonyl)ethyl)benzoic acid, or pharmaceutically acceptable salt thereof.

21. A pharmaceutical composition comprising a pharmaceutically acceptable carrier and the compound, or pharmaceutically acceptable salt thereof, according to claim 20.

## Patentansprüche

1. Eine Verbindung der Formel I oder ein Salz davon:
Q₂-X-Y-CHR₃-CHR₄-Q₁ (I)
wobei:
Q₁ substituiertes Phenyl ist, mit bis zu 5 Substituenten, ausgewählt aus der Gruppe, bestehend aus:
Fluor; Brom; Nitro; -NR₁₀R₁₁; Aroylamino; Cyano; Carboxamido; Trifluormethyl; -O-R₁₀; [-N(-R₁)-(CH₂)ₘ-C(-R₅)(-R₆)-(CH₂)ₙ-COOR₇]_{z}; und einer gesättigten oder ungesättigten, geraden oder verzweigten, cyclischen oder acyclischen, chiralen oder achiralen C₁-C₁₀-Hydrocarbylgruppe, wobei wahlweise mindestens ein Kohlenstoffatom der Hydrocarbylgruppe ersetzt ist durch -N(-R₁)-, -O- oder -S-; wobei Q₁ mindestens einen Substituenten aufweist, ausgewählt aus der Gruppe, bestehend aus -O-R₁₀ und Fluor;
Q₂ substituiertes Phenyl ist, mit bis zu 5 Substituenten, ausgewählt aus der Gruppe, bestehend aus:
Fluor; Chlor; Nitro; -NR₁₀R₁₁; Aroylamino; Cyano; Carboxy; Carboxamido; Trifluormethyl; -O-SO₂-OH; -O-P(=O)(OR₈)₂; -O-R₁₀; [-N(-R₉)-(CH₂)ₘ-C(-R₅)(-R₆)-(CH₂)ₙ-COOR₇]_{z}; und einer gesättigten oder ungesättigten, geraden oder verzweigten, cyclischen oder acyclischen, chiralen oder achiralen C₁-C₁₀-Hydrocarbylgruppe, wobei wahlweise mindestens ein Kohlenstoffatom der Hydrocarbylgruppe ersetzt ist durch - N(-R₁)-, -O- oder -S-;
wobei mindestens die 4-Position von sowohl Q₁ als auch Q₂ substituiert ist, und eines von Q₁ und Q₂ mit einem von unsubstituiertem Phenyl verschiedenen Substituenten substituiert ist;
X -CH2- ist;
Y -S(=O)₂- ist;
R₁ ausgewählt ist aus der Gruppe, bestehend aus H und einer gesättigten oder ungesättigten, geraden oder verzweigten, cyclischen oder acyclischen, chiralen oder achiralen C₁-C₁₀-Hydrocarbylgruppe, wobei wahlweise mindestens ein Kohlenstoffatom der Hydrocarbylgruppe ersetzt ist durch -N(-R₁)-, -O- oder -S-;
R₃ und R₄ jeweils H sind;
R₅ und R₆ jeweils unabhängig ausgewählt sind aus der Gruppe, bestehend aus H; Halogen; und gesättigter oder ungesättigter, gerader oder verzweigter, cyclischer oder acyclischer, chiraler oder achiraler C₁-C₁₀-Hydrocarbylgruppe, wahlweise substituiert mit einem oder mehreren von Halogen, C₁-C₄-Alkyl, C₁-C₄-Alkoxy, Hydroxy, Carboxy, Carboxamido, Amino, (C₁-C₆)-Alkyl, (C₁-C₆)-Dialkylamino oder Acylamino;
oder
R₅ und R₆ sich vereinigen können, um einen gesättigten oder ungesättigten carbocyclischen Ring mit 3 bis 6 Kohlenstoffatomen zu bilden, wobei ein oder mehrere Kohlenstoffatome wahlweise ersetzt ist mit -N(-R₁)-, -O-, oder -S-;
R₇ ausgewählt ist aus der Gruppe, bestehend aus H; und einer gesättigten oder ungesättigten, geraden oder verzweigten, cyclischen oder acyclischen, chiralen oder achiralen C₁-C₁₀-Hydrocarbylgruppe; und einem anorganischen Kation oder einem organischen Kation, um ein Salz zu bilden;
R₈ ausgewählt ist aus der Gruppe, bestehend aus H und (C₁-C₇)-Hydrocarbyl; R₉ ausgewählt ist aus der Gruppe, bestehend aus H und -(CH₂)ₘ-C(-R₅)(-R₆)-(CH₂)ₙ-COOR₇,
R₁₀ ausgewählt ist aus der Gruppe, bestehend aus H, verzweigtem oder unverzweigtem (C₁-C₆)-Alkyl und (C₂-C₈)-Acyl;
R₁₁ ausgewählt ist aus der Gruppe, bestehend aus H, verzweigtem oder unverzweigtem (C₁-C₆)-Alkyl und (C₂-C₈)-Acyl;
m und n jeweils unabhängig 0-2 sind; und
z 1-2 ist.

2. Verbindung gemäß Anspruch 1 oder ein Salz davon, wobei Q₂ mindestens einen Substituenten aufweist, ausgewählt aus der Gruppe, bestehend aus Chlor; -O-R₁₀; -NR₁₀R₁₁; und [-N(-R₉)-(CH₂)ₘ-C(-R₅)(-R₆)-(CH₂)ₙ-COOR₇]_{z}.

3. Verbindung gemäß Anspruch 2 oder ein Salz davon, wobei Q₁ an den 2-, 4- und 6-Positionen substituiert ist mit (C₁-C₃)-Alkoxy und Q₂ an mindestens der 4-Position substituiert ist mit -O-R₁₀ oder -NR₁₀R₁₁.

4. Verbindung gemäß Anspruch 3 oder ein Salz davon, wobei Q₂ an der 4-Position substituiert ist mit -O-R₁₀ oder -NR₁₀R₁₁ und an der 3-Position substituiert ist mit -NR₁₀R₁₁, -O-R₁₀ oder [-N(-R₉)-(CH₂)ₘ-C(-R₅)(-R₆)-(CH₂)ₙ-COOR₇]_{z}.

5. Verbindung gemäß Anspruch 1, ausgewählt aus der Gruppe, bestehend aus:
2-((2-Methoxy-5-(((2,4,6-trimethoxyphenethyl)sulfonyl)methyl)phenyl)amino)essigsäure;
1-Chlor-4-(((4-fluorphenethyl)sulfonyl)methyl)benzol;
2-Methoxy-5-(((2,4,6-trimethoxyphenethyl)sulfonyl)methyl)anilin;
2-Methoxy-5-(((2,4,6-trimethoxyphenethyl)sulfonyl)methyl)phenol;
Methyl-2-((2-methoxy-5-(((2,4,6-trimethoxyphenethyl)sulfonyl)methyl)phenyl)amino)acetat;
Dimethyl-2,2'-((2-methoxy-5-(((2,4,6-trimethoxyphenethyl)sulfonyl)methyl)phenyl)azandiyl)diacetat;
2-(2-Methoxy-5-(((2,4,6-trimethoxyphenethyl)sulfonyl)methyl)phenyl)amino)propansäure;
2-((2-Methoxy-5-(((2,4,6-trimethoxyphenethyl)sulfonyl)methyl)phenyl)amino)-2-methylpropansäure;
2-((2-Methoxy-5-(((2,4,6-trimethoxyphenethyl)sulfonyl)methyl)phenyl)amino)-2-phenylessigsäure;
2-(4-Fluorphenyl)-2-((2-methoxy-5-(((2,4,6-trimethoxyphenethyl)sulfonyl)methyl)phenyl)amino)essigsäure;
2-(4-Chlorphenyl)-2-((2-methoxy-5-(((2,4,6-trimethoxyphenethyl)sulfonyl)methyl)phenyl)amino)essigsäure;
2-((2-Methoxy-5-(((2,4,6-trimethoxyphenethyl)sulfonyl)methyl)phenyl)amino)-3-phenylpropansäure;
2-Cyclopropyl-2-((2-methoxy-5-(((2,4,6-trimethoxyphenethyl)sulfonyl)methyl)phenyl)amino)essigsäure;
2-((2-Methoxy-5-(((2,4,6-trimethoxyphenethyl)sulfonyl)methyl)phenyl)amino)-2-(1H-pyrrol-3-yl)essigsäure;
1,3,5-Trimethoxy-2-(2-((4-methoxybenzyl)sulfonyl)ethyl)benzol;
und Salzen davon.

6. Verbindung gemäß Anspruch 5, wobei die Verbindung 2-((2-Methoxy-5-(((2,4,6-trimethoxyphenethyl)sulfonyl)methyl)phenyl)amino)acetat oder pharmazeutisch annehmbares Salz davon ist.

7. Verbindung gemäß Anspruch 6, die Natrium-2-((2-methoxy-5-(((2,4,6-trimethoxyphenethyl)sulfonyl)methyl)phenyl)amino)acetat ist.

8. Ein Verfahren zum Herstellen einer Verbindung der Formel I oder eines Salzes davon gemäß Anspruch 1, mit einer Formel Ia: 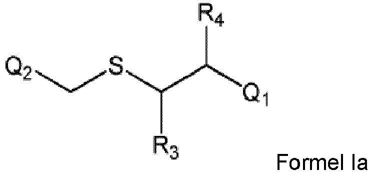 wobei das Verfahren Folgendes beinhaltet:
Umsetzen einer Verbindung der Formel AA: 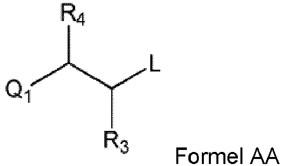 mit einer Verbindung der Formel BB: 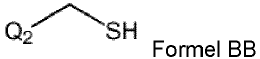 um eine Verbindung der Formel Ia oder ein Salz davon in einer Reaktionsmischung zu produzieren, und wahlweise Isolieren der Verbindung der Formel Ia oder eines Salzes davon aus der Reaktionsmischung, wobei Q₁, Q₂, R₃ und R₄ wie in Anspruch 1 definiert sind und L eine Abgangsgruppe ist, ausgewählt aus der Gruppe, bestehend aus Cl, Br und I.

9. Verfahren zum Herstellen einer Verbindung der Formel I oder eines Salzes davon gemäß Anspruch 1, mit einer Formel Ib: 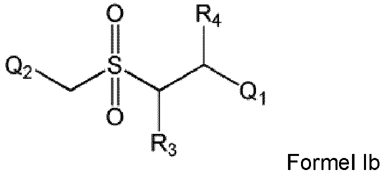 wobei das Verfahren Folgendes beinhaltet:
Oxidieren einer Verbindung der Formel Ia: 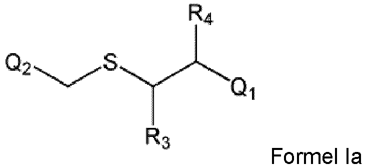 um eine Verbindung der Formel Ib oder ein Salz davon in einer Reaktionsmischung zu produzieren, und wahlweise Isolieren der Verbindung von Formel Ib oder eines Salzes davon aus der Reaktionsmischung, wobei Q₁, Q₂, R₃ und R₄ wie in Anspruch 1 definiert sind.

10. Verfahren zum Herstellen einer Verbindung der Formel I oder eines Salzes davon gemäß Anspruch 1, mit einer Formel If: 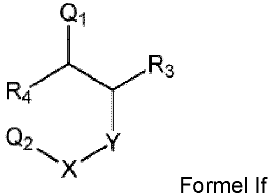 wobei das Verfahren Folgendes beinhaltet:
Umsetzen einer Verbindung der Formel Ie: 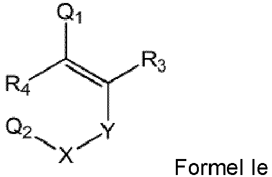 um eine Verbindung der Formel If oder ein Salz davon in einer Reaktionsmischung zu produzieren, und wahlweise Isolieren der Verbindung der Formel If oder eines Salzes davon aus der Reaktionsmischung, wobei X, Y, Q₁, Q₂, R₃ und R₄ wie in Anspruch 1 definiert sind.

11. Verfahren zum Herstellen einer Verbindung der Formel I oder eines Salzes davon gemäß Anspruch 1, mit der Formel Ii: 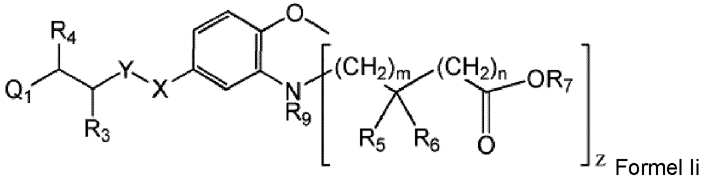 wobei das Verfahren Folgendes beinhaltet:
Umsetzen einer Verbindung der Formel Ih: 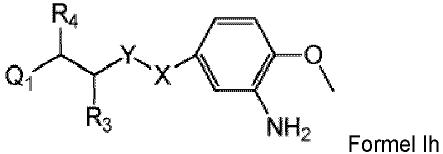 um eine Verbindung der Formel Ii oder ein Salz davon in einer Reaktionsmischung zu produzieren, und wahlweise Isolieren der Verbindung der Formel Ii oder eines Salzes davon aus der Reaktionsmischung, wobei X, Y, Q₁, R₃, R₄, R₅, R₆, R₇, R₉, m, n und z wie in Anspruch 1 definiert sind.

12. Eine pharmazeutische Zusammensetzung, beinhaltend einen pharmazeutisch annehmbaren Träger und eine Verbindung oder ein pharmazeutisch annehmbares Salz davon gemäß Anspruch 1.

13. Pharmazeutische Zusammensetzung, beinhaltend einen pharmazeutisch annehmbaren Träger und eine Verbindung oder ein pharmazeutisch annehmbares Salz davon gemäß einem der Ansprüche 5 bis 7.

14. Pharmazeutische Zusammensetzung, beinhaltend einen pharmazeutisch annehmbaren Träger und eine Verbindung oder ein pharmazeutisch annehmbares Salz davon gemäß Anspruch 6.

15. Pharmazeutische Zusammensetzung, beinhaltend einen pharmazeutisch annehmbaren Träger und die Verbindung gemäß Anspruch 7.

16. Eine Verbindung oder ein pharmazeutisch annehmbares Salz davon gemäß Anspruch 1 zur Verwendung bei der Behandlung einer proliferativen zellulären Störung.

17. Verbindung oder pharmazeutisch annehmbares Salz davon zur Verwendung gemäß Anspruch 16, wobei die Verbindung 2-((2-Methoxy-5-(((2,4,6-trimethoxyphenethyl)sulfonyl)methyl)phenyl)amino)acetat oder pharmazeutisch annehmbares Salz davon ist.

18. Verbindung oder pharmazeutisch annehmbares Salz davon zur Verwendung gemäß Anspruch 16 oder 17, wobei die proliferative zelluläre Störung Krebs ist.

19. Verbindung oder pharmazeutisch annehmbares Salz davon zur Verwendung gemäß Anspruch 18, wobei die proliferative zelluläre Störung ein Krebs ist, ausgewählt aus der Gruppe, bestehend aus Eierstockkrebs; Gebärmutterhalskrebs; Brustkrebs; Prostatakrebs; Hodenkrebs, Lungenkrebs, Nierenkrebs; Darmkrebs; Hautkrebs; Gehirnkrebs; und Leukämie.

20. Verbindung 4-(2-((4-Chlorbenzyl)sulfonyl)ethylbenzoesäure oder pharmazeutisch annehmbares Salz davon.

21. Pharmazeutische Zusammensetzung, beinhaltend einen pharmazeutisch annehmbaren Träger und die Verbindung oder ein pharmazeutisch annehmbares Salz davon gemäß Anspruch 20.

## Revendications

1. Un composé de Formule I ou un sel de celui-ci :
Q₂-X-Y-CHR₃-CHR₄-Q₁ (I)
où :
Q₁ est un phényle substitué, avec jusqu'à 5 substituants sélectionnés dans le groupe constitué de :
fluoro ; bromo ; nitro ; -NR₁₀R₁₁ ; aroylamino ; cyano ; carboxamido ; trifluorométhyle ; -O-R₁₀ ; [-N(-R₁)-(CH₂)ₘ-C(-R₅)(-R₆)-(CH₂)ₙ-COOR₇]_{z} ; et un groupe hydrocarbyle saturé ou insaturé, linéaire ou ramifié, cyclique ou acyclique, chiral ou achiral en C₁ à C₁₀, où facultativement au moins un atome de carbone du groupe hydrocarbyle est remplacé par -N(-R₁)-, -O- ou -S- ; où Q₁ a au moins un substituant sélectionné dans le groupe constitué de -O-R₁₀ et fluoro ;
Q₂ est un phényle substitué, avec jusqu'à 5 substituants sélectionnés dans le groupe constitué de :
fluoro ; chloro ; nitro ; -NR₁₀R₁₁ ; aroylamino ; cyano ; carboxy ; carboxamido ; trifluorométhyle ; -O-SO₂-OH ; -O-P(=O)(OR₈)₂ ; -O-R₁₀ ; [-N(-R₉)-(CH₂)ₘ-C(-R₅)(-R₆)-(CH₂)ₙCOOR₇]_{z} ; et un groupe hydrocarbyle saturé ou insaturé, linéaire ou ramifié, cyclique ou acyclique, chiral ou achiral en C₁ à C₁₀, où facultativement au moins un atome de carbone du groupe hydrocarbyle est remplacé par -N(-R₁)-, -O- ou -S- ; où au moins la position 4 à la fois de Q₁ et de Q₂ est substituée, et l'un de Q₁ et de Q₂ est substitué par un substituant autre qu'un phényle non substitué ;
X est -CH₂- ;
Y est -S(=O)₂- ;
R₁ est sélectionné dans le groupe constitué de H et d'un groupe hydrocarbyle saturé ou insaturé, linéaire ou ramifié, cyclique ou acyclique, chiral ou achiral en C₁ à C₁₀, où facultativement au moins un atome de carbone du groupe hydrocarbyle est remplacé par -N(-R₁)-, -O- ou -S- ;
R₃ et R₄ sont chacun H ;
R₅ et R₆ sont chacun indépendamment sélectionnés dans le groupe constitué de H ; d'un halo ; et d'un groupe hydrocarbyle saturé ou insaturé, linéaire ou ramifié, cyclique ou acyclique, chiral ou achiral en C₁ à C₁₀, facultativement substitué par un ou plusieurs éléments parmi un halogène, un alkyle en C₁ à C₄, un alcoxy en C₁ à C₄, un hydroxy, un carboxy, un carboxamido, un amino, un alkyle en C₁ à C₆, un dialkylamino en C₁ à C₆, ou un acylamino ;
ou
R₅ et R₆ peuvent se combiner afin de former un noyau carbocyclique saturé ou insaturé avec de 3 à 6 atomes de carbone, où un ou plusieurs atomes de carbone sont facultativement remplacés par -N(-R₁)-, -O-, ou -S- ;
R₇ est sélectionné dans le groupe constitué de H ; et d'un groupe hydrocarbyle saturé ou insaturé, linéaire ou ramifié, cyclique ou acyclique, chiral ou achiral en C₁ à C₁₀ ; et d'un cation inorganique ou d'un cation organique afin de former un sel ;
R₈ est sélectionné dans le groupe constitué de H et d'un hydrocarbyle en C₁ à C₇ ; Rg est sélectionné dans le groupe constitué de H et de -(CH₂)ₘ-C(-R₅)(-R₆)-(CH₂)ₙ-COOR₇ ;
R₁₀ est sélectionné dans le groupe constitué de H, d'un alkyle en C₁ à C₆ ramifié ou non ramifié, et d'un acyle en C₂ à C₈ ;
R₁₁ est sélectionné dans le groupe constitué de H, d'un alkyle en C₁ à C₆ ramifié ou non ramifié, et d'un acyle en C₂ à C₈ ;
m et n valent chacun indépendamment 0 à 2 ; et
z vaut 1 à 2.

2. Un composé selon la revendication 1, ou un sel de celui-ci, où Q₂ a au moins un substituant sélectionné dans le groupe constitué de chloro ; -O-R₁₀ ; -NR₁₀R₁₁ ; et [-N(-R₉)-(CH₂)ₘ-C(-R₅)(-R₆)-(CH₂)ₙ-COOR₇]_{z}.

3. Un composé selon la revendication 2, ou un sel de celui-ci, où Q₁ est substitué en positions 2, 4 et 6 par un alcoxy en C₁ à C₃, et Q₂ est substitué au moins en position 4 par -O-R₁₀ ou -NR₁₀R₁₁.

4. Un composé selon la revendication 3, ou un sel de celui-ci, où Q₂ est substitué en position 4 par -O-R₁₀ ou -NR₁₀R₁₁ et substitué en position 3 par -NR₁₀R₁₁, -O-R₁₀ ou [-N(-R₉)-(CH₂)ₘ-C(-R₅)(-R₆)-(CH₂)ₙ-COOR₇]_{z}.

5. Un composé selon la revendication 1 sélectionné dans le groupe constitué de :
l'acide 2-((2-méthoxy-5-(((2,4,6-triméthoxyphénéthyl)sulfonyl)méthyl)phényl)amino)acétique ;
le 1-chloro-4-(((4-fluorophénéthyl)sulfonyl)méthyl)benzène ;
la 2-méthoxy-5-(((2,4,6-triméthoxyphénéthyl)sulfonyl)méthyl)aniline ;
le 2-méthoxy-5-(((2,4,6-triméthoxyphénéthyl)sulfonyl)méthyl)phénol ;
le 2-((2-méthoxy-5-(((2,4,6-triméthoxyphénéthyl)sulfonyl)méthyl)phényl)amino)acétate de méthyle ;
le 2,2'-((2-méthoxy-5-(((2,4,6-triméthoxyphénéthyl)sulfonyl)méthyl)phényl)azanediyl)diacétate de diméthyle ;
l'acide 2-((2-méthoxy-5-(((2,4,6-triméthoxyphénéthyl)sulfonyl)méthyl)phényl)amino)propanoïque ;
l'acide 2-((2-méthoxy-5-(((2,4,6-triméthoxyphénéthyl)sulfonyl)méthyl)phényl)amino)-2-méthylpropanoïque ;
l'acide 2-((2-méthoxy-5-(((2,4,6-triméthoxyphénéthyl)sulfonyl)méthyl)phényl)amino)-2-phénylacétique ;
l'acide 2-(4-fluorophényl)-2-((2-méthoxy-5-(((2,4,6-triméthoxyphénéthyl)sulfonyl)méthyl)phényl)amino)acétique ;
l'acide 2-(4-chlorophényl)-2-((2-méthoxy-5-(((2,4,6-triméthoxyphénéthyl)sulfonyl)méthyl)phényl)amino)acétique ;
l'acide 2-((2-méthoxy-5-(((2,4,6-triméthoxyphénéthyl)sulfonyl)méthyl)phényl)amino)-3-phénylpropanoïque ;
l'acide 2-cyclopropyl-2-((2-méthoxy-5-(((2,4,6-triméthoxyphénéthyl)sulfonyl)méthyl)phényl)amino)acétique ;
l'acide 2-((2-méthoxy-5-(((2,4,6-triméthoxyphénéthyl)sulfonyl)méthyl)phényl)amino)-2-(1H-pyrrol-3-yl)acétique ;
le 1,3,5-triméthoxy-2-(2-((4-méthoxybenzyl)sulfonyl)éthyl)benzène ;
et des sels de celui-ci.

6. Un composé selon la revendication 5, le composé étant le 2-((2-méthoxy-5-(((2,4,6-triméthoxyphénéthyl)sulfonyl)méthyl)phényl)amino)acétate ou un sel pharmaceutiquement acceptable de celui-ci.

7. Le composé selon la revendication 6 qui est le 2-((2-méthoxy-5-(((2,4,6-triméthoxyphénéthyl)sulfonyl)méthyl)phényl)amino)acétate de sodium.

8. Un procédé de fabrication d'un composé de Formule I ou d'un sel de celui-ci, selon la revendication 1, ayant une Formule Ia : 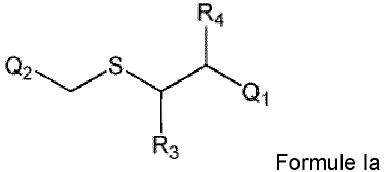 ledit procédé comprenant :
la réaction d'un composé de Formule AA : 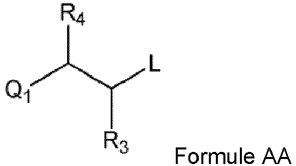 avec un composé de Formule BB : 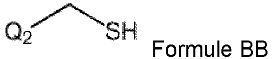 afin de produire un composé de Formule la ou un sel de celui-ci dans un mélange réactionnel, et facultativement l'isolement du composé de Formule la ou d'un sel de celui-ci à partir du mélange réactionnel, où Q₁, Q₂, R₃, et R₄ sont tels que définis dans la revendication 1, et L est un groupe partant sélectionné dans le groupe constitué de Cl, Br, et I.

9. Un procédé de fabrication d'un composé de Formule I ou d'un sel de celui-ci selon la revendication 1, ayant une Formule Ib : 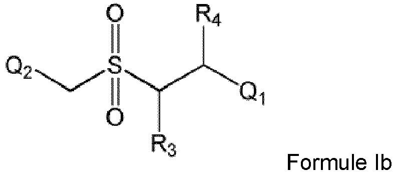 ledit procédé comprenant :
l'oxydation d'un composé de Formule la : 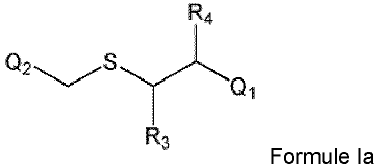 afin de produire un composé de Formule Ib ou un sel de celui-ci dans un mélange réactionnel, et facultativement l'isolement du composé de Formule Ib ou d'un sel de celui-ci à partir du mélange réactionnel, où Q₁, Q₂, R₃, et R₄ sont tels que définis dans la revendication 1.

10. Un procédé de fabrication d'un composé de Formule I ou d'un sel de celui-ci selon la revendication 1, ayant une Formule If ; 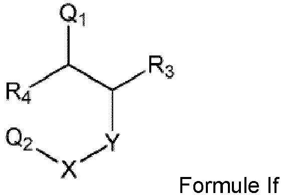 ledit procédé comprenant :
la réaction d'un composé de Formule le: 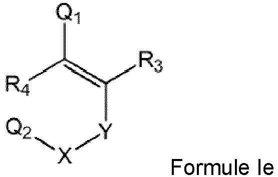 afin de produire un composé de Formule If ou un sel de celui-ci dans un mélange réactionnel, et facultativement l'isolement du composé de Formule If ou d'un sel de celui-ci à partir du mélange réactionnel, où X, Y, Q₁, Q₂, R₃, et R₄ sont tels que définis dans la revendication 1.

11. Un procédé de fabrication d'un composé de Formule I ou d'un sel de celui-ci selon la revendication 1, ayant la Formule Ii : 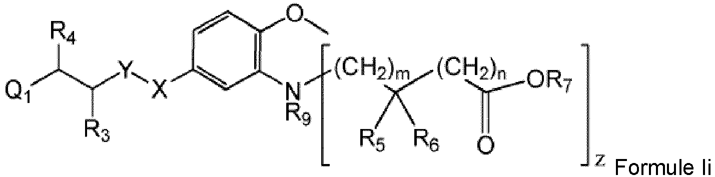 ledit procédé comprenant :
la réaction d'un composé de Formule Ih: 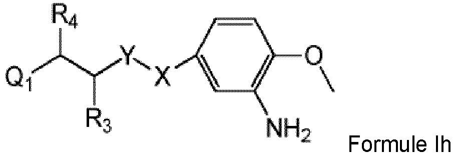 afin de produire un composé de Formule Ii ou un sel de celui-ci dans un mélange réactionnel, et facultativement l'isolement du composé de Formule Ii ou d'un sel de celui-ci à partir du mélange réactionnel, où X, Y, Q₁, R₃, R₄, R₅, R₆, R₇, R₉, m, n, et z sont tels que définis dans la revendication 1.

12. Une composition pharmaceutique comprenant un support pharmaceutiquement acceptable et un composé, ou un sel pharmaceutiquement acceptable de celui-ci, selon la revendication 1.

13. Une composition pharmaceutique comprenant un support pharmaceutiquement acceptable et un composé, ou un sel pharmaceutiquement acceptable de celui-ci, selon l'une quelconque des revendications 5 à 7.

14. Une composition pharmaceutique comprenant un support pharmaceutiquement acceptable et un composé, ou un sel pharmaceutiquement acceptable de celui-ci, selon la revendication 6.

15. Une composition pharmaceutique comprenant un support pharmaceutiquement acceptable et le composé selon la revendication 7.

16. Un composé, ou un sel pharmaceutiquement acceptable de celui-ci, selon la revendication 1 pour utilisation dans le traitement d'un trouble prolifératif cellulaire.

17. Le composé ou sel pharmaceutiquement acceptable de celui-ci pour utilisation selon la revendication 16, où le composé est le 2-((2-méthoxy-5-(((2,4,6-triméthoxyphénéthyl)sulfonyl)méthyl)phényl)amino)acétate ou un sel pharmaceutiquement acceptable de celui-ci.

18. Le composé ou sel pharmaceutiquement acceptable de celui-ci pour utilisation selon la revendication 16 ou la revendication 17, où le trouble prolifératif cellulaire est un cancer.

19. Le composé ou sel pharmaceutiquement acceptable de celui-ci pour utilisation selon la revendication 18, où le trouble prolifératif cellulaire est un cancer sélectionné dans le groupe constitué du cancer de l'ovaire ; du cancer du col de l'utérus ; du cancer du sein ; du cancer de la prostate ; du cancer du testicule, du cancer du poumon, du cancer du rein ; du cancer colorectal ; du cancer de la peau ; du cancer du cerveau ; et de la leucémie.

20. Le composé acide 4-(2-((4-chlorobenzyl)sulfonyl)éthyl)benzoïque, ou sel pharmaceutiquement acceptable de celui-ci.

21. Une composition pharmaceutique comprenant un support pharmaceutiquement acceptable et le composé, ou sel pharmaceutiquement acceptable de celui-ci, selon la revendication 20.
